# EUROPEAN PATENT SPECIFICATION

(11) **EP 4 153 242 B1**
(45) Date of publication and mention of the grant of the patent: **15.05.2024**
(21) Application number: 21726398.7
(22) Date of filing: 18.05.2021
(51) Int. Cl.: A61K 47/68, C07C 275/16, C07D 207/09, C07D 207/452, C07D 207/456, C07D 405/12, C07D 491/22

(54) **PARA-AMINO-BENZYL LINKERS, PROCESS FOR THEIR PREPARATION AND THEIR USE IN CONJUGATES**
PARA-AMINO-BENZYL-LINKER, VERFAHREN ZU IHRER HERSTELLUNG UND IHRE VERWENDUNG IN KONJUGATEN
LIEURS PARA-AMINO-BENZYLIQUES, LEUR PROCÉDÉ DE PRÉPARATION ET LEUR UTILISATION DANS DES CONJUGUÉS

(30) Priority: 19.05.2020 EP 20315247; 24.11.2020 EP 20209379
(43) Date of publication of application: 29.03.2023
(73) Proprietor: Les Laboratoires Servier, 92284 Suresnes Cedex (FR)
(72) Inventor: DESOS, Patrice, 92270 BOIS-COLOMBES (FR); FRANZETTI, Georges-Alain, 75014 PARIS (FR); STARCK, Jérôme-Benoît, 92500 Rueil-Malmaison (FR); KOSTOVA, Vesela, 92210 Saint Cloud (FR)
(74) Representative: Bernad, Stéphane
(86) International application number: PCT/EP2021/063195
(87) International publication number: WO 2021/233944

(56) References cited:
- WO-A1-2011/130598
- WO-A1-2017/214282
- WO-A1-2019/108974

## Description

The present invention relates to para-amino-benzyl linker compounds useful for linking drug moieties to antibodies, to linker-drug compounds in which said para-amino-benzyl linker compounds are covalently linked to drug moieties, and to antibody-drug conjugates comprising said para-amino-benzyl linker compounds covalently linked to drug wherein said drug is enzymatically cleaved from the conjugate at a particular cell or tissue type targeted by said antibody The scope of the invention is defined by the claims. Any references in the description to methods of treatment refer to the compounds, pharmaceutical compositions and medicaments of the present invention for use in a method for treatment of the human (or animal) body by therapy.

### BACKGROUND OF THE INVENTION

Targeted therapeutics are designed to reduce nonspecific toxicities and increase efficacy relative to conventional therapy. This approach is embodied by the powerful targeting ability of monoclonal antibodies or antigen-binding fragments to specifically deliver highly potent, conjugated small molecule therapeutics to antigen-positive cells. To address the issue of toxicity, chemotherapeutic agents (drugs) have been coupled to targeting molecules such as antibodies or protein receptor ligands that bind with a high degree of specificity to tumor cells to form compounds referred to as antibody-drug conjugates (ADC) or immunoconjugates (Chau et al. Lancet 2019, 394, 793-804). ADC or immunoconjugates in theory should be less toxic because they direct the cytotoxic drug to disease cells that overexpress the particular cell surface antigen or receptor compared to normal cells. Promising advancements with ADC or immunoconjugates have seen cytotoxic drugs linked to antibodies through a linker that is cleaved at the tumor site or inside tumor cells. Such advancements can be applied to any diseases needing a targeting delivery (Tumey, Innovations for Next-Generation Antibody-Drug Conjugates. Humana Press 2018, 187-214). Recent examples show also an interest in immune-mediated inflammatory diseases (Wang et al. J Am Chem Soc. 2015, 137, 3229-32), muscle-related diseases (Sugo et al. J. Control. Release 2016, 237, 1-13) and diseases caused by bacteria infection (Mariathasan et al. Trends Mol. Med. 2017, 23, 135-149). Moreover, ADCs are not restricted to cytotoxic drugs and can be used to deliver specifically imaging agents (Dammes et al. Theranostics 2020, 10, 938-955) or oligonucleotides (Dovgan et al. Bioconjug. Chem. 2019, 30, 2483-2501) to antigen positive cells.

Linkers may impact the physico-chemical properties of an ADC. As many cytotoxic agents are hydrophobic in nature, linking them to the antibody with an additional hydrophobic moiety may lead to aggregation. ADC aggregates are insoluble and often limit achievable drug loading onto the antibody, which can negatively affect the potency of the ADC. Protein aggregates of biologics, in general, have also been linked to increased immunogenicity.

A chemical solution to targeted delivery of drugs, e.g. cytotoxic or cytostatic drugs, conjugated to cell-specific ligands is the "self-immolative linker", PABC or PAB (para-aminobenzyloxycarbonyl or para-aminobenzyl), attaching the drug moiety to the ligand in the conjugate (Alouane et al. Angew. Chem. Int. Ed. 2015, 54, 7492-7509; Bargh et al. Chem. Soc. Rev. 2019, 48, 4361-4374). The PAB linker unit is also referred to as an electronic cascade spacer. The amide bond linking the carboxy terminus of a peptide unit and the para-aminobenzyl of PABC or PAB may be a substrate and cleavable by certain proteases. The aromatic amine becomes electron-donating and initiates an electronic cascade that leads to the expulsion of the leaving group, which releases the free drug after elimination of carbon dioxide (de Groot et al. J. Org. Chem. 2001, 66, 8815-8830). Upon cleavage of a peptide bond adjacent to the PABC or PAB, i.e. by an intracellular enzyme, the drug is released from the ligand whereby no remaining portion of the linker is bound (de Groot et al. Molecular Cancer Therapeutics 2002, 1, 901-911; de Groot et al. J. Med. Chem. 1999, 42, 5277-5283).

Linkers containing the para-aminobenzyl or para-aminobenzyloxycarbonyl (PAB or PABC) unit, in conjunction with a peptide unit, have been developed with a "self-immolating" or "self-immolative" mechanism of 1,6-elimination and fragmentation under enzymatic, hydrolytic, or other metabolic conditions to release a drug moiety from a targeting ligand, such as an antibody (EP 1 718 667; WO 2016/040684). For the use of the PAB unit in prodrugs and conjugates, see also: Alouane et al. Angew. Chem. Int. Ed. 2015, 54, 7492-7509; Bargh et al. Chem. Soc. Rev. 2019, 48, 4361-4374; Dal Corso et al. Chem. Eur. J. 2019, 25,14740-14757.

Limitations of the PAB type self-immolating linkers are the propensity to cause poor solubility and aggregation of the conjugates. For example, in WO 2017/214282, PAB type self-immolating linkers containing several hydrophilic groups (such as glucoronides, polyhydroxylated side chains ...) lead to high aggregation of conjugates (>15%). In addition, some PAB-containing conjugates may not be suitable substrates for certain cleaving enzymes or cleave too slowly to achieve efficacy. Consequently, it would be desirable to improve the properties of antibody-drug conjugates by optimizing the structure of a self-immolative linker in order to reduce aggregation, while keeping pharmacological activity and stability. Unexpectedly, these novel para-amino-benzyl linkers, comprising a para-amino-benzyl unit and a peptide unit as disclosed herein, address the above-mentioned drawbacks and their use result in ADCs with low aggregation levels, good stability, desirable levels of drug loading and expected pharmacological activity, as demonstrated in the following Examples.

### BRIEF DESCRIPTION OF THE FIGURES

**Figure 1** shows site-specific antibody conjugation using bacterial transglutaminase (LAR2).
**Figure 2** shows conjugation using 4 inter-chain disulfide bridges of the antibody.
**Figure 3** shows conjugation using rebridging technology generating fragmentation.

### DEFINITIONS

The term "antibody" or "Ab" is used in the broadest sense to refer to an immunoglobulin molecule that recognizes and specifically binds to a target, such as a protein, polypeptide, carbohydrate, polynucleotide, lipid, or combinations of the foregoing through at least one antigen recognition site within the variable region of the immunoglobulin molecule. An antibody can be polyclonal or monoclonal, multiple or single chain, or an intact immunoglobulin, and may be derived from natural sources or from recombinant sources. An "intact" antibody is a glycoprotein that typically comprises at least two heavy (H) chains and two light (L) chains inter-connected by disulfide bonds. Each heavy chain is comprised of a heavy chain variable region (abbreviated herein as VH) and a heavy chain constant region. The heavy chain constant region comprises three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxyl-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system. An antibody can be a monoclonal antibody, human antibody, humanized antibody, camelised antibody, or chimeric antibody. The antibodies can be of any isotype (e.g., IgG, IgE, IgM, IgD, IgA and IgY), class (e.g., IgG1, IgG2, IgG3, IgG4, IgA1 and IgA2), or subclass. An antibody can be an intact antibody or an antigen-binding fragment thereof.

The term "antibody fragment" or "antigen-binding fragment" or "fragment" as used herein, refers to at least one portion of an antibody that retains the ability to specifically interact with (e.g., by binding, steric hinderance, stabilizing/destabilizing, spatial distribution) an epitope of an antigen. Antigen-binding fragments may also retain the ability to internalize into an antigen-expressing cell. In some embodiments, antigen-binding fragments also retain immune effector activity. The terms antibody, antibody fragment, antigen-binding fragment, and the like, are intended to embrace the use of binding domains from antibodies in the context of larger macromolecules such as ADCs. It has been shown that fragments of a full-length antibody can perform the antigen binding function of a full-length antibody. Examples of antibody fragments include, but are not limited to, Fab, Fab', F(ab')2, Fv fragments, scFv antibody fragments, disulfide-linked Fvs (sdFv), a Fd fragment consisting of the VH and CH1 domains, linear antibodies, single domain antibodies such as sdAb (either VL or VH), camelid VHH domains, multi-specific antibodies formed from antibody fragments such as a bivalent fragment comprising two Fab fragments linked by a disulfide bridge at the hinge region, and an isolated CDR or other epitope binding fragments of an antibody. An antigen-binding fragment can also be incorporated into single domain antibodies, maxibodies, minibodies, nanobodies, intrabodies, diabodies, triabodies, tetrabodies, bispecific or multi-specific antibody constructs, ADCs, v-NAR and bis-scFv (see, e.g., Holliger and Hudson Nat. Biotechnol. 2005, 23, 1126-1136). Antigen-binding fragments can also be grafted into scaffolds based on polypeptides such as a fibronectin type III (Fn3) (see US Patent No. 6,703,199, which describes fibronectin polypeptide minibodies).

The term "scFv" refers to a fusion protein comprising at least one antigen-binding fragment comprising a variable region of a light chain and at least one antigen-binding fragment comprising a variable region of a heavy chain, wherein the light and heavy chain variable regions are contiguously linked, e.g., via a synthetic linker, e.g., a short flexible polypeptide linker, and capable of being expressed as a single chain polypeptide, and wherein the scFv retains the specificity of the intact antibody from which it is derived. Unless specified, an scFv may have the VL and VH variable regions in either order, e.g., with respect to the N-terminal and C-terminal ends of the polypeptide, the scFv may comprise VL-linker-VH or may comprise VH-linker-VL. Antigen-binding fragments are obtained using conventional techniques known to those of skill in the art, and the binding fragments are screened for utility (e.g., binding affinity, internalization) in the same manner as are intact antibodies. Antigen-binding fragments, for example, may be prepared by cleavage of the intact protein, e.g., by protease or chemical cleavage.

The term "drug" or "agent" or "therapeutic drug" or "therapeutic agent", as used herein, refers to a chemical compound, a mixture of chemical compounds, a biological macromolecule, an extract made from biological materials, or a combination of two or more thereof, that is capable of modulating a biological process and/or has biological activity. Particularly, the term "drug" can represent a "chemotherapeutic agent" or an "anti-cancer agent".

The term "chemotherapeutic agent" or "anti-cancer agent" is used herein to refer to all agents that are effective in treating cancer (regardless of mechanism of action). Inhibition of metastasis or angiogenesis is frequently a property of a chemotherapeutic agent. Chemotherapeutic agents include antibodies, biological molecules, and small molecules. A chemotherapeutic agent may be a cytotoxic or cytostatic agent. Examples of chemotherapeutic agent include, without implying any limitation, monomethyl auristatin E, auristatin E, SN-38, doxorubicin, etc.

The term "cytostatic agent" refers to an agent that inhibits or suppresses cell growth and/or multiplication of cells.

The term "cytotoxic agent" refers to a substance that causes cell death primarily by interfering with a cell's expression activity and/or functioning.

As used herein the term "halo(C₁-C₄)alkyl" means (C₁-C₄)alkyl radical which is substituted by one or more halogen atoms such as fluorine, chlorine, bromine or iodine including, but not limited to, fluoromethyl, difluoromethyl, trifluoromethyl, 2-fluoroethyl, 2,2-difluoroethyl, 2,2,2-trifluoroethyl, 2,2,2-trifluoro-1,1-dimethylethyl, 2,2,2-trichloroethyl, 3-fluoropropyl, 4-fluorobutyl, chloromethyl, trichloromethyl, iodomethyl, and bromomethyl.

The term "protecting group" refers to a substituent that is commonly employed to block or protect a particular functionality while reacting other functional groups on the compound. For example, an "amino-protecting group" is a substituent attached to an amino group that blocks or protects the amino functionality in the compound. Suitable amino-protecting groups include acetyl, trifluoroacetyl, t-butoxycarbonyl (Boc), benzyloxycarbonyl (CBz) and 9-fluorenylmethylenoxycarbonyl (Fmoc). Similarly, a "hydroxy-protecting group" refers to a substituent of a hydroxy group that blocks or protects the hydroxy functionality. Suitable hydroxy-protecting groups include acetyl, benzyl, benzoyl, tetrahydropyranyl, and trialkylsilyl. Also, a "carbonyl-protecting group" refers to a substituent of a carbonyl group that blocks or protects the carbonyl functionality. Suitable carbonyl-protecting groups include acetal, acylal, and dithianes. Similarly, a "carboxyl-protecting group" refers to a substituent of a carboxyl group that blocks or protects the carboxyl functionality. Suitable carboxyl-protecting groups include methyl ester, benzyl ester, tert-butyl ester, silyl ester, and orthoester. For a general description of protecting groups and their use, see T. W. Greene, Protective Groups in Organic Synthesis, John Wiley & Sons, New York, 1991.

The term "moiety" as used herein means a specified segment, fragment or functional group of a molecule or compound. Chemical moieties are sometimes indicated as chemical entities that are embedded in or appended (i.e., a substituent or variable group) to a molecule, compound or chemical formula.

The term "bridging spacer", as used herein, refers to one or more linker components which are covalently attached together to form a bivalent moiety which links the bivalent peptide A₁-A₂ to the reactive group. In certain embodiments, the "bridging spacer" comprises a carbonyl group attached to the N-terminus of the A₂ group via an amide bond. In certain embodiments, the "bridging spacer" comprises an amino group attached to the carbonyl group of the A₂ group via an amide bond. In some embodiments, the bridging spacer comprises a polyoxyethylene (PEG) group. In other embodiments, the bridging spacer comprises a butanoyl, pentanoyl, hexanoyl, heptanoyl, or octanoyl group. In some embodiments, the bridging spacer comprises a hexanoyl group (i.e. a -CO-(CH₂)₅- group). In preferred embodiments, the bridging spacer may comprise: -CO-CH₂-CH₂-PEG1-, -CO-CH₂-PEG3-, or -NH-CH₂-CH₂-PEG1-.

The term "polyoxyethylene", "polyethylene glycol" or "PEG", as used herein, refers to a linear chain, a branched chain or a star shaped configuration comprised of (OCH₂CH₂) groups. In certain embodiments, a polyoxyethylene or PEG group is *-(OCH₂CH₂)ₜ-**, where t is 1-20, and where the "*" indicates the end directed toward the A₂-A₁ group and the "**" indicates the point of attachment to a reactive group. In some embodiments, the bridging spacer comprises a PEG group which is *-(OCH₂CH₂)ₜ-**, where t is 1-20, and where the "*" indicates the point of attachment to a -(CH₂)ₙ-C(O)- group wherein the carbonyl group is attached to the N-terminus of the A₂ group and n is 1-5, and the "**" indicates the point of attachment to a reactive group. In some embodiments, the bridging spacer comprises a PEG group which is *-(OCH₂CH₂)ₜ-**, where t is 1-20, and where the "*" indicates the point of attachment to a -(CH₂)ₙ-NH- group wherein the amino group is attached to the carbonyl group of the A₂ group and n is 1-5, and the "**" indicates the point of attachment to a reactive group. For example, the term "PEG3" as used herein means that t is 3.

The term "reactive group", as used herein, is a functional group capable of forming a covalent bond with a functional group of an antibody or an antibody fragment. In some embodiments, the reactive group is selected from, but are not limited to, a thiol, a maleimide, a dibromomaleimide, a haloacetamide, an azide, an alkyne, a cyclcooctene, a triaryl phosphine, an oxanobornadiene, a cyclooctyne, a diaryl tetrazine, a monoaryl tetrazine, a norbornene, an aldehyde, a hydroxylamine, a hydrazine, NH₂-NH-C(=O)-, a ketone, a vinyl sulfone, an aziridine, an amino, an amino acid residue. In a preferred embodiment, the reactive group is selected from a maleimide group, a dibromomaleimide group, a thiol group, a cyclooctyne group, or an azido group, more preferably, a maleimide group, a dibromomaleimide group or an azido group. For example, maleimide or dibromomaleimide groups may have the structure: 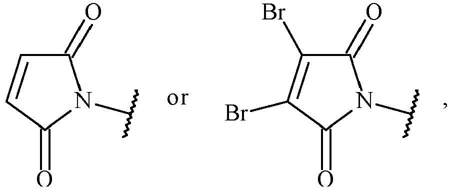 respectively.

For example, the azido group may have the structure: -N=N⁺=N⁻.

The term "attachment group", as used herein, refers to a bivalent or trivalent moiety which links the bridging spacer to the antibody or fragment thereof. The attachment group is a bivalent or trivalent moiety formed by the reaction between a reactive group and a functional group on the antibody or fragment thereof. In some embodiments, the reactive group for the formation of the attachment group is selected from, but are not limited to, a thiol, a maleimide, a dibromomaleimide, a haloacetamide, an azide, an alkyne, a cyclcooctene, a triaryl phosphine, an oxanobornadiene, a cyclooctyne, a diaryl tetrazine, a monoaryl tetrazine, a norbornene, an aldehyde, a hydroxylamine, a hydrazine, NH₂-NH-C(=O)-, a ketone, a vinyl sulfone, an aziridine, an amino, an amino acid residue. In a preferred embodiment, the reactive group for the formation of the attachment group is selected from a maleimide group, a dibromomaleimide group, a thiol group, a cyclooctyne group, or an azido group, more preferably, a maleimide group, a dibromomaleimide group or an azido group. The term "Spacer Unit precursor" or "Spacer Unit precursor Z' " or "Z' ", as used herein, refers to a component comprising a bridging spacer and a reactive group.

The term "Spacer Unit" or "Spacer Unit Z" or "Z", as used herein, refers to a component comprising a bridging spacer and an attachment group.

The term "conjugate" or "antibody-drug conjugate" or "ADC" or "immunoconjugate", as used herein, refers to an antibody which is covalently attached to a drug moiety through a para-amino-benzyl linker of Formula (I). In one embodiment, antibody-drug conjugate of Formula (III) wherein the Spacer Unit Z is coupled to antibody Ab via an amide group may be prepared by reacting a free amine functional group on antibody Ab with an active ester containing precursor of Spacer Unit Z. For example, a carboxyl group on Spacer Unit precursor Z' may be activated by reacting with N-hydroxysuccinimide and then reacted with Ab-NH₂ to form a conjugate in which Ab and Z are coupled by way of an amide group. Useful functional groups on an antibody for linking to the Spacer Unit Z, either naturally or via chemical manipulation include, but are not limited to, sulfhydryl (-SH), amino, hydroxyl, the anomeric hydroxyl group of a carbohydrate, and carboxyl. In one embodiment, the reactive functional groups on the antibody are sulfhydryl and amino. Sulfhydryl groups can be generated by reduction of an intramolecular cysteine disulfide bond of an antibody. Alternatively, sulfhydryl groups can be generated by reaction of an amino group of a lysine moiety of an antibody using 2-iminothiolane (Traut's reagent) or another sulfhydryl generating reagent. In another embodiment, the Spacer Unit Z is linked to the antibody Ab via a disulfide bond between a sulfur atom of the antibody and a sulfur atom of the Spacer Unit Z. In yet another embodiment, the reactive group of the Spacer Unit Z contains a reactive site that can form a bond with a primary or secondary amino group of an antibody. Example of these reactive sites include, but are not limited to, activated esters such as succinimide esters, 4-nitrophenyl esters, pentafluorophenyl esters, tetrafluorophenyl esters, anhydrides, acid chlorides, sulfonyl chlorides, isocyanates and isothiocyanates. In yet another embodiment, the reactive group of the Spacer Unit Z reacts with an aldehyde, acetal, or ketal group on a sugar (carbohydrate) of a glycosylated antibody. For example, a carbohydrate can be mildly oxidized using a reagent such as sodium periodate and the resulting (-CHO) unit of the oxidized carbohydrate can be condensed with a Spacer Unit that contains a functionality such as a hydrazide, an oxime, a primary or secondary amine, a hydrazine, a thiosemicarbazone, a hydrazine carboxylate, and an arylhydrazide such as those described by Kaneko et al. Bioconjugate Chem. 1991, 2, 133-141.

The generation of the ADCs can be accomplished by techniques known to the skilled artisan. In an exemplary embodiment, the invention provides for an ADC of Formula (III) prepared from contacting a Linker-Drug compound of Formula (II) with an antibody (or fragment thereof) having a reactive sulfhydryl, amino or carboxyl moiety under suitable conditions to effect condensation of the reactive moiety with the Spacer Unit precursor Z' moiety of the Linker-Drug compound of Formula (II), wherein Z' is converted to Z resulting from said contact.

In some embodiment, the ADC of Formula (III) incorporates a Spacer Unit Z which is a divalent moiety that couples the N-terminus of the A₂-A₁ group to the antibody. In some embodiment, the ADC of Formula (III) incorporates a Spacer Unit Z which is a divalent moiety that couples the carbonyl group of the A₂-A₁ group to the antibody.

The Spacer Unit Z is covalently bound to a functional group pending from the antibody such as an amine (e.g. -NH₂ from a Lys residue), a carboxyl (-COOH from an Asp or Glu residue) or a sulfhydryl (e.g. -SH from a Cys residue) which forms an amide or a thioether or disulfide group. Antibody-drug conjugate of the invention in which the Spacer Unit precursor Z' is reacted with a sulfhydryl functional group of the antibody (for example, Cys containing peptide or a reduced antibody) to form a thioether linkage include the one represented by the following formula: 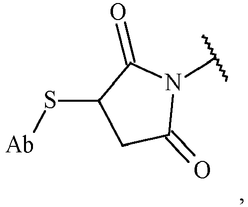 wherein Ab is an antibody or an antigen-binding fragment thereof. In other embodiment, antibody-drug conjugate of the invention in which the Spacer Unit precursor Z' is reacted with two sulfhydryl functional groups of the antibody to form two thioether linkages include the one represented by the following formula: 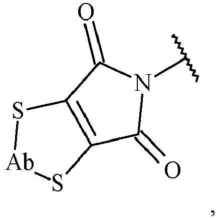 wherein Ab is an antibody or an antigen-binding fragment thereof.

In some embodiments, the antibody or antigen-binding fragment is functionalized to prepare a functional group that is reactive with a Linker-Drug intermediate. In some embodiments, an antibody or antigen-binding fragment is prepared with bacterial transglutaminase (BTG) which are reactive glutamines specifically functionalized with an amine containing cyclooctyne BCN (N [(1R,8S,9s)-Bicyclo[6.1.0]non-4-yn-9-ylmethyloxycarbonyl]-1,8-diamino-3,6-dioxaoctane) moiety. In some embodiments, ADC of the invention in which the Spacer Unit precursor Z' is reacted with a glutamine functional group of antibody to form an amide linkage include the one represented by the following formula: 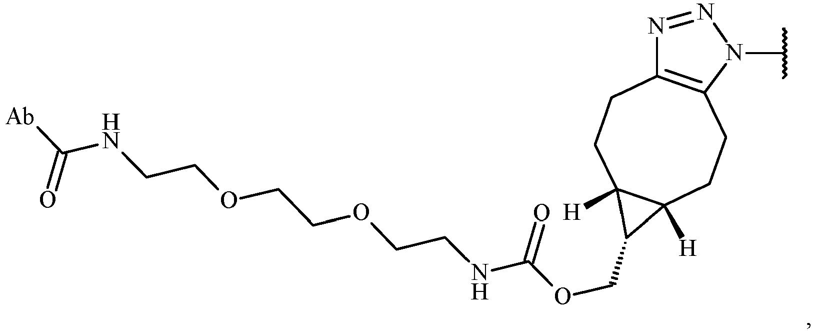 wherein Ab is an antibody or an antigen-binding fragment thereof.

The term "drug loading" is represented by p, and is also referred to herein as the drug-to-antibody ratio (DAR or drug:antibody ratio). Drug loading may range from 1 to 8 drug moieties per antibody or antigen-binding fragment. In some embodiments, higher drug loading (e.g., p > 8) may cause aggregation, insolubility, toxicity, or loss of cellular permeability of certain antibody-drug conjugates. Higher drug loading may also negatively affect the pharmacokinetics (e.g., clearance) of certain ADCs. In some embodiments, lower drug loading (e.g., p < 2) may reduce the potency of certain ADCs against target-expressing cells. In some embodiments, p is an integer from 2 to 8. In some embodiments, p is 2, or p is 4, or p is 8.

The drug loading of an ADC may be controlled in different ways, e.g., but not limited to, by limiting the molar excess of drug-linker intermediate or linker reagent relative to antibody, or by limiting the conjugation reaction time or temperature.

In some embodiments, the drug loading in a mixture of ADCs resulting from a conjugation reaction ranges from 1 to 8 drug moieties attached per antibody or antigen-binding fragment. The average number of drug moieties per antibody or antigen-binding fragment (i.e., the average drug loading, or average p) may be calculated by any conventional method known in the art, e.g., by mass spectrometry (e.g., liquid chromatography-mass spectrometry (LC-MS)) and/or high-performance liquid chromatography (e.g., HIC-HPLC). In some embodiments, the average number of drug moieties per antibody or antigen-binding fragment is from about 1.5 to about 2.5, or about 7.0 to about 8. In some embodiments, the average number of drug moieties per antibody or antigen-binding fragment is about 2. In some embodiments, the average number of drug moieties per antibody or antigen-binding fragment is 2. In some embodiments, the average number of drug moieties per antibody or antigen-binding fragment is about 4. In some embodiments, the average number of drug moieties per antibody or antigen-binding fragment is 4. In some embodiments, the average number of drug moieties per antibody or antigen-binding fragment is about 8. In some embodiments, the average number of drug moieties per antibody or antigen-binding fragment is 8.

### DETAILED DESCRIPTION OF THE INVENTION

According to a first aspect of the invention, there is provided a para-amino-benzyl linker compound of Formula (I): 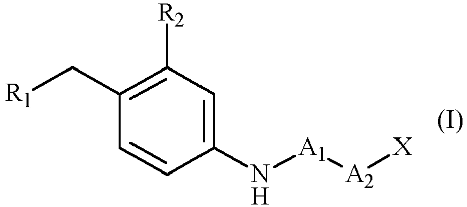 wherein:
- R₁ represents a hydroxy group or a halogen atom;
- R₂ represents a -S(O)₂(OH) group, a -S(O)₂(O⁻M⁺) group, a linear or branched -(C₁-C₄)alkyl-S(O)₂(OH) group, a linear or branched -(C₁-C₄)alkyl-S(O)₂(O⁻M⁺) group, a linear or branched -halo(C₁-C₄)alkyl-S(O)₂(OH) group, or a linear or branched -halo(C₁-C₄)alkyl-S(O)₂(O⁻M⁺) group;
- A₁ represents a -C(O)-CH(R₃)-NH- group;
- A₂ represents a -C(O)-CH(R₄)-NH- group, a 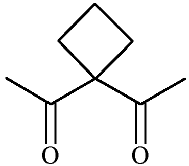 group, a 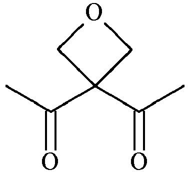 group, or a 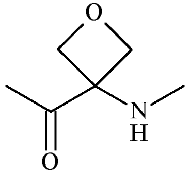 group;
- R₃ and R₄, independently of one another, represent the side chain of an amino acid;
- X represents a hydrogen atom, a hydroxy group, or a protecting group;
- M⁺ represents a pharmaceutically acceptable monovalent cation.

In a preferred embodiment, R₁ represents a hydroxy group, a bromine atom, a chlorine atom, or an iodine atom. More preferably, R₁ represents a hydroxy group, a chlorine atom, or an iodine atom. Even more preferably, R₁ represents a hydroxy group.

Preferably, R₂ represents a -S(O)₂(OH) group, a -S(O)₂(O⁻M⁺) group, a -CH₂-S(O)₂(OH) group, a -CH₂-S(O)₂(O⁻M⁺) group, a -CH₂-CH₂-S(O)₂(OH) group, a -CH₂-CH₂-S(O)₂(O⁻M⁺) group, a -CH₂-CH₂-CH₂-S(O)₂(OH) group, or a -CH₂-CH₂-CH₂-S(O)₂(O⁻M⁺) group. Examples of M⁺ include Li⁺, Na⁺, or K⁺. In a preferred embodiment, M⁺ represents Na⁺.

In a particular embodiment, A₁ represents a -C(O)-CH(R₃)-NH- group and A₂ represents a -C(O)-CH(R₄)-NH- group, in which R₃ and R₄, independently of one another, represent the side chain of an amino acid. The side chain-bearing carbon may be in either D or L (R or S) configuration. Preferably, the side chain-bearing carbon is L configuration. In a particular embodiment, R₃ and R₄, independently of one another, represent, but are not limited to, hydrogen, methyl, n-propyl, isopropyl, n-butyl, isobutyl, sec-butyl, phenyl, benzyl, p-hydroxybenzyl, -CH₂OH, -CH₂SH, -CH₂SeH -CH₂CH₂OH, -CH₂CH₂SH, -CH(OH)CH₃, -CH₂SCH₃, -CH₂CH₂SCH₃, -CH₂CONH₂, -CH₂COOH, -CH₂CH₂CONH₂, -CH₂CH₂COOH, -(CH₂)₃NHC(=NH)NH₂, -(CH₂)₃NH₂, -(CH₂)₃NHCOCH₃, -(CH₂)₃NHCHO, -(CH₂)₄NHC(=NH)NH₂, -(CH₂)₄NH₂, -(CH₂)₄NHCO-(3-methyl-3,4-dihydro-2H-pyrrol-2-yl), -(CH₂)₄NHCO-(3,4-dihydro-2H-pyrrol-2-yl), -(CH₂)₄NHCOCH₃, -(CH₂)₄NHCHO, -(CH₂)₃NHCONH₂, -(CH₂)₄NHCONH₂, -CH₂CH₂CH(OH)CH₂NH₂, 4-imidazolylmethyl, 3-indolylmethyl.

In a preferred embodiment, A₁-A₂ group form a dipeptide containing 2 amino acid units, wherein said amino acid units are selected from natural and non-natural amino acids, preferably natural amino acids. Said amino acid unit may be alanine (Ala), cysteine (Cys), aspartic acid (Asp), glutamic acid (Glu), phenylalanine (Phe), glycine (Gly), histidine (His), isoleucine (Ile), lysine (Lys), leucine (Leu), methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucine (Nle), selenocysteine (Sec), pyrrolysine (Pyl), homoserine, homocysteine, and desmethyl pyrrolysine.

In one embodiment, when A₁ represents a proline (Pro) amino acid unit, A₁ is defined by the formula: 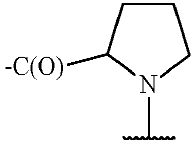 wherein the carbonyl group is attached to the amino group of the para-amino-benzyl moiety and the wavy line indicates the covalent attachment site to the carbonyl group of the A₂ group.

In one embodiment, when A₂ represents a proline (Pro) amino acid unit, A₂ is defined by the formula: 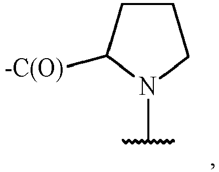 wherein the carbonyl group is attached to the amino group of the A₁ group and the wavy line indicates the covalent attachment site to the X group, Z' group or Z group as defined in the invention.

In a particular embodiment, A₁-A₂ group can represent Val-Cit; Cit-Val; Ala-Ala; Ala-Cit; Cit-Ala; Asn-Cit; Cit-Asn; Cit-Cit; Val-Glu; Glu-Val; Ser-Cit; Cit-Ser; Lys-Cit; Cit-Lys; Asp-Cit; Cit-Asp; Ala-Val; Val-Ala; Phe-Ala; Ala-Phe; Phe-Lys; Lys-Phe; Val-Lys; Lys-Val; Ala-Lys; Lys-Ala; Phe-Cit; Cit-Phe; Leu-Cit; Cit-Leu; Ile-Cit; Cit-Ile; Phe-Arg; Arg-Phe; Cit-Trp; Trp-Cit. In a preferred embodiment, A₁-A₂ group represents Cit-Val or Ala-Val.

Preferably, A₁ represents a -C(O)-CH(R₃)-NH- group in which R₃ represents a -(CH₂)₃-NH-CO-NH₂ group or a methyl group.

Advantageously, A₂ represents a -C(O)-CH(R₄)-NH- group in which R₄ represents an isopropyl group; a 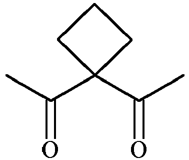 group; a 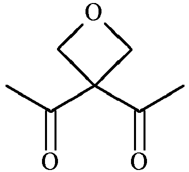 group; or a 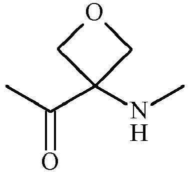 group.

In a preferred embodiment, A₁ represents a -C(O)-CH(R₃)-NH- group in which R₃ represents a -(CH₂)₃-NH-CO-NH₂ group or a methyl group and A₂ represents a -C(O)-CH(R₄)-NH- group in which R₄ represents an isopropyl group.

In another preferred embodiment, A₁ represents a -C(O)-CH(R₃)-NH- group in which R₃ represents a -(CH₂)₃-NH-CO-NH₂ group, and A₂ represents a 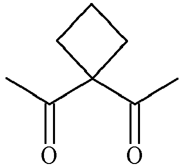 group or a 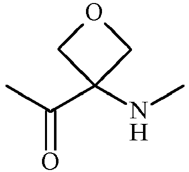 group.

In one embodiment, X represents a hydrogen atom or a protecting group. Preferably, X represents a hydrogen atom, or a protecting group for amino functionality, carbonyl functionality, or carboxyl functionality. More preferably, X represents a hydrogen atom, or a protecting group selected from Fmoc, Boc, or CBz. Even more preferably, X represents a Fmoc protecting group.

Preferred para-amino-benzyl linker compound of Formula (I) are:
- sodium 5-[[(2S)-2-[[(2S)-2-(9H-fluoren-9-ylmethoxycarbonylamino)-3-methyl-butanoyl]amino]-5-ureido-pentanoyl]amino]-2-(hydroxymethyl)benzenesulfonate;
- sodium 5-[[(2S)-2-[[(2S)-2-(9H-fluoren-9-ylmethoxycarbonylamino)-3-methyl-butanoyl]amino]propanoyl]amino]-2-(hydroxymethyl)benzenesulfonate;
- 5-[[(2S)-2-[[(2S)-2-(9H-fluoren-9-ylmethoxycarbonylamino)-3-methyl-butanoyl]amino]propanoyl]amino]-2-(hydroxymethyl)benzenesulfonic acid;
- sodium [5-[[(2S)-2-[[(2S)-2-(9H-fluoren-9-ylmethoxycarbonylamino)-3-methyl-butanoyl]amino]-5-ureido-pentanoyl]amino]-2-(hydroxymethyl)phenyl] methanesulfonate;
- 2-(chloromethyl)-5-[[(2S)-2-[[(2S)-2-(9H-fluoren-9-ylmethoxycarbonylamino)-3-methyl-butanoyl]amino]-5-ureido-pentanoyl]amino]benzenesulfonic acid;
- 2-(chloromethyl)-5-[[(2S)-2-[[(2S)-2-(9H-fluoren-9-ylmethoxycarbonylamino)-3-methyl-butanoyl]amino]propanoyl]amino]benzenesulfonic acid;
- 5-[[(2S)-2-[[(2S)-2-(9H-fluoren-9-ylmethoxycarbonylamino)-3-methyl-butanoyl]amino]-5-ureido-pentanoyl]amino]-2-(iodomethyl)benzenesulfonic acid;
- sodium *N*-{[(9*H*-fluoren-9-yl)methoxy]carbonyl}-L-valyl-*N*⁵-carbarnoyl-*N*-[4-(hydroxymethyl)-3-(2-sulfonatoethyl)phenyl]-L-ornithinamide;
- *N*-{[(9*H*-fluoren-9-yl)methoxy]carbonyl}-L-valyl-*N*⁵-carbamoyl-*N*-[4-(hydroxymethyl)-3-(3-sulfopropyl)phenyl]-L-ornithinamide.

The invention relates also to a process for the preparation of para-amino-benzyl linker compounds of Formula (I), which process is characterized in that there is used as starting material the compound of Formula (IV): 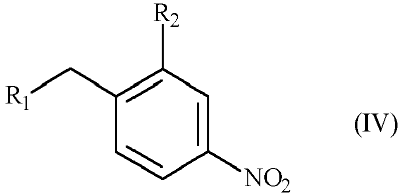 wherein R₁ and R₂ are as defined for Formula (I),
which is subjected to a reduction reaction to yield the compound of Formula (V): 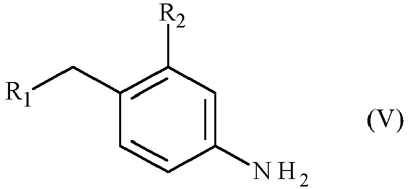 wherein R₁ and R₂ are as defined herein before,
compound of Formula (V) which is further subjected to coupling with the compound of Formula (VI):

HO-A₁-P (VI)

wherein P is an amino-protecting group and A₁ is as defined in Formula (I),
to yield the compound of Formula (VII) 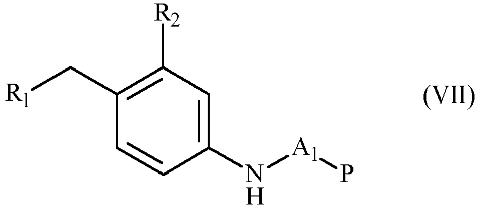 wherein A₁, P, R₁ and R₂ are as defined herein before,
compound of Formula (VII) which is further subjected to coupling with the compound of Formula (VIII):

HO-A₂-X (VIII)

wherein X and A₂ are as defined in Formula (I),
to yield the para-amino-benzyl linker compound of Formula (I).

An alternative process for the preparation of para-amino-benzyl linker compounds of Formula (I) is characterized in that there is used as starting material the compound of Formula (V), obtained from compound of Formula (IV), which is subjected to coupling with the compound of Formula (IX):

HO-A₁-A₂-X (IX)

wherein A₁, A₂ and X are as defined in Formula (I),
to yield the para-amino-benzyl linker compound of Formula (I).

In one embodiment, para-amino-benzyl linker compound of Formula (I) obtained at the end of the above-mentioned processes may be further purified according to a conventional separation technique, which are converted, if desired, into their addition salts with a pharmaceutically acceptable acid or base and which may be separated into their isomers according to a conventional separation technique, it being understood that at any moment considered appropriate during the course of the process described above, some groups (hydroxy, amino...) of the starting reagents or of the synthesis intermediates can be protected, subsequently deprotected and functionalized, as required by the synthesis. The compounds of Formulae (IV), (VI), (VIII) and (IX) are either commercially available or can be obtained by the person skilled in the art using conventional chemical reactions described in the literature.

The invention relates also to the use of para-amino-benzyl linker compounds of Formula (I), in the preparation of an antibody-drug conjugate. More particularly, the invention relates to the use of para-amino-benzyl linker compound of Formula (I) selected from:
- sodium 5-[[(2S)-2-[[(2S)-2-(9H-fluoren-9-ylmethoxycarbonylamino)-3-methyl-butanoyl]amino]-5-ureido-pentanoyl]amino]-2-(hydroxymethyl)benzenesulfonate,
- sodium 5-[[(2S)-2-[[(2S)-2-(9H-fluoren-9-ylmethoxycarbonylamino)-3-methyl-butanoyl]amino]propanoyl]amino]-2-(hydroxymethyl)benzenesulfonate,
- 5-[[(2S)-2-[[(2S)-2-(9H-fluoren-9-ylmethoxycarbonylamino)-3-methyl-butanoyl]amino]propanoyl]amino]-2-(hydroxymethyl)benzenesulfonic acid,
- sodium [5-[[(2S)-2-[[(2S)-2-(9H-fluoren-9-ylmethoxycarbonylamino)-3-methyl-butanoyl]amino]-5-ureido-pentanoyl]amino]-2-(hydroxymethyl)phenyl]methanesulfonate,
- 2-(chloromethyl)-5-[[(2S)-2-[[(2S)-2-(9H-fluoren-9-ylmethoxycarbonylamino)-3-methyl-butanoyl]amino]-5-ureido-pentanoyl]amino]benzenesulfonic acid, or
- 2-(chloromethyl)-5-[[(2S)-2-[[(2S)-2-(9H-fluoren-9-ylmethoxycarbonylamino)-3-methyl-butanoyl]amino]propanoyl]amino]benzenesulfonic acid,
- 5-[[(2S)-2-[[(2S)-2-(9H-fluoren-9-ylmethoxycarbonylamino)-3-methyl-butanoyl]amino]-5-ureido-pentanoyl]amino]-2-(iodomethyl)benzenesulfonic acid,
- sodium *N*-{[(9*H*-fluoren-9-yl)methoxy]carbonyl}-L-valyl-*N*⁵-carbamoyl-*N*-[4-(hydroxymethyl)-3-(2-sulfonatoethyl)phenyl]-L-ornithinamide, or
- *N*-{[(9*H*-fluoren-9-yl)methoxy]carbonyl}-L-valyl-*N*⁵-carbamoyl-*N*-[4-(hydroxymethyl)-3-(3-sulfopropyl)phenyl]-L-ornithinamide,
in the preparation of an antibody-drug conjugate.

In a second aspect, the invention provides a Linker-Drug compound of Formula (II): 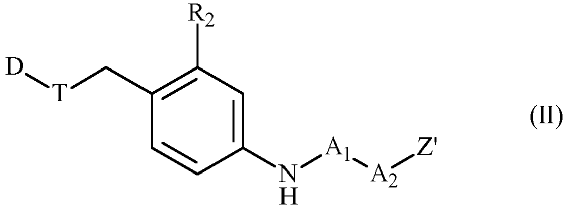 wherein:
- D represents a drug moiety;
- T is a bond, -O-C(O)-N(CH₃)-CH₂-CH₂-N(CH₃)-C(O)-*, -O-*, -NR₅-*, -NRs-C(O)-*, or -O-C(O)-*, wherein the * indicates the point of attachment to D;
- R₂ represents a -S(O)₂(OH) group, a -S(O)₂(O⁻M⁺) group, a linear or branched -(C₁-C₄)alkyl-S(O)z(OH) group, a linear or branched -(C₁-C₄)alkyl-S(O)₂(O⁻M⁺) group, a linear or branched -halo(C₁-C₄)alkyl-S(O)₂(OH) group, or a linear or branched -halo(C₁-C₄)alkyl-S(O)₂(O⁻M⁺) group;
- A₁ represents a -C(O)-CH(R₃)-NH- group;
- A₂ represents a -C(O)-CH(R₄)-NH- group, a 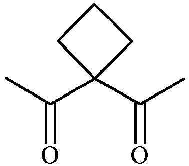 group, a 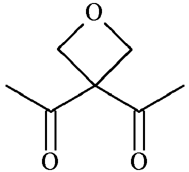 group, or a 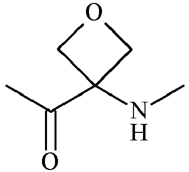 group;
- R₃ and R₄, independently of one another, represent the side chain of an amino acid;
- R₅ represents a hydrogen atom or a (C₁-C₄)alkyl group;
- Z' represents a Spacer Unit precursor;
- M⁺ represents a pharmaceutically acceptable monovalent cation.

In one embodiment in the second aspect of the invention, D is a drug moiety comprising a nitrogen atom or an oxygen atom, wherein D is directly connected to T via the said nitrogen atom or the oxygen atom of the drug moiety. In some embodiment, D is a quaternized tertiary amine-containing drug moiety. In a particular embodiment, D is monomethyl auristatin E (IUPAC Name: (*S*)-*N*-((3*R*,4*S*,5*S*)-1-((*S*)-2-((1*R*,2*R*)-3-(((1*S*,2*R*)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-*N*,3-dimethyl-2-((*S*)-3-methyl-2-(methylamino)butanamido)butanamide or MMAE), auristatin E (IUPAC Name: (2S)-2-[[(2*S*)-2-(dimethylamino)-3-methyl-butanoyl]amino]-*N*-[(1*S*,2*R*)-4-[(2,*S*)-2-[(1*R*,2*R*)-3-[[(1*R*,2*S*)-2-hydroxy-1-methyl-2-phenyl-ethyl]amino]-1-methoxy-2-methyl-3-oxopropyl]pyrrolidin-1-yl]-2-methoxy-1-[(1*S*)-1-methylpropyl]-4-oxo-butyl]-N,3-dimethylbutanamide), SN-38 (IUPAC Name: (4*S*)-4,11-diethyl-4,9-dihydroxy-1*H-*pyrano[3',4':6,7]indolizino[1,2-*b*]quinoline-3,14(4*H*,12*H*)-dione), or doxorubicin (IUPAC Name: (1*S*,3*S*)-3,5,12-trihydroxy-3-(hydroxyacetyl)-10-methoxy-6,11-dioxo-1,2,3,4,6,11-hexahydrotetracen-1-yl 3-amino-2,3,6-trideoxy-α-L-*lyxo*-hexopyranoside).

Preferably, T is a bond or -O-C(O)-*, wherein the * indicates the point of attachment to D. In some embodiment, when T is a bond, D is a quaternized tertiary amine-containing drug moiety.

Preferably in the second aspect of the invention, R₂ represents a -S(O)₂(OH) group, a -S(O)₂(O⁻M⁺) group, a -CH₂-S(O)₂(OH) group, a -CH₂-S(O)₂(O⁻M⁺) group, a -CH₂-CH₂-S(O)₂(OH) group, a -CH₂-CH₂-S(O)₂(O⁻M⁺) group, a -CH₂-CH₂-CH₂-S(O)₂(OH) group, or a -CH₂-CH₂-CH₂-S(O)₂(O⁻M⁺) group. In a preferred embodiment of the second aspect of the invention, M⁺ represents Na⁺.

Advantageously in the second aspect of the invention, A₁ represents a -C(O)-CH(R₃)-NH-group in which R₃ represents a -(CH₂)₃-NH-CO-NH₂ group or a methyl group.

In a preferred embodiment of the second aspect of the invention, A₂ represents a -C(O)-CH(R₄)-NH- group in which R₄ represents an isopropyl group; a 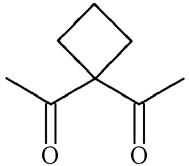 group; a 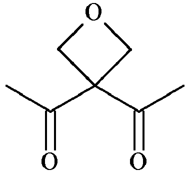 group; or a 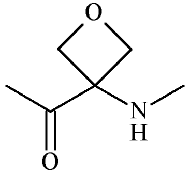 group.

In another preferred embodiment of the second aspect of the invention, A₁ represents a -C(O)-CH(R₃)-NH- group in which R₃ represents a -(CH₂)₃-NH-CO-NH₂ group, and A₂ represents a 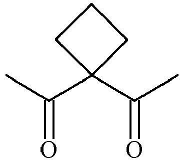 group, or a 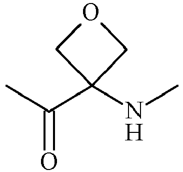 group.

In another preferred embodiment of the second aspect of the invention, A₁ represents a -C(O)-CH(R₃)-NH- group and A₂ represents a -C(O)-CH(R₄)-NH- group, in which R₃ and R₄, independently of one another, represent the side chain of an amino acid. Particularly, when R₃ and R₄ represent a side chain of an amino acid, the side chain-bearing carbon is L configuration. In a preferred embodiment, A₁ represents a -C(O)-CH(R₃)-NH- group in which R₃ represents a -(CH₂)₃-NH-CO-NH₂ group or a methyl group and A₂ represents a -C(O)-CH(R₄)-NH- group in which R₄ represents an isopropyl group. Preferably in the second aspect of the invention, A₁-A₂ group represents Cit-Val or Ala-Val.

Preferably, Spacer Unit precursor Z' is selected from: 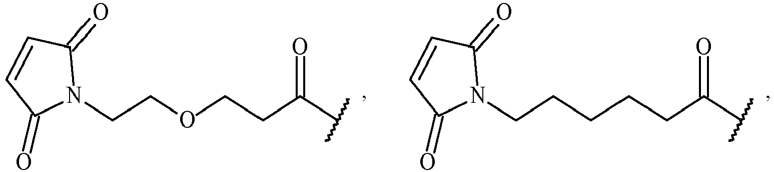 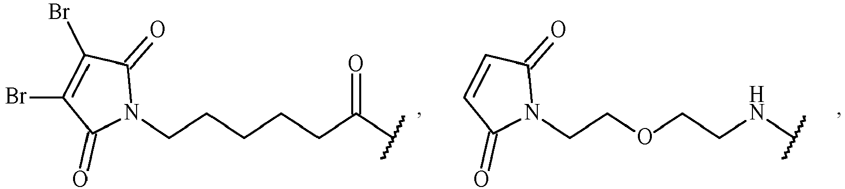 or 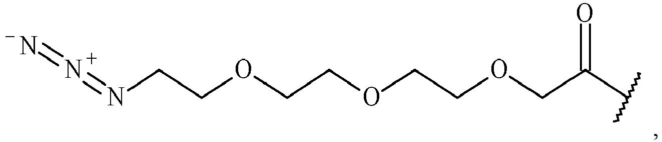 wherein the wavy line indicates the covalent attachment site to the N-terminus or the carbonyl group of A₂ group.

Preferred Linker-Drug compound of Formula (II) are selected from: 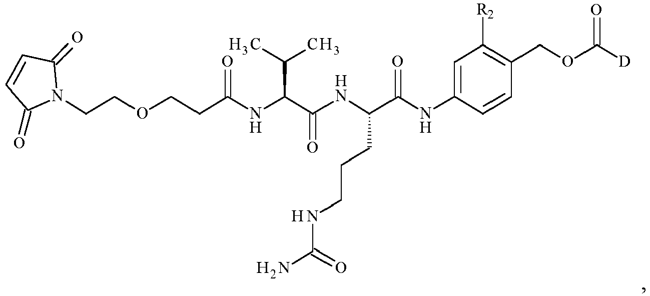 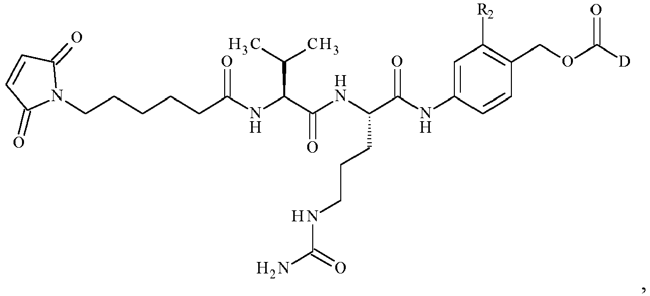 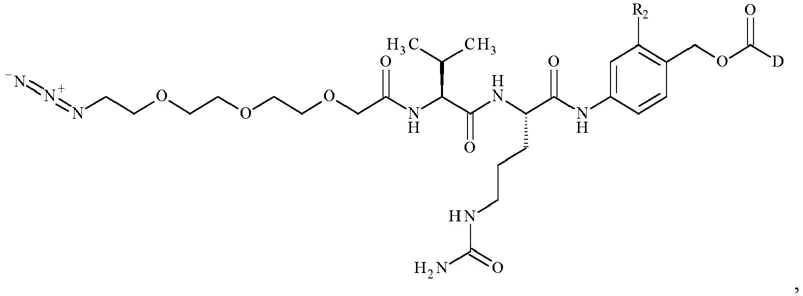 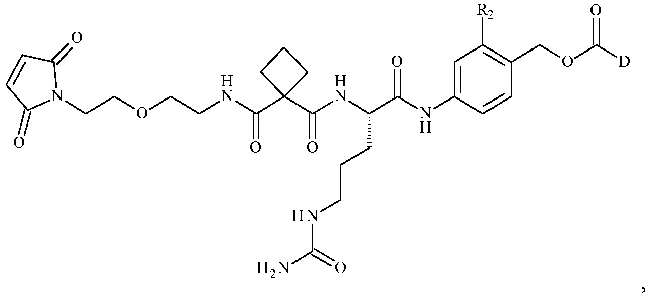 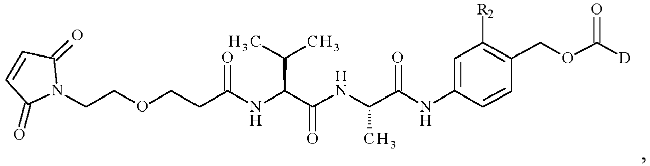 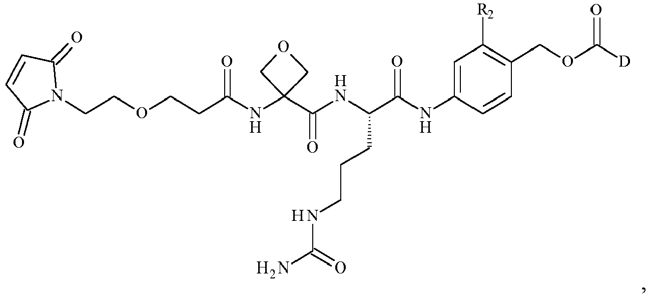 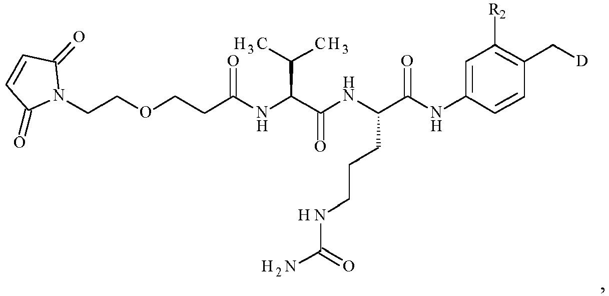 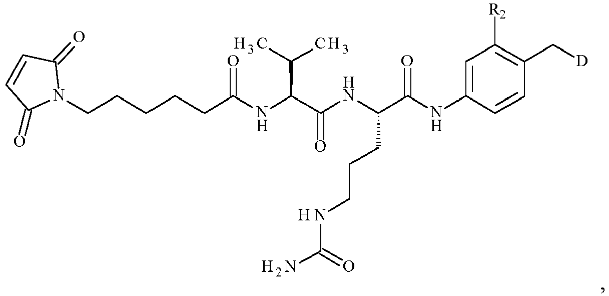 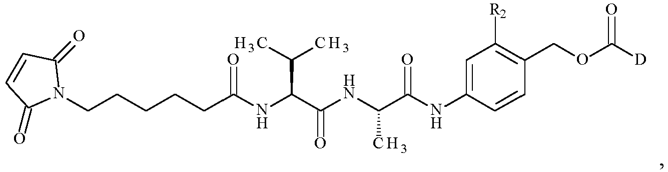 and 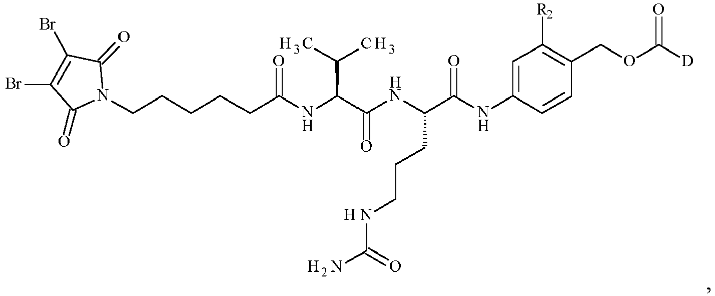 wherein R₂ and D are as defined herein before.

More preferred Linker-Drug compound of Formula (II) are selected from: 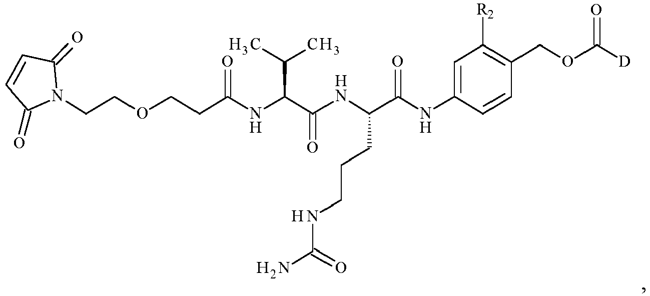 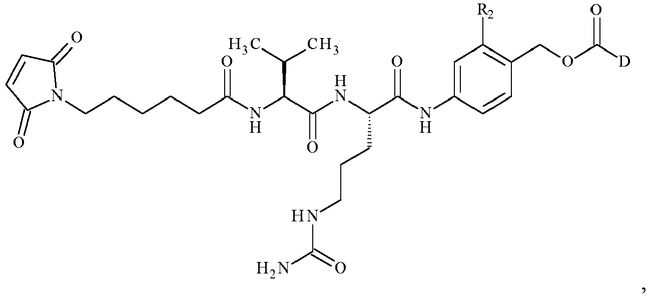 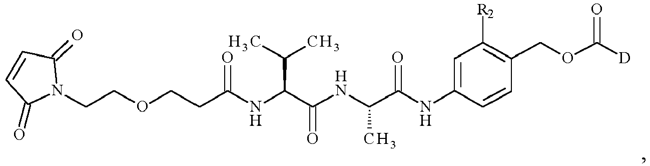 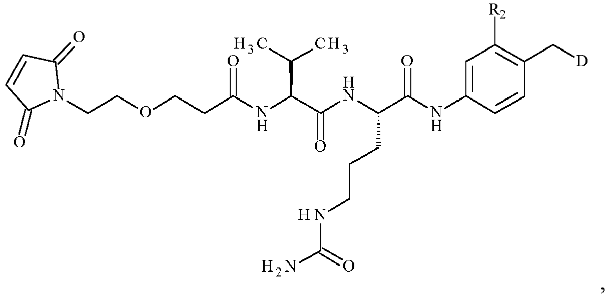 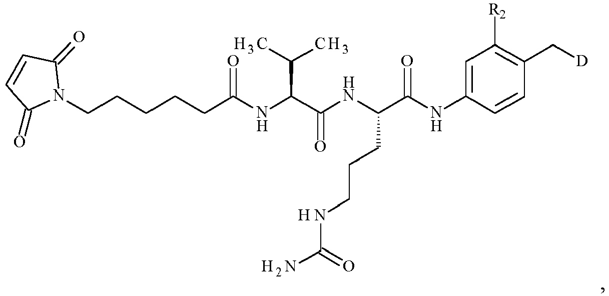 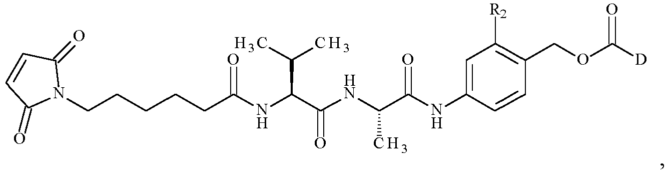 and 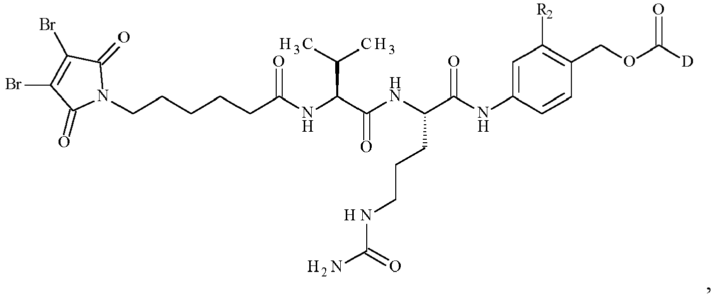 wherein R₂ and D are as defined herein before.

In a preferred embodiment, Linker-Drug compound of Formula (II) are selected from: 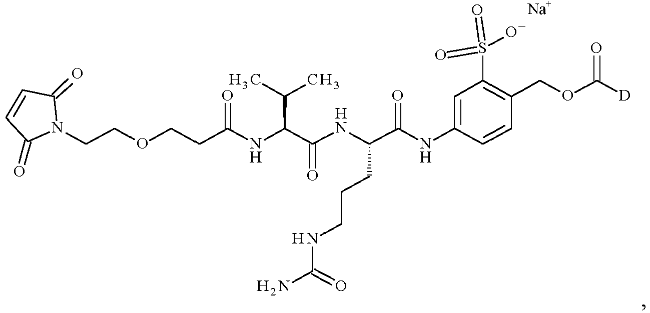 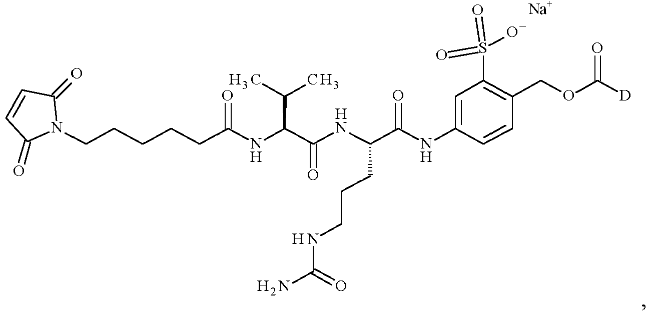 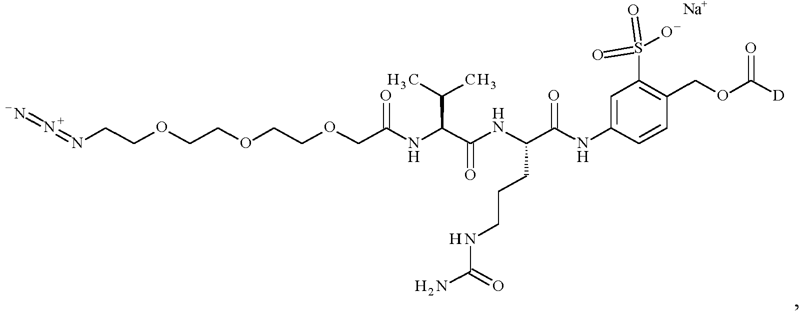 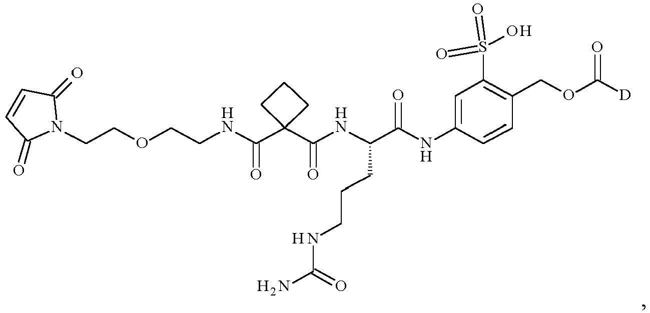 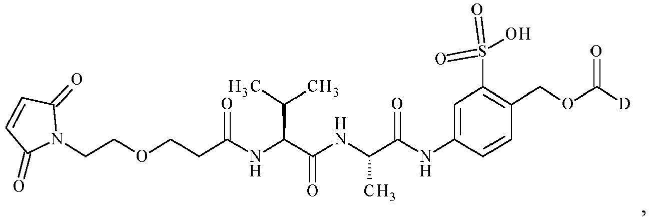 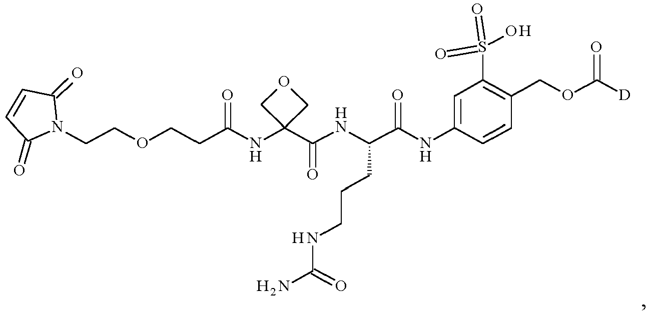 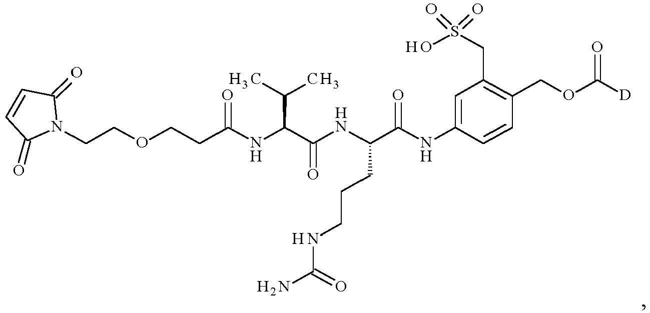 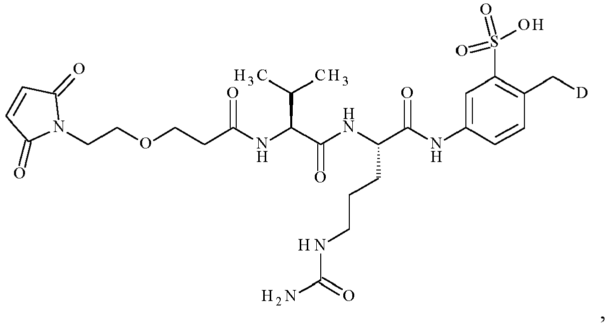 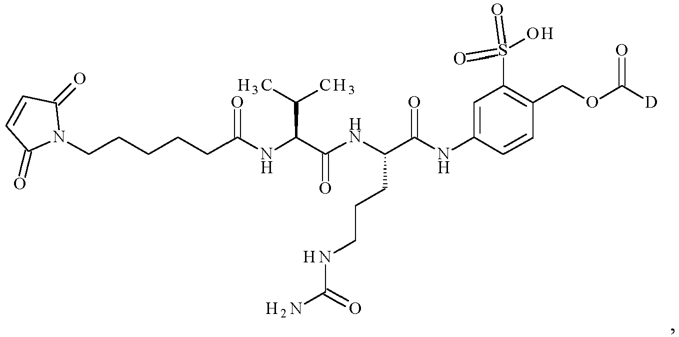 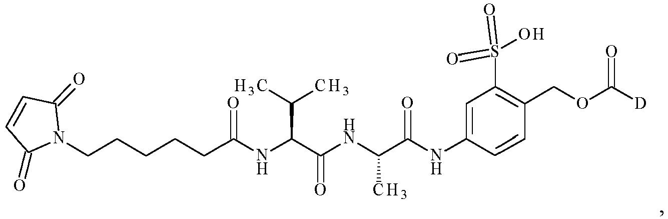 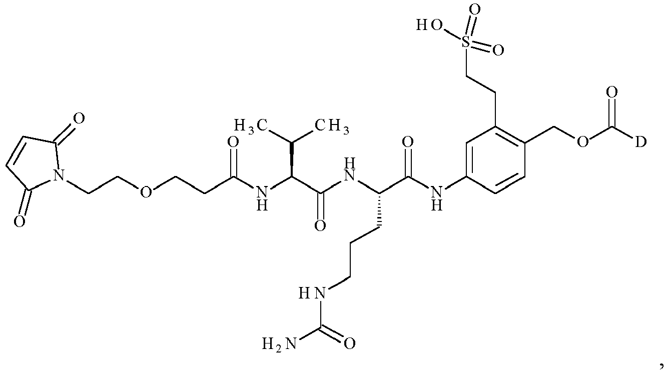 and 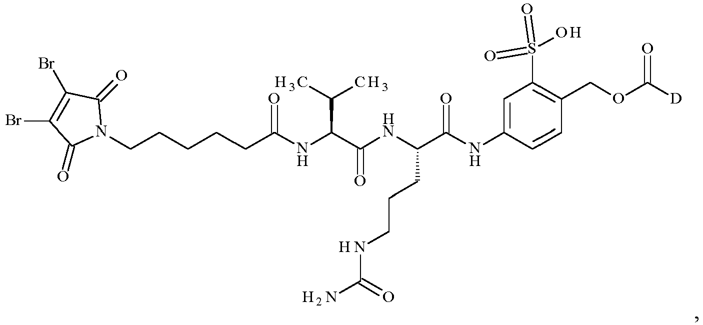 and 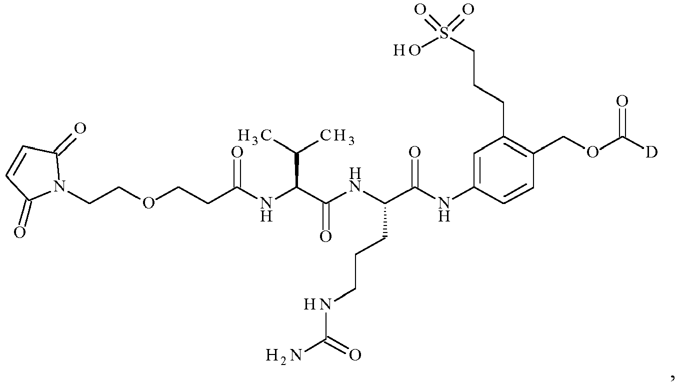 wherein D is as defined herein before.

In a particular embodiment, preferred Linker-Drug compounds of Formula (II) are:
- sodium 5-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl] carbamoyl]-2-methyl-propyl]-methyl-carbamoyl]oxymethyl]benzenesulfonate;
- sodium 5-[[(2S)-2-[[(2S)-2-[6-(2,5-dioxopyrrol-1-yl)hexanoylamino]-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonate;
- sodium 5-[[(2S)-2-[[(2S)-2-[[2-[2-[2-(2-azidoethoxy)ethoxy]ethoxy]acetyl]amino]-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl] amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonate;
- 5-[[(2S)-2-[[1-[2-[2-(2,5-dioxopyrrol-1-yl)ethoxy]ethylcarbamoyl] cyclobutanecarbonyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl] amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonic acid;
- sodium 5-[[(2S)-2-[[(2S)-2-[3-[2-(2,5-dioxopyrrol-1-yl)ethoxy]propanoylamino]-3-methyl-butanoyl]amino]propanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxobutyl]-methyl-carbamoyl]-2-methyl-propyl]carbamoyl]-2-methyl-propyl]-methylcarbamoyl]oxymethyl]benzenesulfonate;
- 5-[[(2S)-2-[[(2S)-2-[3-[2-(2,5-dioxopyrrol-1-yl)ethoxy]propanoylamino]-3-methyl-butanoyl]amino]propanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxobutyl]-methyl-carbamoyl]-2-methyl-propyl]carbamoyl]-2-methyl-propyl]-methylcarbamoyl]oxymethyl]benzenesulfonic acid;
- 5-[[(2S)-2-[[3-[2-[2-(2,5-dioxopyrrol-1-yl)ethoxy]ethylcarbamoyl]oxetane-3-carbonyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonic acid;
- [5-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl] amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]phenyl]methanesulfonic acid;
- [4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]-2-sulfo-phenyl]methyl-[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1 S)-1-methylpropyl]-4-oxo-butyl]-methyl-carbamoyl]-2-methylpropyl]carbamoyl]-2-methyl-propyl]-dimethyl-ammonium; 2,2,2-trifluoroacetate;
- *N*-({[4-({*N*-[6-(2,5-dioxo-2,5-dihydro-1*H*-pyrrol-1-yl)hexanoyl]-L-valyl-L-alanyl}amino)-2-sulfophenyl]methoxy}carbonyl)-*N*-methyl-L-valyl-*N*-[(3*R*,4*S*,5*S*)-1-{(2*S*)-2-[(1*R*,2*R*)-3-{[(1*S*,2*R*)-1-hydroxy-1-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamide;
- *N*-[6-(2,5-dioxo-2,5-dihydro-1*H*-pyrrol-1-yl)hexanoyl]-L-valyl-*N*⁵-carbamoyl-*N-*(4-{[({[(4*S*)-4,11-diethyl-9-hydroxy-3,14-dioxo-3,4,12,14-tetrahydro-1*H-*pyrano[3',4':6,7]indolizino[1,2-*b*]quinolin-4-yl]oxy}carbonyl)oxy]methyl}-3-sulfophenyl)-L-ornithinamide;
- (1*S*,3*S*)-3,5,12-trihydroxy-3-(hydroxyacetyl)-10-methoxy-6,11-dioxo-1,2,3,4,6,11-hexahydrotetracen-1-yl2,3,6-trideoxy-3-[({[4-({*N*-[6-(2,5-dioxo-2,5-dihydro-1*H-*pyrrol-1-yl)hexanoyl]-L-valyl-*N*⁵-carbamoyl-L-ornithyl}amino)-2-sulfophenyl]methoxy}carbonyl)amino]-α-L-*lyxo*-hexopyranoside;
- *N*-{3-[2-(2,5-dioxo-2,5-dihydro-1*H*-pyrrol-1-yl)ethoxy]propanoyl}-L-valyl-*N*-{4-[(5*S*,8*S*,11*S*,12*R*)-11-[(2*S*)-butan-2-yl]-12-(2-{(2*S*)-2-[(1*R*,2*R*)-3-{[(1*S*,2*R*)-1-hydroxy-1-phenylpropan-2-yl]amino}-1-methoxy-2-methyl-3-oxopropyl] pyrrolidin-1-yl}-2-oxoethyl)-4,10-dimethyl-3,6,9-trioxo-5,8-di(propan-2-yl)-2,13-dioxa-4,7,10-triazatetradecan-1-yl]-3-(2-sulfoethyl)phenyl}-*N*⁵-carbamoyl-L-ornithinamide;
- 5-[[(2S)-2-[[(2S)-2-[6-(3,4-dibromo-2,5-dioxo-pyrrol-1-yl)hexanoylamino]-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl] amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonic acid.

The invention relates also to a process for the preparation of Linker-Drug compounds of Formula (II), which process is characterized in that there is used as starting material the compound of Formula (I): 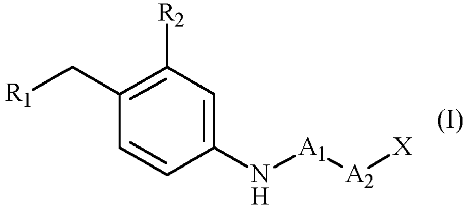 wherein R₁, R₂, A₁, A₂ and X are as defined hereinbefore
which is then modified to yield the compound of Formula (X): 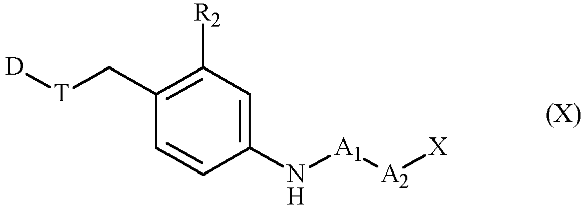 wherein A₁, A₂, D, R₂, T and X are as defined in Formula (II),
compound of Formula (X) which is further subjected, after removing the protecting group X, to coupling with the compound of Formula (XI):

HO-Z' (XI)

wherein Z' is as defined in Formula (II),
to yield the Linker-Drug compound of Formula (II).

An alternative process for the preparation of Linker-Drug compounds of Formula (II) is characterized in that there is used as starting material the compound of Formula (VII), obtained in two steps from compound of Formula (IV), which is subjected to coupling with group D-T to yield the compound of Formula (XII): 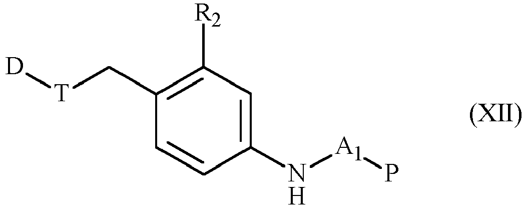 wherein A₁, D, P, R₂, and T are as defined herein before,
compound of Formula (XII) which is further subjected to coupling with the compound of Formula (XIII):

HO-A₂-Z' (XIII)

wherein Z and A₂ are as defined in Formula (I),
to yield the Linker-Drug compound of Formula (II).

In one embodiment, Linker-Drug compound of Formula (II) obtained at the end of the above-mentioned processes may be further purified according to a conventional separation technique, which are converted, if desired, into their addition salts with a pharmaceutically acceptable acid or base and which may be separated into their isomers according to a conventional separation technique, it being understood that at any moment considered appropriate during the course of the process described above, some groups (hydroxy, amino...) of the starting reagents or of the synthesis intermediates can be protected, subsequently deprotected and functionalized, as required by the synthesis. The compounds of Formulae (IV), (XI) and (XIII) are either commercially available or can be obtained by the person skilled in the art using conventional chemical reactions described in the literature.

The invention relates also to the use of Linker-Drug compound of Formula (II) in the preparation of an antibody-drug conjugate.

In a third aspect of the invention, there is provided an antibody-drug conjugate (ADC) of Formula (III): 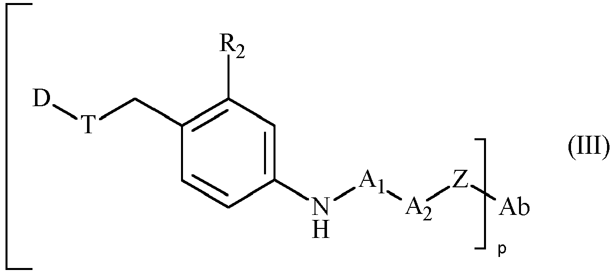 wherein
- Ab represents an antibody or an antigen-binding fragment thereof;
- D is a drug moiety;
- T is a bond, -O-C(O)-N(CH₃)-CH₂-CH₂-N(CH₃)-C(O)-*, -O-*, -NR₅-*, -NRs-C(O)-*, or -O-C(O)-*, wherein the * indicates the point of attachment to D;
- Z represents a Spacer Unit;
- A₁ represents a -C(O)-CH(R₃)-NH- group;
- A₂ represents a -C(O)-CH(R₄)-NH- group, a 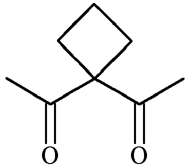 group, a 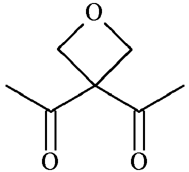 group, or a 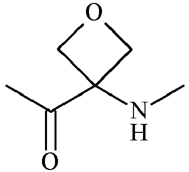 group;
- R₂ represents a -S(O)₂(OH) group, a -S(O)₂(O⁻M⁺) group, a linear or branched -(C₁-C₄)alkyl-S(O)₂(OH) group, a linear or branched -(C₁-C₄)alkyl-S(O)₂(O⁻M⁺) group, a linear or branched -halo(C₁-C₄)alkyl-S(O)₂(OH) group, or a linear or branched -halo(C₁-C₄)alkyl-S(O)₂(O⁻M⁺) group;
- R₃ and R₄, independently of one another, represent the side chain of an amino acid;
- R₅ represents a (C₁-C₄)alkyl group;
- M⁺ represents a pharmaceutically acceptable monovalent cation; and
- p is an integer from 1 to 8.

In one embodiment in the third aspect of the invention, D is a drug moiety comprising a nitrogen atom or an oxygen atom, wherein D is directly connected to T via the said nitrogen atom or the oxygen atom of the drug moiety. In some embodiment in the third aspect of the invention, D is a quaternized tertiary amine-containing drug moiety. In a particular embodiment in the third aspect of the invention, D is monomethyl auristatin E (IUPAC Name: (*S*)-*N*-((3*R*,4*S*,5*S*)-1-((*S*)-2-((1*R*,2*R*)-3-(((1*S*,2*R*)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-*N*,3-dimethyl-2-((*S*)-3-methyl-2-(methylamino)butanamido)butanamide or MMAE), auristatin E (IUPAC Name: (2*S*)-2-[[(2*S*)-2-(dimethylamino)-3-methyl-butanoyl]amino]-*N*-[(1*S*,2*R*)-4-[(2*S*)-2-[(1*R*,2*R*)-3-[[(1*R*,2*S*)-2-hydroxy1-methyl-2-phenyl-ethyl] amino]-1-methoxy-2-methyl-3 -oxo-propyl]pyrrolidin-1-yl]-2-methoxy-1-[(1*S*)-1-methylpropyl]-4-oxo-butyl]-N,3-dimethyl-butanamide), SN-38 (IUPAC Name: (4*S*)-4,11-diethyl-4,9-dihydroxy-1*H*-pyrano[3',4':6,7]indolizino[1,2-*b*]quinoline-3,14(4*H*,12*H*)-dione), or doxorubicin (IUPAC Name: (1*S*,3*S*)-3,5,12-trihydroxy-3-(hydroxyacetyl)-10-methoxy-6,11-dioxo-1,2,3,4,6,11-hexahydrotetracen-1-yl 3-amino-2,3,6-trideoxy-α-L-*lyxo*-hexopyranoside).

Preferably in the third aspect of the invention, T is a bond or -O-C(O)-*, wherein the * indicates the point of attachment to D. In some embodiment in the third aspect of the invention, when T is a bond, D is a quaternized tertiary amine-containing drug moiety.

Preferably in the third aspect of the invention, R₂ represents a -S(O)₂(OH) group, a -S(O)₂(O⁻M⁺) group, a -CH₂-S(O)₂(OH) group, a -CH₂-S(O)₂(O⁻M⁺) group, a -CH₂-CH₂-S(O)₂(OH) group, a -CH₂-CH₂-S(O)₂(O⁻M⁺) group, a -CH₂-CH₂-CH₂-S(O)₂(OH) group, or a -CH₂-CH₂-CH₂-S(O)₂(O⁻M⁺) group. In a preferred embodiment of the third aspect of the invention, M⁺ represents Na⁺.

Advantageously in the third aspect of the invention, A₁ represents a -C(O)-CH(R₃)-NH-group in which R₃ represents a -(CH₂)₃-NH-CO-NH₂ group or a methyl group.

In a preferred embodiment of the third aspect of the invention, A₂ represents a -C(O)-CH(R₄)-NH- group in which R₄ represents an isopropyl group; a 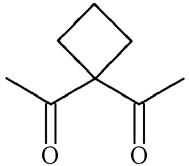 group; a 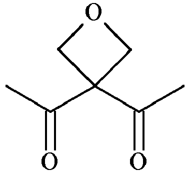 group; or a 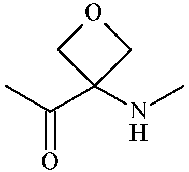 group.

In another preferred embodiment of the third aspect of the invention, A₁ represents a -C(O)-CH(R₃)-NH- group in which R₃ represents a -(CH₂)₃-NH-CO-NH₂ group, and A₂ represents a 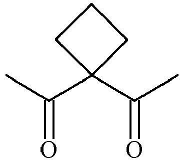 group, or a 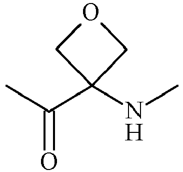 group.

In another preferred embodiment of the third aspect of the invention, A₁ represents a -C(O)-CH(R₃)-NH- group and A₂ represents a -C(O)-CH(R₄)-NH- group, in which R₃ and R₄, independently of one another, represent the side chain of an amino acid. Particularly, when R₃ and R₄ represent a side chain of an amino acid, the side chain-bearing carbon is L configuration. In a preferred embodiment, A₁ represents a -C(O)-CH(R₃)-NH- group in which R₃ represents a -(CH₂)₃-NH-CO-NH₂ group or a methyl group and A₂ represents a -C(O)-CH(R₄)-NH- group in which R₄ represents an isopropyl group. Preferably in the third aspect of the invention, A₁-A₂ group represents Cit-Val or Ala-Val.

In a preferred embodiment, an ADC of Formula (III) is formed from a Linker-Drug compound of Formula (II) selected from: 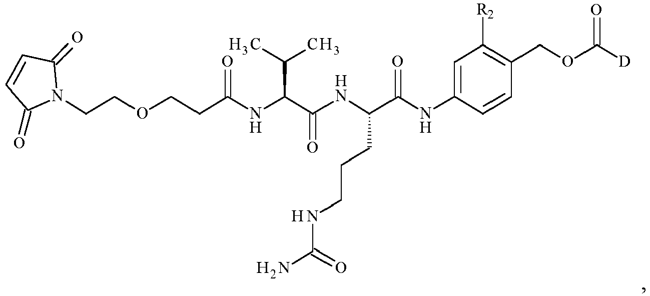 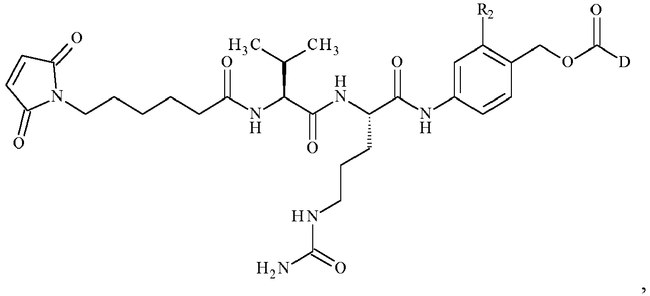 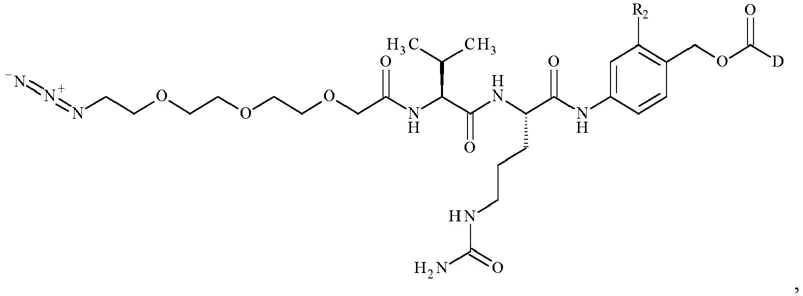 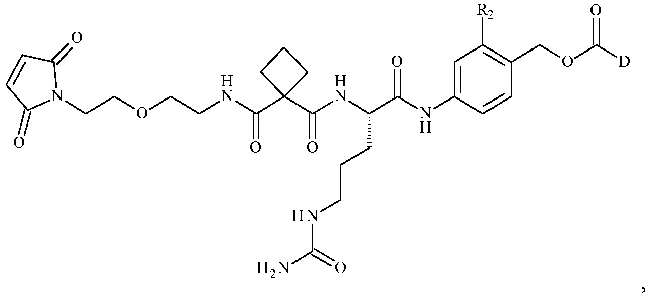 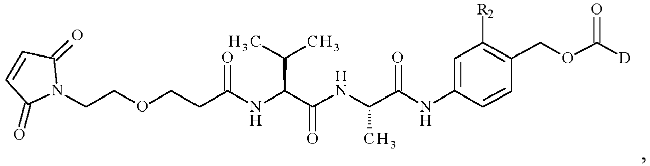 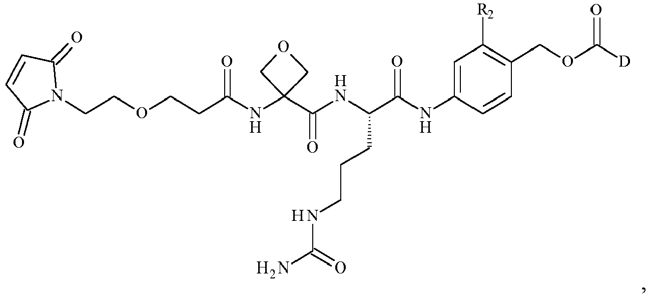 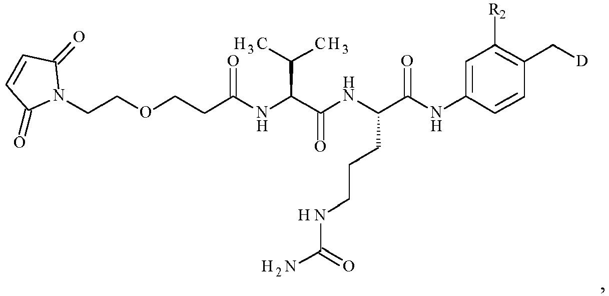 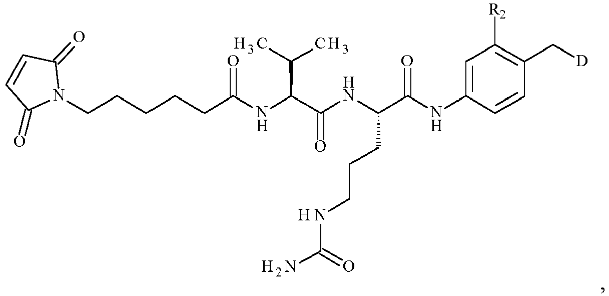 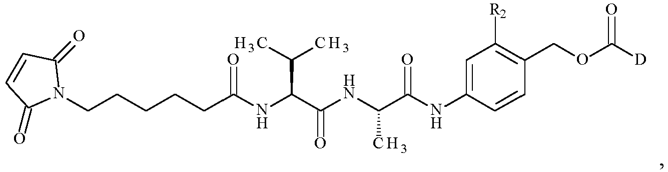 and 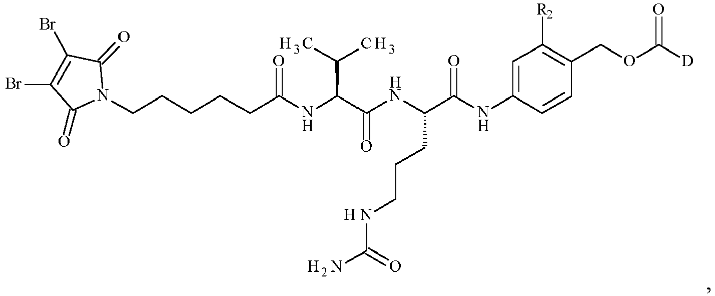 wherein R₂ and D are as defined herein before.

More preferably, an ADC of Formula (III) is formed from a Linker-Drug compound of Formula (II) selected from: 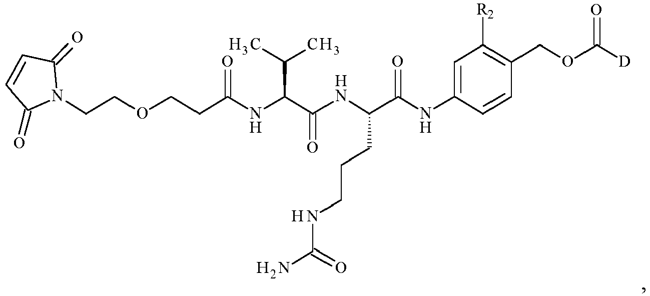 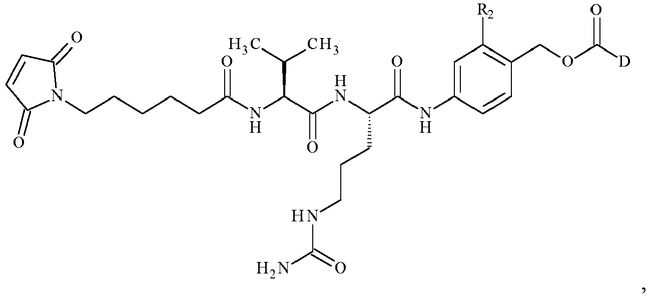 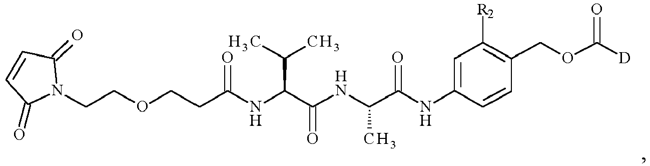 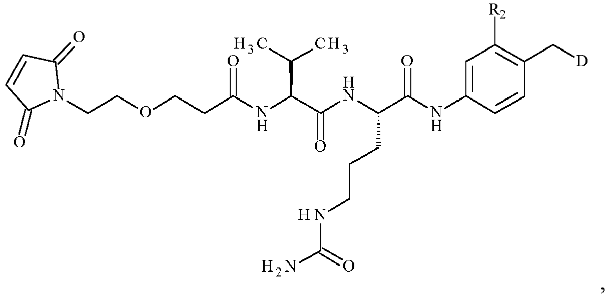 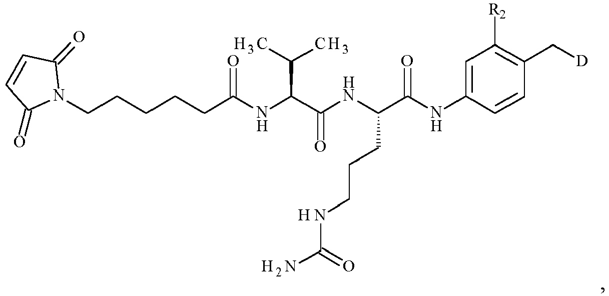 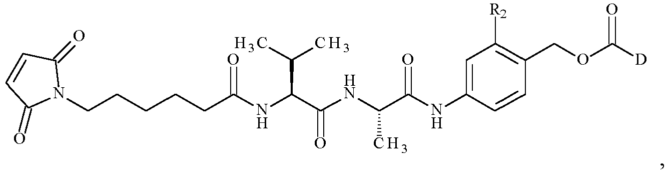 and 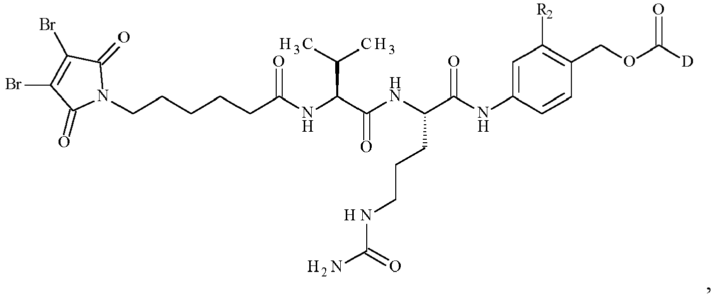 wherein R₂ and D are as defined herein before.

In a preferred embodiment, an ADC of Formula (III) is formed from a Linker-Drug compound of Formula (II) selected from: 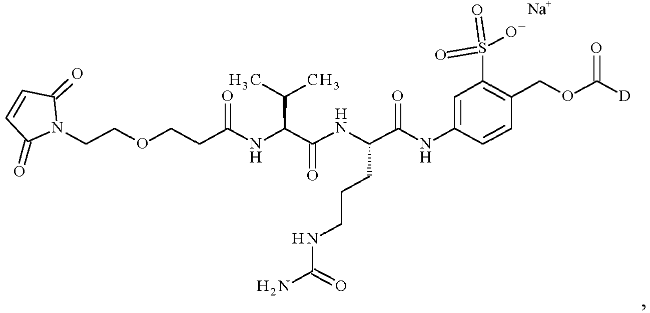 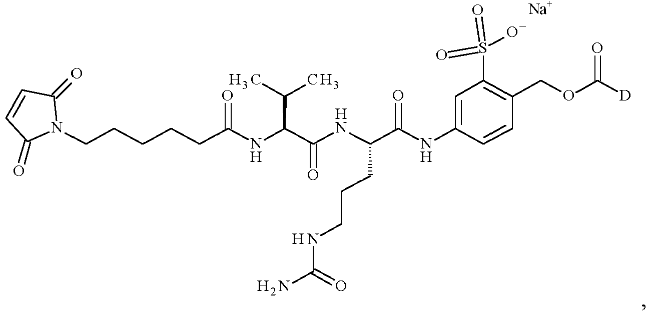 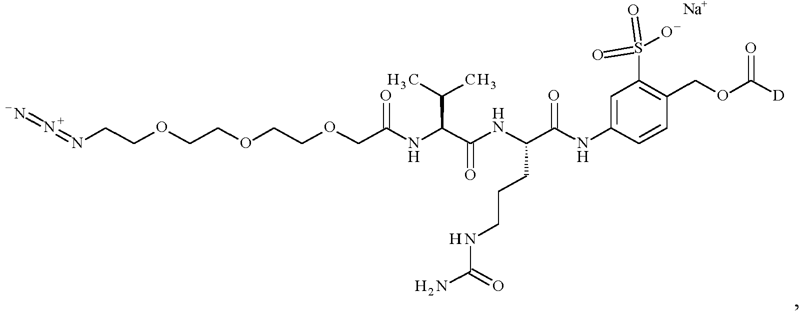 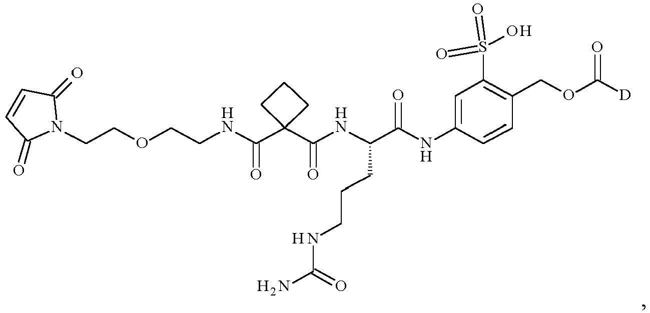 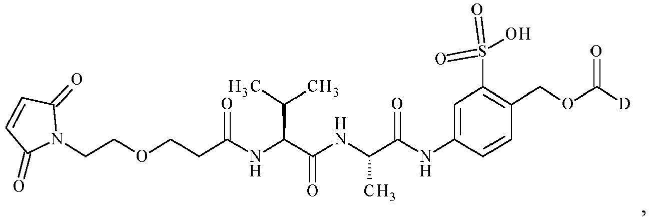 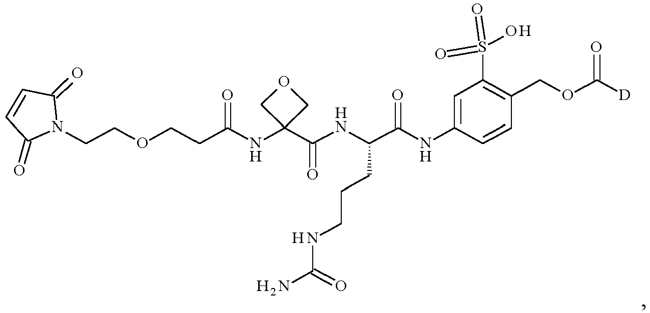 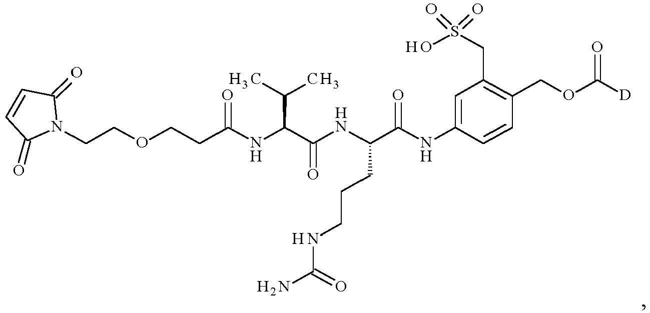 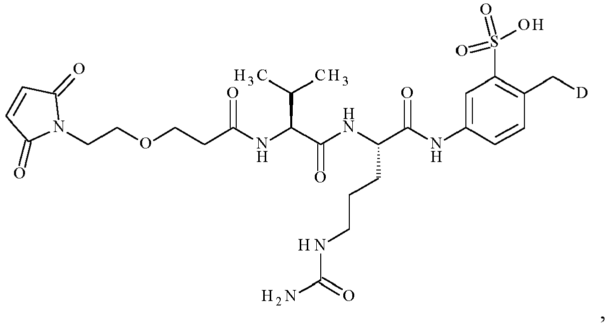 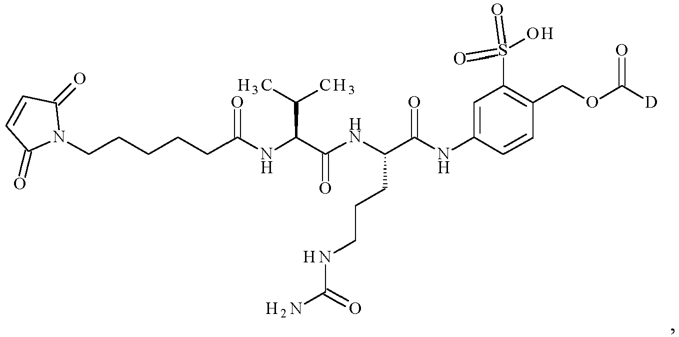 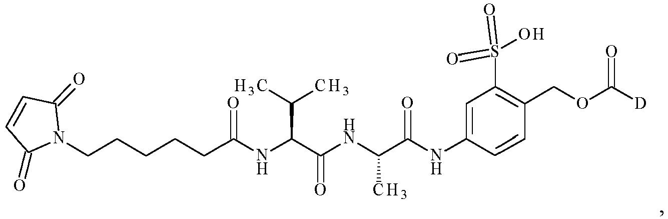 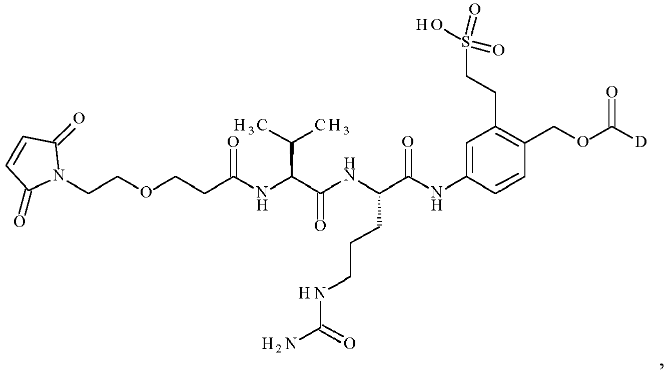 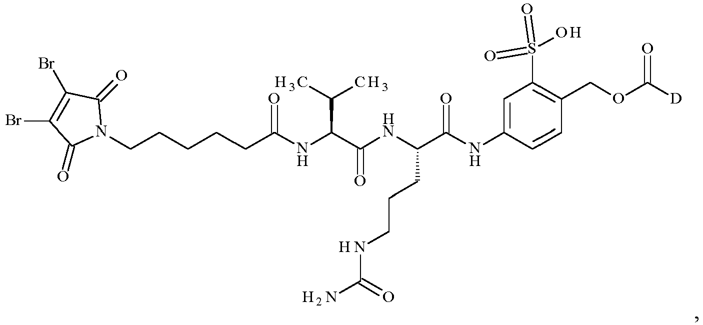 and 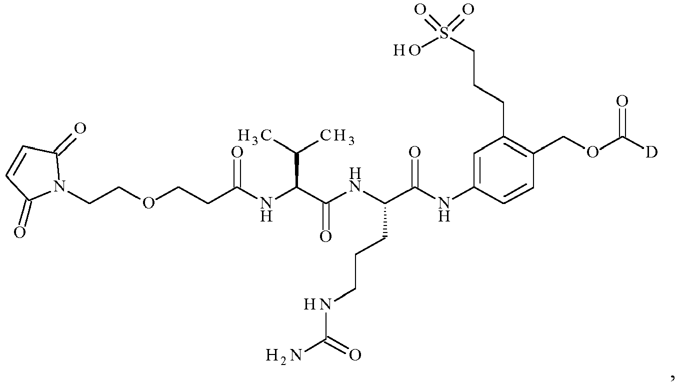 wherein D is as defined herein before.

In a particular embodiment, the invention provides an antibody-drug conjugate of Formula (III) wherein 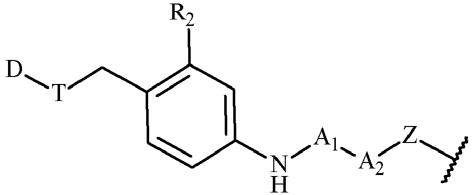 comprises a formula selected from: 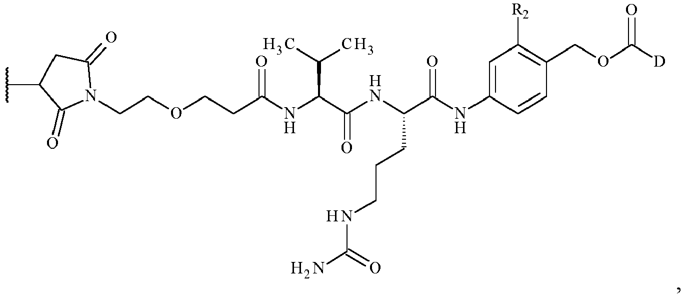 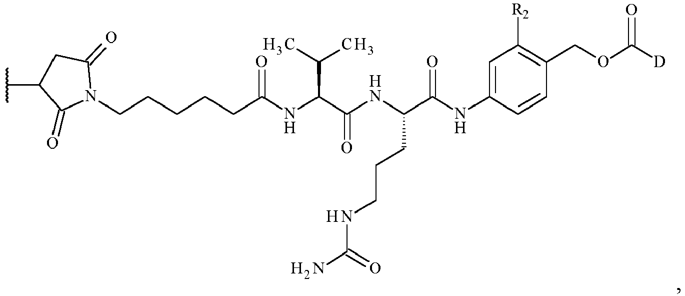 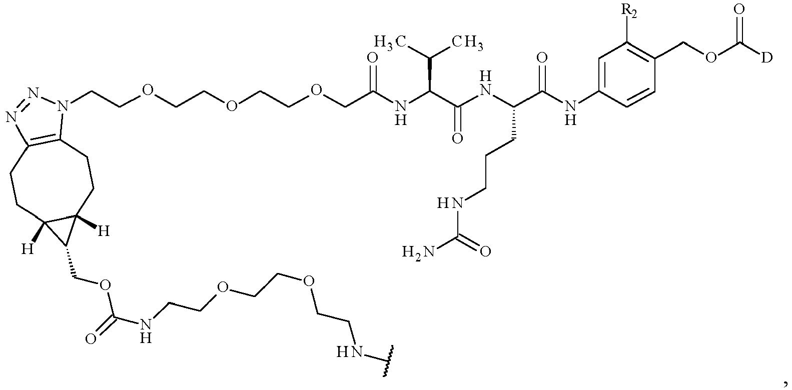 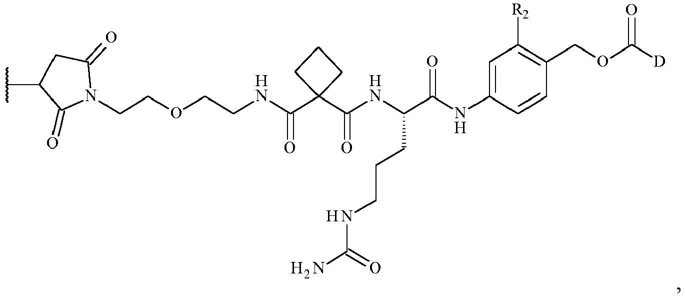 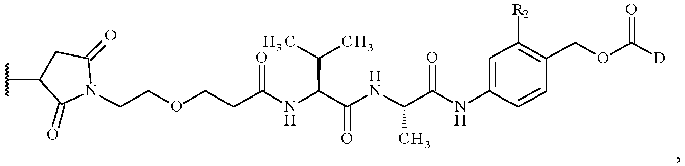 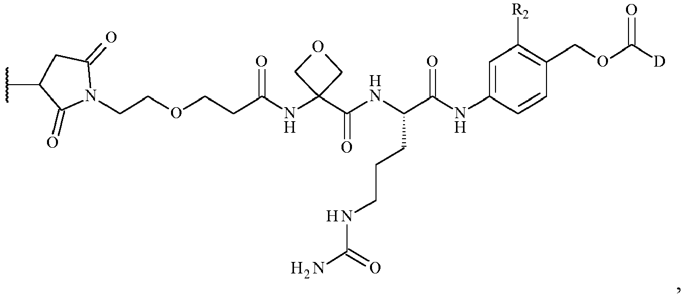 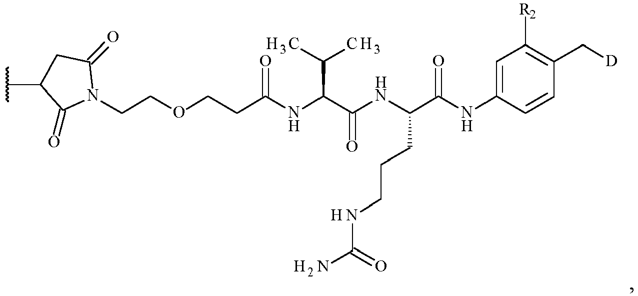 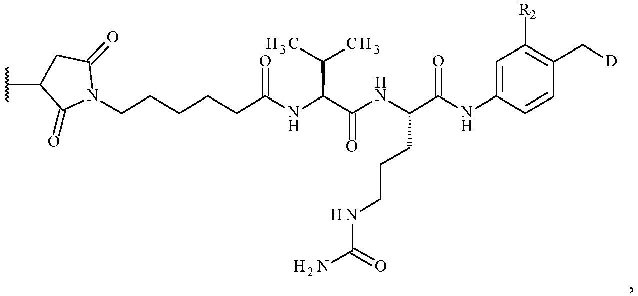 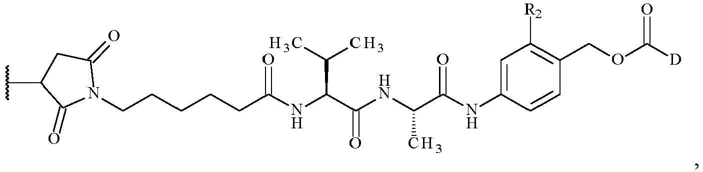 and 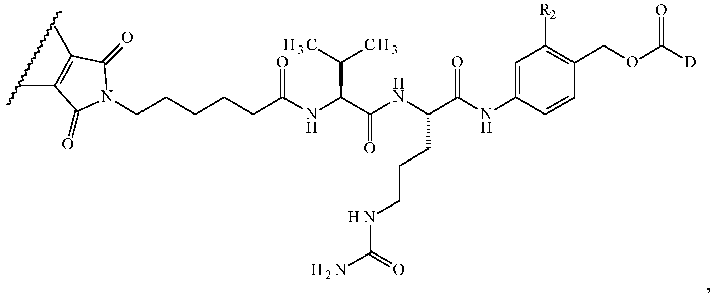 wherein wavy line indicates the covalent attachment site to the antibody and, R₂ and D are as defined herein before.

In a preferred embodiment, the invention provides an antibody-drug conjugate of Formula (III) wherein 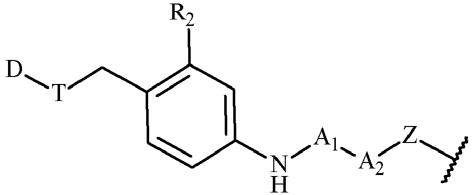 comprises a formula selected from: 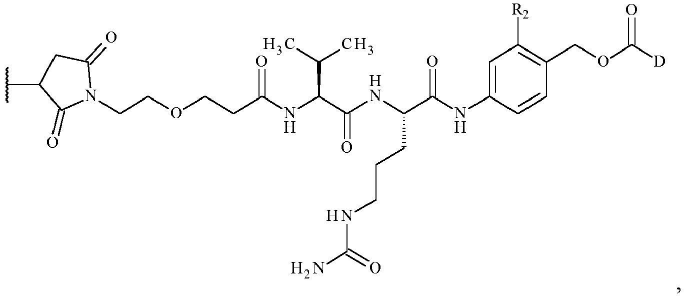 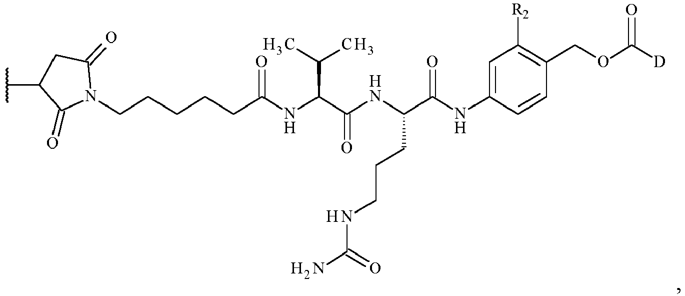 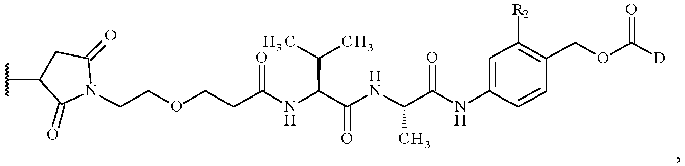 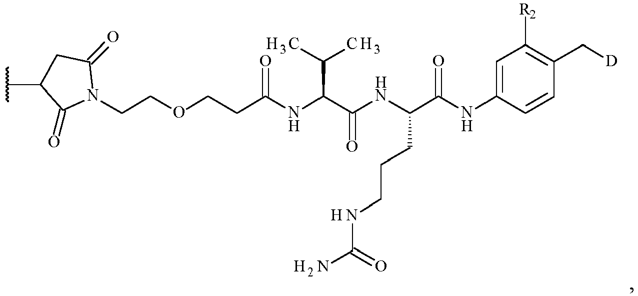 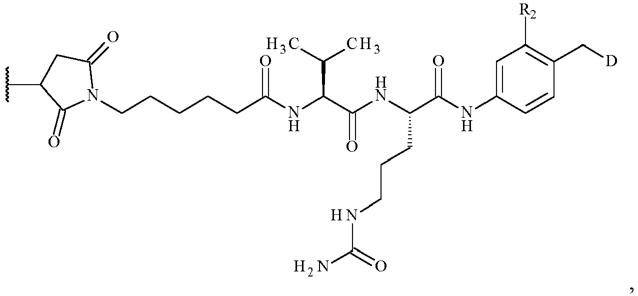 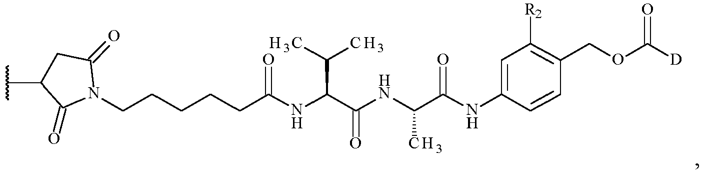 and 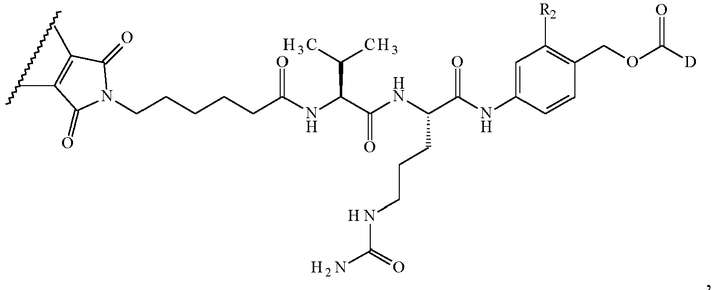 wherein wavy line indicates the covalent attachment site to the antibody and, R₂ and D are as defined herein before.

In a preferred embodiment, the invention provides an antibody-drug conjugate of Formula (III) wherein 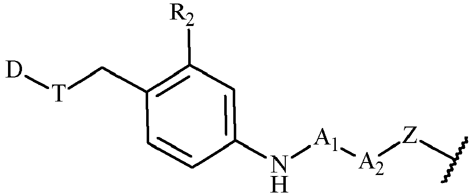 comprises a formula selected from: 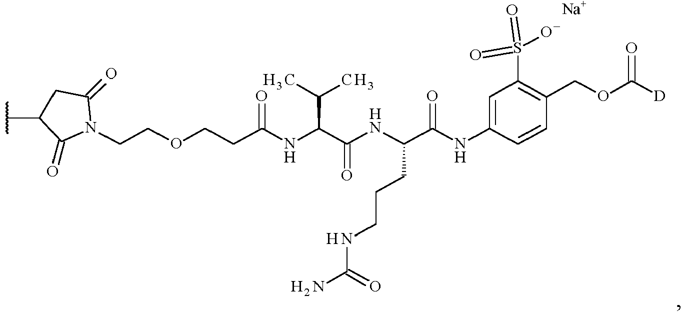 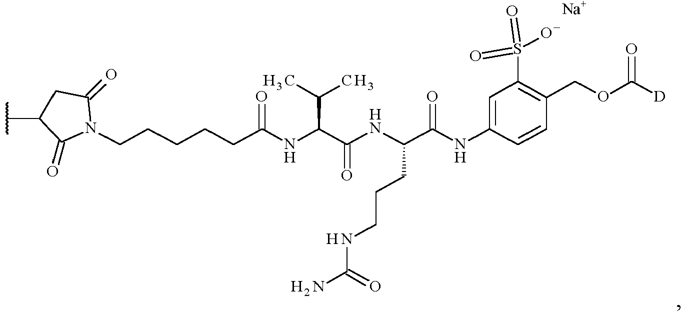 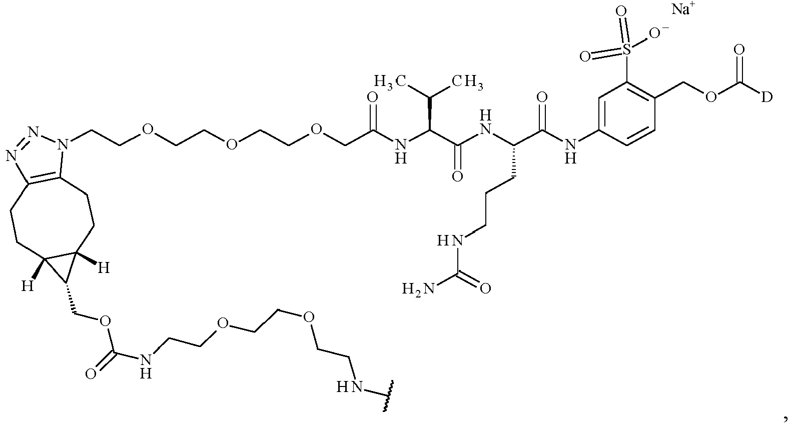 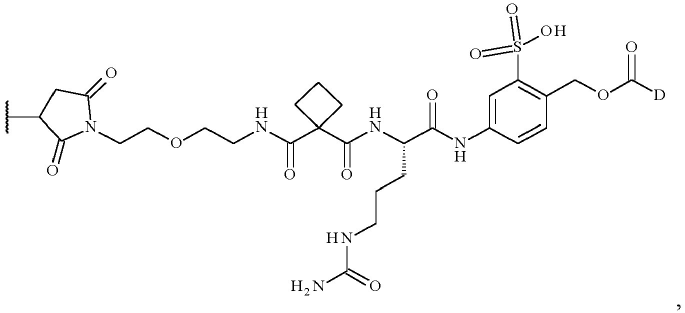 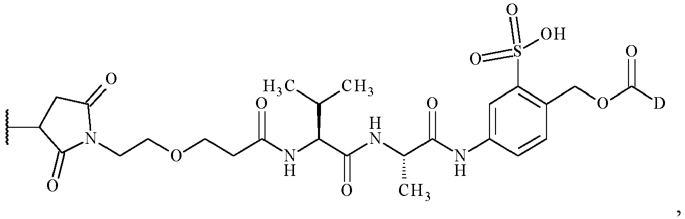 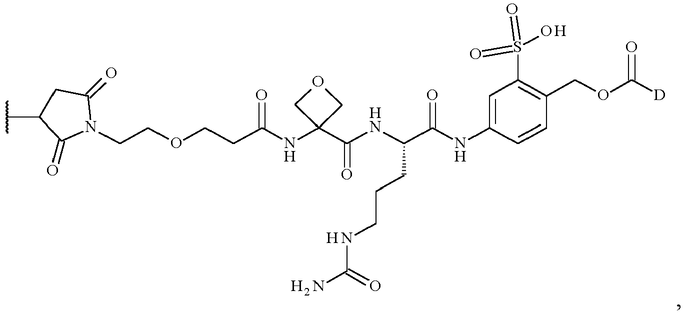 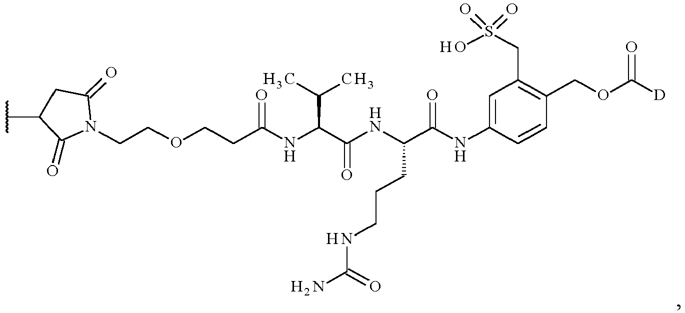 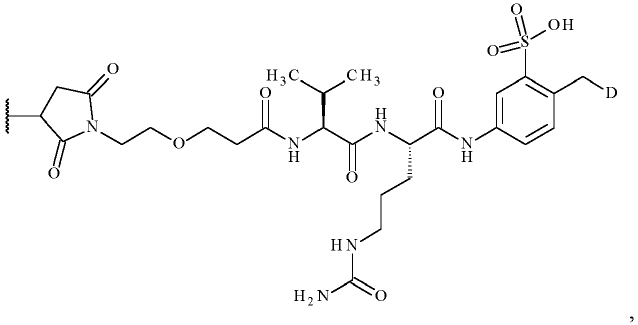 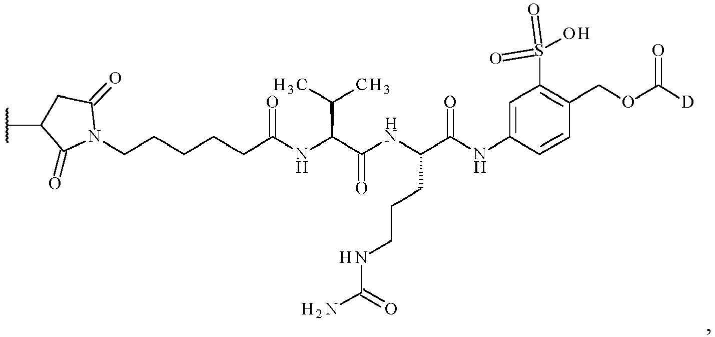 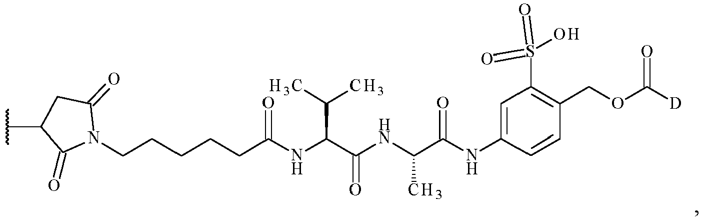 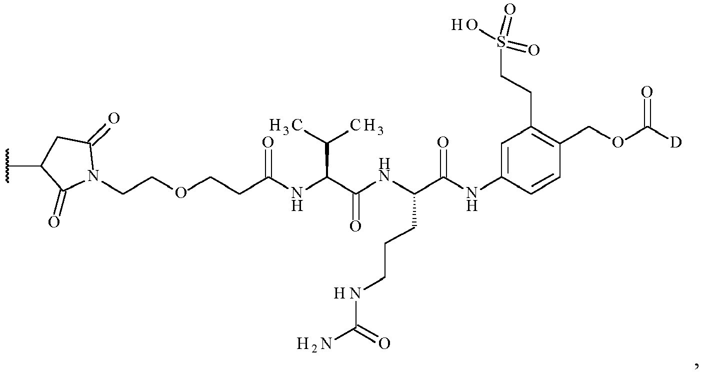 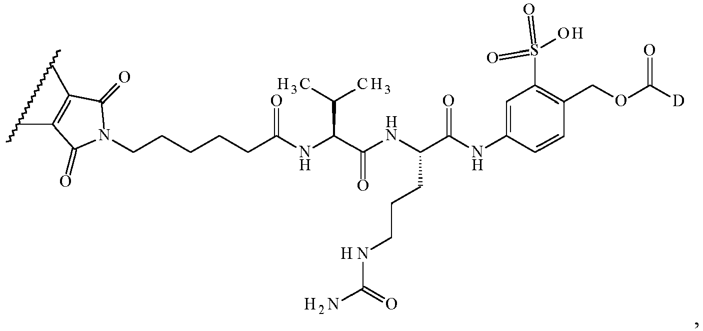 and 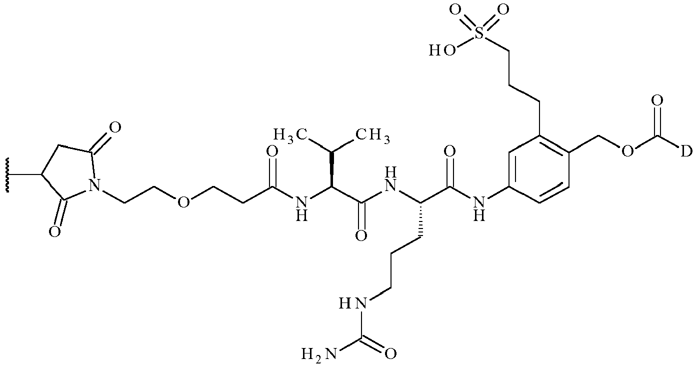 wherein wavy line indicates the covalent attachment site to the antibody and D is as defined herein before.

The ADC according to the invention are useful for treating diseases in mammal, e.g. a patient, in need thereof. The ADC can be used accordingly in a variety of settings for the treatment of a disorder associated with expression of the antigen to which the antibody of the ADC binds, e.g. cancer. The ADC can be used to deliver a drug to a targeted cell. Without being bound by theory, in one embodiment, the antibody of an ADC binds to or associates with a cell-surface antigen or receptor, and upon binding the ADC can be taken up (internalized) inside said cell through antigen- or receptor-mediated endocytosis or other internalization mechanism. The antigen can be attached to the said cell or can be an extracellular matrix protein associated with the said cell. Once inside the cell, via an enzymatic or non-enzymatic cleavable mechanism, depending upon the components of the linker system, the drug is released within the cell. In an alternative embodiment, the drug is cleaved from the ADC within the vicinity of the said cell, and the drug subsequently penetrates the cell.

The present invention provides pharmaceutical compositions comprising an ADC composition described herein and a pharmaceutically acceptable carrier. The pharmaceutical compositions can be in any form that allows for an ADC to be administered to a patient for treatment of a disorder associated with expression of the antigen to which the antibody of the ADC binds. Particularly, the present invention relates to pharmaceutical compositions comprising at least one antibody-drug conjugate of Formula (III) in combination with one or more pharmaceutically acceptable excipients. For example, the pharmaceutical compositions can be in the form of a liquid or a lyophilized solid. Among the pharmaceutical compositions according to the invention there may be mentioned more especially those that are suitable for oral, parenteral, nasal, per- or trans-cutaneous, rectal, perlingual, ocular or respiratory administration. The preferred route of administration is parenteral. Parenteral administration includes subcutaneous injections, intravenous, intramuscular, and intrasternal injection or infusion techniques. In preferred embodiments, a pharmaceutical composition comprising an ADC is administered intravenously in the form of a liquid solution. The liquid can be useful for delivery by injection. In a composition for administration by injection, one or more of a surfactant, preservative, wetting agent, dispersing agent, suspending agent, buffer, stabilizer, diluents, lubricants, binders, disintegration agents, absorbents and isotonic agent can also be included.

In another aspect, the present invention relates to the use of any of the antibody-drug conjugates defined above for the manufacture of a pharmaceutical preparation for the treatment of a mammal being in need thereof. In another aspect, the present invention relates to any of the antibody-drug conjugates defined above for use in the treatment of a mammal being in need thereof. The invention also relates to methods of treating a mammal being in need thereof, whereby the method comprises the administration of a pharmaceutical composition to the mammal in a therapeutically effective dose.

The invention is further exemplified by the following Examples. These examples are for illustrative purposes and are not intended to limit the scope of the invention.

### Abbreviations

- **ACN**: acetonitrile (or CH₃CN)
- **BBS**: borate buffered saline
- **DBCO**: dibenzocyclooctyne
- **DEA**: *N*,*N*-diethylamine
- **DCM**: dichloromethane
- **DIEA**: diisopropylethylamine
- **DIPEA**: *N*,*N*-diisopropylethylamine
- **DMA**: dimethylacetamide
- **DMAP**: 4-dimethylaminopyridine
- **DMF**: dimethylformamide (or *N*,*N*-dimethylformadide)
- **DMSO**: dimethylsulfoxide
- **EDC**: *N*-ethyl-*N*',*N*'-dimethylamino-propylcarbodiimide
- **EDTA**: ethylenediaminetetraacetic acid
- **EEDQ**: *N*-Ethoxycarbonyl-2-ethoxy-1,2-dihydroquinoline
- **EtOAc**: ethyl acetate
- **EtOH**: ethanol
- **FA**: formic acid
- **HATU**: 1-[bis(dimethylamino)methylene]-1H-1,2,3-triazolo[4,5-b]pyridinium 3-oxide hexafluorophosphate
- **HBTU**: [benzotriazol-1-yloxy(dimethylamino)methylene]-dimethyl-ammonium; hexafluorophosphate
- **HOAt**: 1-hydroxy-7-azabenzotriazole
- **HOBt**: 1-hydroxy-benzotriazole
- **MeOH**: methanol
- **MMAE**: (2,*S*)-*N*-[(1,*S*)-1-[[(1*S*,2*R*)-4-[(2,*S*)-2-[(1*R*,2*R*)-3-[[(1*R*,2*S*)-2-hydroxy-1-methyl-2-phenyl-ethyl]amino]-1-methoxy-2-methyl-3-oxo-propyl]pyrrolidin-1-yl]-2-methoxy-1-[(1*S*)-1-methylpropyl]-4-oxo-butyl]-methyl-carbamoyl]-2-methyl-propyl]-3-methyl-2-(methylamino)butanamide
- **NMP**: *N*-methyl-2-pyrrolidone
- **PBS**: phosphate-buffered saline
- **Pd/C**: Palladium on carbon
- **rmp**: multi-point attachment of Protein A
- **r.t.**: room temperature
- **TEA**: triethylamine
- **TFA**: trifluoroacetic acid
- **THE**: tetrahydrofuran
- **TSTU**: [dimethylamino-(2,5-dioxopyrrolidin-1-yl)oxy-methylene]-dimethyl-ammonium; tetrafluoroborate

### Materials and Methods/General Procedures

All reagents obtained from commercial sources were used without further purification. Anhydrous solvents were obtained from commercial sources and used without further drying. Flash chromatography was performed on CombiFlash Rf (Teledyne ISCO) with prepacked silica-gel cartridges (Macherey-Nagel Chromabond Flash). Thin layer chromatography was conducted with 5 x 10 cm plates coated with Merck Type 60 F254 silica-gel. Microwave heating was performed in CEM Discover^{®} instrument.

¹H-NMR measurements were performed on 400 MHz Bruker Avance or 500 MHz Avance Neo spectrometer, using DMSO-d6 (also written as dmso-d6 or DMSO) or CDCl₃ as solvent. ¹H NMR data is in the form of delta values, given in part per million (ppm), using the residual peak of the solvent (2.50 ppm for DMSO-d6 and 7.26 ppm for CDCl₃) as internal standard. Splitting patterns are designated as: s (singlet), d (doublet), t (triplet), q (quartet), m (multiplet), brs (broad singlet), dd (doublet of doublets), brm (broad multiplet), td (triplet of doublets), dt (doublet of triplets), br dd (broad doublet of doublets).

IR measurements were performed on a Bruker Tensor 27 equipped with ATR Golden Gate device (SPECAC). HRMS measurements were performed on a LTQ OrbiTrap Velos Pro mass spectrometer (ThermoFisher Scientific). Samples were dissolved in CH₃CN/H₂O (2/1:v/v) at a concentration range from 0.01 to 0.05 mg/mL approximately and introduced in the source by an injection of 2 µL in a flow of 0.1 mL/min.

ESI ionization parameters were as follows: 3.5 kV and 350 °C transfer ion capillary. All the spectra were acquired in positive ion mode with a resolving power of 30 000 or 60 000 using a lock mass.

### UPLC-MS:

Waters Aquity A-class with diode array UV detector "PDA" and "ZQ detector 2" mass device and MassLinks software.
ZQ detector 2: MS scan from 0.15 to 6 min and from 100 to 2372 Da
PDA detector: from 190 to 400 nm
Column: Acquity UPLC^{®} BEH column C₁₈, 1.7 µm, 130 Å, 2.1×50 mm
Column used at 40 °C with a flowrate of 0.6 mL/min
Solvent A: water + 0.02% TFA, Solvent B: acetonitrile + 0.02% TFA
Gradient from 2% B to 100% B in 5 minutes, then 0.3 minutes washing with 100% B and 0.5 minutes equilibration at 2% B for the next injection (total gradient of 6 minutes)

### Prep-HPLC:

Interchim Puriflash 4100^{®} with a maximum of 100 bars and a maximum flow rate of 250 mL/min or Interchim Puriflash 4250^{®} with a maximum of 250 bars and a maximum flow rate of 250 mL/min
Quaternary solvent pump with the possibility to use 4 solvents at the same time in a gradient UV: 2 wavelengths for the collection between 200 and 400 nm
Collection: 8 mL or 32 mL tubes
Columns Waters Xbridge^{®} 10 µm
3 Prep-HPLC methods were used:
   1) TFA method: solvent: A = water + 0.05% TFA, B = acetonitrile + 0.05% TFA, gradient from 5 to 100% B in 15 to 30 CV (Column Volume)
   2) NH₄HCO₃ method: solvent: A = water + 0.02 M NH₄HCO₃, B = acetonitrile/water 80/20 + 0.02M NH₄HCO₃, gradient from 5 to 100% B in 15 to 30 CV
   3) Neutral method: solvent: A = water, B = acetonitrile, gradient from 5 to 100 % B in 15 to 30 CV

All the fractions containing the pure compound were combined and directly freeze-dried to afford the compound as an amorphous powder.

### Preparative SFC purification:

Preparative chiral SFC was performed on a PIC solution Prep200 system. The sample was dissolved in ethanol at a concentration of 150 mg/mL. The mobile phase was held isocratically at 40% ethanol/CO₂. The instrument was fitted with a Chiralpak IA column and a loop of 3 mL. The ABPR (automatic back-pressure regulator) was set at 100 bars.

IUPAC chemical names were generated using Biovia Draw Version 18.1.NET software or using ACD/Name 2018.2.2 (File Version N50E41, Build 103230, 21 July 2018) software.

### Preparation 1: (2,5-dioxopyrrolidin-1-yl) 1-[2-[2-(2,5-dioxopyrrol-1-yl)ethoxy] ethylcarbamoyl] cyclobutanecarboxylate

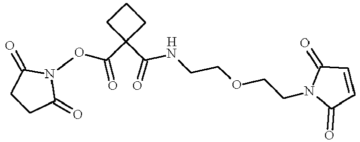

### Step 1: tert-butyl 1-[2-[2-(2,5-dioxopyrrol-1-yl)ethoxy]ethylcarbamoyl] cyclobutanecarboxylate

To a solution of 1-tert-butoxycarbonylcyclobutanecarboxylic acid (58.6 mg; 0.293 mmol) in DCM (5.85 mL), were successively added 1-[2-(2-aminoethoxy)ethyl]pyrrole-2,5-dione (53.9 mg; 0.293 mmol), EDC (84.2 mg; 0.439 mmol), HOBt (59.3 mg; 0.439 mmol) and DIPEA (204 µL; 1.17 mmol). The reaction mixture was stirred at r.t. for 18 hours and then was concentrated to dryness and solubilized in DMF (1 mL). After solubilization of the residue in DMF (1 mL), the crude was purified by C18 reverse phase prep-HPLC by direct deposit of the reaction mixture on the Xbridge^{®} column and using the TFA method to afford the title compound (57.3 mg; 0.156 mmol). IR (cm⁻¹): 3390, 1697/1666. ¹H NMR (400 MHz, dmso-d6) δ ppm 7.5 (t, 1H), 7.02 (s, 2H), 3.55/3.5 (2t, 4H), 3.38 (t, 2H), 3.17 (q, 2H), 2.33 (m, 4H), 1.77 (m, 2H), 1.38 (s, 9H). UPLC-MS: MS(ESI): m/z [M+Na]+ = 389.26, [M+H-tBu]+ = 311.22

### Step 2: 1-[2-[2-(2,5-dioxopyrrol-1-yl)ethoxy]ethylcarbamoyl]cyclobutanecarboxylic acid

To a solution of tert-butyl 1-[2-[2-(2,5-dioxopyrrol-1-yl)ethoxy]ethylcarbamoyl] cyclobutanecarboxylate (7 mg; 0.0191 mmol) in DCM (0.175 mL), was added TFA (51.2 µL; 0.668 mmol). The reaction mixture was stirred at r.t. for 3.5 hours, then was concentrated to dryness to obtain the title compound (5.8 mg; 0.0187 mmol) as a colorless oil. The crude product was used in the next step. UPLC-MS: MS(ESI): m/z [M+H]+ = 311.35, [M+Na]+ = 333.37

### Step 3: Preparation 1

To a solution of 1-[2-[2-(2,5-dioxopyrrol-1-yl)ethoxy]ethylcarbamoyl] cyclobutanecarboxylic acid (8.47 mg; 0.0273 mmol) in DMF (0.560 mL), were successively added TSTU (9.04 mg; 0.030 mmol) and DIPEA (9.5 µL; 0.0540 mmol). The reaction mixture was stirred at r.t. for 2 hours to afford a solution of **Preparation 1** in DMF. The crude product will be used directly for next steps. UPLC-MS: MS(ESI): m/z [M+H]+ = 408.43, [M+Na]+ = 430.38

### Preparation 2: (2,5-dioxopyrrolidin-1-yl) 3-[3-[2-(2,5-dioxopyrrol-1-yl)ethoxy] propanoylamino] oxetane-3-carboxylate

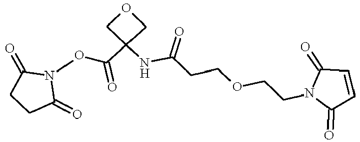

### Step 1 synthesis of 3-[3-[2-(2,5-dioxopyrrol-1-yl)ethoxy]propanoylamino]oxetane-3-carboxylic acid

To a solution of 3-aminooxetane-3-carboxylic acid (115 mg; 0.982 mmol) in DMF (3.45 mL), were successively added (2,5-dioxopyrrolidin-1-yl) 3-[2-(2,5-dioxopyrrol-1-yl)ethoxy]propanoate (274.3 mg; 0.883 mmol) and DIPEA (855 µL; 4.91 mmol). The reaction mixture was stirred at r.t. for 18 hours, then was concentrated to dryness and solubilized in DMF (1 mL). The crude product solution was purified by Cis reverse phase prep-HPLC by direct deposit of the reaction mixture on the Xbridge^{®} column and using the TFA method to afford the title compound (16 mg; 0.0512 mmol). UPLC-MS: MS(ESI): m/z [M+H]+ = 313.09, [M+Na]+ = 335.06. ¹H NMR (400 MHz, dmso-d6) δ ppm 8.92 (s, 1H), 7.02 (s, 2H), 4.8/4.45 (2d, 4H), 3.57/3.48 (2m, 6H), 2.32 (t, 2H). IR (cm⁻¹): 3700-2300, 1769/1740/1697, 692

### Step 2: Preparation 2

To a solution of 3-[2-[2-(2,5-dioxopyrrol-1-yl)ethoxy]ethylcarbamoyl]oxetane-3-carboxylic acid (5.8 mg; 0.0187 mmol) in DMF (0.380 mL), were successively added TSTU (6.32 mg; 0.0210 mmol) and DIPEA (6.7 µL; 0.0382 mmol). The reaction mixture was stirred at r.t. for 2 hours to afford a solution of **Preparation 2** in DMF. The crude product will be used directly for next steps. UPLC-MS: MS(ESI): m/z [M+H]+ = 310.29, [M+Na]+ = 332.27

### EXAMPLE 1: sodium 5-[[(2S)-2-[[(2S)-2-(9H-fluoren-9-ylmethoxycarbonylamino)-3-methyl-butanoyl]amino]-5-ureido-pentanoyl] amino]-2-(hydroxymethyl)benzenesulfonate

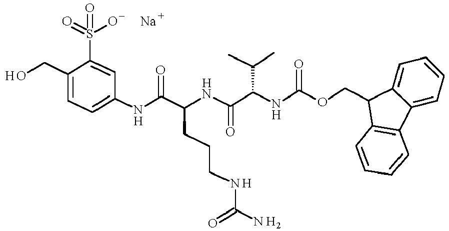

### Step 1: sodium 2-(hydroxymethyl)-5-nitro-benzenesulfonate

To a solution of sodium 5-nitro-2-[(E)-2-(4-nitro-2-sulfo-phenyl)vinyl]benzenesulfonate (25.0 g; 52.7 mmol; 1 eq.) in water (336 mL), was introduced a stream of ozone for 1.5 hours. After completion of the reaction, the mixture was purged with argon for 30 minutes in order to remove the excess of ozone. Then, sodium carbonate (39.1 g; 368 mmol) and sodium borohydride (3.99 g; 105 mmol) were added and the orange solution was stirred at r.t. for 16 hours. The reaction mixture was concentrated to dryness to furnish the title compound (39.9 g; 156 mmol) as a brown solid. ¹H NMR (DMSO): δ 4.99 (d, 2H, J = 3.6 Hz), 5.36 (t, 1H, J = 5.6 Hz), 7.83 (d, 1H, J = 8.4 Hz), 8.21 (d, 1H, J = 8.4 Hz), 8.45 (s, 1H).

### Step 2: sodium 5-amino-2-(hydroxymethyl)benzenesulfonate

Sodium 2-(hydroxymethyl)-5-nitro-benzenesulfonate (26.9 g; 105 mmol) was solubilized in water (403 mL). Then, the reaction mixture was flushed with argon. Pd/C 10% (2.65 g) was added then the black suspension was flushed with argon and then with hydrogen. The reaction mixture was stirred at r.t. for 3.5 days under hydrogen atmosphere. After filtration over Celite^{®} and washing with water and methanol, the filtrate was concentrated to dryness and co-evaporated three times with toluene in order to remove the remaining traces of water. Purification by column chromatography on silica gel using ethyl acetate/methanol (90/10 to 70/30) as eluent afforded the title compound (14.29 g; 63.46 mmol) as a pale yellow solid. ¹H NMR (DMSO): δ 4.52 (d, 2H, J = 5.2 Hz), 4.95 (t, 1H, J = 5.2 Hz), 5.04 (s, 2H), 6.42 (d, 1H, J = 7.6 Hz), 6.93 (d, 1H, J = 7.6 Hz), 7.03 (s, 1H).

### Step 3: sodium 5-[[(2S)-2-(9H-fluoren-9-ylmethoxycarbonylamino)-5-ureido-pentanoyl]amino]-2-(hydroxymethyl)benzenesulfonate

To a solution of Fmoc-L-Cit-OH (CAS No. 133174-15-9; 882 mg; 2.22 mmol) in DMF (32.5 mL), were added sodium 5-amino-2-(hydroxymethyl)benzenesulfonate (500 mg; 2.22 mmol), HBTU (1.01 g; 2.66 mmol) and DIPEA (917 mL; 5.55 mmol). The reaction mixture was stirred at r.t. for 16 hours, then was concentrated to dryness and co-evaporated with water (2 x 100 mL). The crude was purified by column chromatography on Cis using neutral method, to afford the title compound (1.0 g; 1.40 mmol) as pale red oil. ¹H NMR (DMSO): δ 4.30-4.12 (m, 4H), 4.74 (d, 2H, J = 4.4 Hz), 5.05 (t, 1H, J = 5.6 Hz), 5.37 (s, 2H), 5.97 (t, 1H, J = 4.8 Hz), 7.34-7.42 (m, 4H), 7.62-7.90 (m, 7H), 8.15 (s, 1H), 10.05 (s, 1H).

### Step 4: sodium 5-[[(2S)-2-amino-5-ureido-pentanoyl]amino]-2-(hydroxymethyl) benzenesulfonate

To a solution of sodium 5-[[(2*S*)-2-(9H-fluoren-9-ylmethoxycarbonylamino)-5-ureido-pentanoyl]amino]-2-(hydroxymethyl)benzenesulfonate (11.2 g; 15.73 mmol) in DMF (224 mL), was added piperidine (3.1 mL; 31.47 mmol). The reaction mixture was stirred at r.t. for 3 hours then water (400 mL) was added. The aqueous layer was extracted with ethyl acetate (2 x 300 mL) and with DCM (300 mL). Sodium carbonate (5.01 g; 47.1 mmol; 3 eq.) was added to the aqueous layer and the mixture was stirred for 3 hours at r.t. The mixture was lyophilized to give the title compound (6.01 g; 15.73 mmol) as a white solid. ¹H NMR (DMSO): δ 1.55-1.64 (m, 4H), 2.99-3.01 (m, 2H), 3.58 (m, 1H), 4.75 (s, 2H), 5.06 (s, 1H), 5.38 (s, 2H), 5.98 (t, 1H, J = 5.6 Hz), 7.38 (d, 1H, J = 8.4 Hz), 7.72 (dd, 1H, J = 8.4 and 2.4 Hz), 7.86 (d, 1H, J = 2.4 Hz), 10.17 (s, 1H).

### Step 5: Example 1

To a solution of sodium 5-[[(2S)-2-amino-5-ureido-pentanoyl]amino]-2-(hydroxymethyl) benzenesulfonate (6.01 g; 15.73 mmol) in DMF (150 mL), was added Fmoc-L-Val-OSu (CAS No. 130878-68-1; 6.85 g; 15.69 mmol). The beige solution was stirred at r.t. for 3 hours then the reaction mixture was diluted with saturated sodium hydrogenocarbonate (100 mL) and water (100 mL) and concentrated to dryness. The residue was purified on silica gel using ethyl acetate/methanol 90/10 to 50/50 as eluent to afford the title compound (4.44 g; 6.31 mmol) as a white solid. ¹H NMR (DMSO): 0.85-0.90 (m, 6H), 1.31-1.76 (m, 4H), 1.95-2.06 (m, 1H), 2.91-3.05 (m, 2H), 3.95 (t, 1H, J = 8.4 Hz), 4.24-4.35 (m, 3H), 4.37-4.45 (m, 1H), 4.76 (d, 2H, J = 6 Hz), 5.07 (t, 1H, J = 6.4 Hz,), 5.40 (s, 2H), 6.03 (t, 1H, J = 5.6 Hz), 7.32-7.46 (m, 6H), 7.67 (d, 1H, J = 8 Hz), 7.76 (t, 2H, J = 7.2 Hz), 7.88-7.91 (m, 3H), 8.12 (d, 1H, J = 7.6 Hz), 10.08 (s, 1H). ¹³C NMR (DMSO): 18.25, 19.24, 26.70, 29.56, 30.45, 39.50, 46.67, 53.17, 60.01, 60.96, 65.66, 117.85, 119.15, 120.05, 125.36, 127.06, 127.62, 128.09, 134.39, 136.79, 140.67, 143.89, 145.34, 156.08, 158.82, 170.37, 171.16. LCMS (2-100 acetonitrile/H₂O + 0.1% formic acid): 93.85% Tr = 8.4 min. Positiv mode: 682.15 detected (MH⁺). Negativ mode: 680.17 detected (MH⁻)

### EXAMPLE 2: 5-[[(2S)-2-[[(2S)-2-(9H-fluoren-9-ylmethoxycarbonylamino)-3-methyl-butanoyl]amino]propanoyl]amino]-2-(hydroxymethyl)benzenesulfonic acid

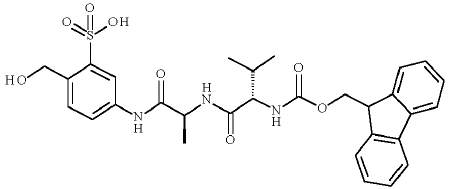

### Step 1: sodium 5-[[(2S)-2-(tert-butoxycarbonylamino)propanoyl]amino]-2-(hydroxymethyl) benzenesulfonate

To a solution of Boc-L-Ala-OH (CAS No. 15761-38-3; 588 mg; 3.11 mmol) in DMF (38.6 mL), were successively added HATU (1.77 g; 4.67 mmol), sodium 5-amino-2-(hydroxymethyl)benzenesulfonate (771 mg; 3.42 mmol) and DIPEA (1.29 mL; 7.78 mmol). The reaction mixture was stirred for 16 hours at r.t. then concentrated to dryness and co-evaporated with water to afford the crude reaction mixture. The resulting residue was purified by column chromatography on silica gel using ethyl acetate/methanol 95:5 to 80:20 as eluent to afford the title compound (1.17 g; 2.95 mmol) as a white solid. ¹H NMR (DMSO): δ 1.24 (s, 9H), 1.38 (m, 3H), 4.05-1.44 (m, 1H), 4.73 (d, 2H, J = 4.8 Hz), 5.04 (t, 1H, J = 5.6 Hz), 6.97-7.02 (m, 1H), 7.33 (d, 1H, J = 8 Hz), 7.65-7.70 (m, 1H), 7.83 (s, 1H), 9.91 (s, 1H).

### Step 2: 5-[[(2S)-2-aminopropanoyl]amino]-2-(hydroxymethyl)benzenesulfonic acid, hydrochloride

Sodium 5-[[(2S)-2-(tert-butoxycarbonylamino)propanoyl]amino]-2-(hydroxymethyl) benzenesulfonate (1.17 g; 2.95 mmol; 1 eq.) was suspended in a solution of HCl 4N in dioxane (10 mL). The mixture was stirred at r.t. for 2 hours then concentrated to dryness to afford the crude mixture (982 mg; 2.95 mmol) as a white solid. ¹H NMR (DMSO): δ 1.45 (d, 3H, J = 5.6 Hz), 3.91-4.0 (m, 1H), 4.76 (s, 2H), 7.41 (d, 1H, J = 7.6 Hz), 7.66 (d, 1H, J = 7.6 Hz), 7.85 (s, 1H), 8.17 (s, 2H), 10.44 (s, 1H)

### Step 3: Example 2

To a solution of 5-[[(2S)-2-aminopropanoyl]amino]-2-(hydroxymethyl)benzenesulfonic acid, hydrochloride (981 mg; 2.95 mmol) in DMF (34.5 mL), were added Fmoc-L-Val-OSu (CAS No. 130878-68-1; 1.29 g; 2.95 mmol; 1 eq.) and DIPEA (975 µL; 5.9 mmol). The mixture was stirred overnight at r.t. then concentrated to dryness and co-evaporated with water to afford the crude mixture. The resulting residue was purified by column chromatography on silica gel using ethyl acetate / methanol 95:5 to 80:20 as eluent to afford **Example 2** (1.28 g; 2.072 mmol) as colorless oil. ¹H NMR (DMSO): δ 0.80-0.92 (m, 6H), 1.30 (d, 3H, J = 6.4 Hz), 2.02-2.10 (m, 1H), 4.17-4.31 (m, 3H), 4.37-4.44 (m, 1H), 4.73 (d, 2H, J = 5.6 Hz), 5.04 (t, 1H, J = 6.4 Hz), 7.28-7.36 (m, 3H), 7.37-7.47 (m, 3H), 7.66 (d, 1H, J = 8.4 Hz), 7.71-7.77 (m, 2H), 7.83-7.85 (m, 1H), 7.88 (d, 2H, J = 7.6 Hz), 8.14 (d, 1H, J = 6.4 Hz), 9.99 (s, 1H).

### EXAMPLE 3: Sodium [5-[[(2S)-2-[[(2S)-2-(9H-fluoren-9-ylmethoxycarbonylamino)-3-methyl-butanoyl]amino]-5-ureido-pentanoyl]amino]-2-(hydroxymethyl)phenyljmethanesulfonate

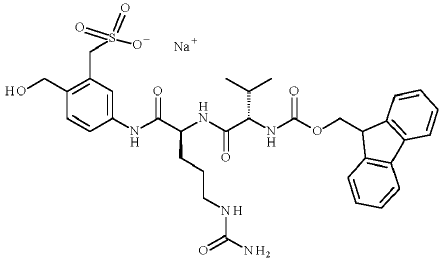

### Step 1: (2S)-2-[[(2S)-2-(9H-fluoren-9-ylmethoxycarbonylamino)-3-methyl-butanoyl]amino]-5-ureido-pentanoic acid

Fmoc-L-Val-OSu (CAS No. 130878-68-1; 1.59 g; 3.64 mmol; 1.0 eq.) was added at r.t. to a solution of L(+)-citrulline (0.67 g; 3.82 mmol; 1.05 eq.) and sodium bicarbonate (0.32 g; 3.82 mmol; 1.05 eq.) in a mixture of 1,2-dimethoxyethane (9.5 mL), water (9.5 mL) and THF (4.7 mL). The reaction mixture was stirred at r.t. for 16 hours, then the solvents were removed under vacuum and the residue was acidified with a solution of hydrochloric acid 1N until pH 1. The white suspension was filtered, washed with water (3 x 40 mL), diethyl ether (3 x 40 mL) and co-evaporated with acetonitrile (2 x 150 mL). The resulting white solid was purified by flash chromatography over silica gel using (DCM/acetic acid (99/1))/ methanol (10/0 to 7/3) as eluent, then triturated in diethyl ether (2 x 30 mL).The resulting solid was dissolved in a mixture of methanol (50 mL) and water (50 mL), concentrated to dryness, co-evaporated with acetonitrile (100 mL) and dried to afford the title compound (0.75 g; 1.5 mmol) as a white solid. ¹H NMR (DMSO): δ 0.84-0.88 (m, 6H), 1.34-1.41 (m, 2H), 1.51-1.73 (m, 2H), 1.95-2.04 (m, 1H), 2.91-2.96 (m, 2H), 3.88-3.92 (m, 1H), 4.06-4.11 (m, 1H), 4.19-4.31 (m, 3H), 5.36 (s, 2H), 5.91-5.94 (m, 1H), 7.30-7.34 (m, 2H), 7.39-7.45 (m, 3H), 7.75 (t, 2H, J = 7.3 Hz), 7.89 (d, 2H, J = 7.4 Hz), 8.02 (brs, 1H), 12.56 (brs, 1H)

### Step 2: Sodium (2-methoxycarbonyl-5-nitro-phenyl)methanesulfonate

To a solution of sodium sulfite (14.39 g; 114.17 mmol) in water (228 mL), was added a suspension of methyl 2-(bromomethyl)-4-nitrobenzoate (10.43 g; 38.06mmol) in methanol (37 mL). The reaction mixture was stirred at r.t. for 20 hours and then concentrated to dryness. The resulting crude was purified by flash chromatography over silica gel using ethyl acetate / methanol (10/0 to 6/4) as eluent to afford the title compound (13.9 g; 31.07 mmol) as white solid. ¹H NMR (DMSO): δ 3.82 (s, 3H), 4.29 (s, 2H), 7.87 (d, 1H, J = 8.6 Hz), 8.16 (dd, 1H, J = 2.5 and 8.6 Hz), 8.25 (d, 1H, J = 2.5 Hz)

### Step 3: Sodium [2-(hydroxymethyl)-5-nitro-phenyl]methanesulfonate

To a suspension of sodium (2-methoxycarbonyl-5-nitro-phenyl)methanesulfonate (10.1 g; 22.09 mmol) in THF (368 mL), was added lithium borohydride (0.59 g; 24.3 mmol). The reaction mixture was stirred at r.t. for 16 hours, then lithium borohydride (0.27 g; 11.04 mmol) was again added and the reaction mixture was stirred at r.t. for 24 hours. The reaction mixture was diluted with methanol (50 mL) and concentrated to dryness. The crude was purified by flash chromatography over silica gel using ethyl acetate/methanol (10/0 to 5/5) as eluent to afford the title compound (5.09 g; 6.52 mmol) as yellow solid. ¹H NMR (DMSO): δ 3.90 (s, 2H), 4.76 (d, 2H, J = 5.7 Hz), 5.38 (t, 1H, J = 5.6 Hz), 7.66 (d, 1H, J = 8.8 Hz), 8.07-8.09 (m, 2H)

### Step 4: Sodium [5-amino-2-(hydroxymethyl)phenyl]methanesulfonate

To a solution of sodium [2-(hydroxymethyl)-5-nitro-phenyl]methanesulfonate (2 g; 3.57 mmol) in methanol (102 mL) was added Pd/C 10% (0.56 g; 0.53 mmol) under argon. The reaction mixture was purged with hydrogen and stirred at r.t. under atmospheric pressure of hydrogen for 2 hours. The reaction mixture was then filtered over Celite^{®}, washed with methanol (3 x 50 mL) and concentrated to dryness. The resulting crude was purified by flash chromatography on Cis using acetonitrile/water (2/98 to 50/50) to afford the title compound (1.98 g; 3.46 mmol) as a grey solid. ¹H NMR (DMSO): δ 3.72 (s, 2H), 4.3 (d, 2H, J = 5.9 Hz), 4.89 (s, 2H), 4.96 (t, 1H, J = 5.9 Hz), 6.38 (dd, 1H, J = 2.4 and 8.1 Hz), 6.49 (d, 1H, J = 2.4 Hz), 6.9 (d, 1H, J = 8.1 Hz)

### Step 5: Example 3

To a solution of (2S)-2-[[(2S)-2-(9H-fluoren-9-ylmethoxycarbonylamino)-3-methyl-butanoyl]amino]-5-ureido-pentanoic acid (543 mg; 1.09 mmol) in dry DMF (6 mL), were added HATU (600 mg; 1.58 mmol), sodium hydrogenocarbonate (102 mg; 1.22 mmol) and a suspension of sodium [5-amino-2-(hydroxymethyl)phenyl]methanesulfonate (650 mg; 1.41 mmol) in dry DMF (14 mL). The reaction mixture was stirred at r.t. for 3 hours and then co-evaporated with dioxane (200 mL). The crude was purified by flash chromatography over silica gel using DCM/methanol (10/0 to 5/5) as eluent, followed by flash chromatography on Cis using acetonitrile/water + 0.1% formic acid (2/98 to 50/50) as eluent to afford after freeze-drying **Example 3** (233mg; 0.32 mmol) as a white solid. ¹H NMR (DMSO): δ 0.84-0.89 (m, 6H), 1.32-1.49 (m, 2H), 1.54-1.73 (m, 2H), 1.94-2.04 (m, 1H), 2.89-3.09 (m, 2H), 3.81 (s, 2H), 3.91-3.95 (m, 1H), 4.20-4.33 (m, 3H), 4.41-4.46 (m, 1H), 4.47 (s, 2H), 5.07 (brs, 1H), 5.40 (brs, 2H), 5.98 (t, 1H, J = 5.7 Hz), 7.21 (d, 1H, J = 8.2 Hz), 7.31-7.36 (m, 3H), 7.39-7.45 (m, 3H), 7.63 (dd, 1H, J = 2.2 and 8.3Hz), 7.75 (t, 2H, J = 7.5Hz), 7.89 (d, 2H, J = 7.4 Hz), 8.08 (d, 1H, J = 7.6 Hz), 10.01-10.8 (m, 1H). LCMS (2-100 ACN/H₂O+0.1% TFA): 93.75% Tr = 8.1 min. Negative mode 694.14 detected (M-H)

### EXAMPLE 4: 2-(chloromethyl)-5-[[(2S)-2-[[(2S)-2-(9H-fluoren-9-ylmethoxycarbonylamino)-3-methyl-butanoyl]amino]-5-ureido-pentanoyl]amino]benzenesulfonic acid

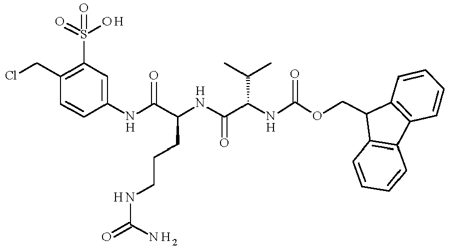

5-[[(2S)-2-[[(2S)-2-(9H-fluoren-9-ylmethoxycarbonylamino)-3-methyl-butanoyl]amino]-5-ureido-pentanoyl]amino]-2-(hydroxymethyl)benzenesulfonic acid (free acid of **Example 1;** 300 mg, 0.4263 mmol) was dissolved in anhydrous NMP (6 mL) at r.t. In parallel, a solution of SOCl₂ (206 µL) in NMP (6 mL) was prepared. To the reaction, were added 6 times over a 75 minutes period, a solution 900 µL of the said SOCl₂ solution. After the last addition, the reaction mixture was stirred at r.t. for 15 minutes. The crude product was purified by direct deposit of the reaction mixture on an Oasis column in using the TFA method to afford the title compound (138 mg; 0.1971 mmol) as a white powder. ¹H NMR (400 MHz, dmso-d6) δ ppm 10.15+8.1+7.42+6.0 (s+2d+m, 4H), 7.9 (m, 3H), 7.75 (m, 3H), 7.42+7.31 (2m, 5H), 5.23 (s, 2H), 4.4 (m, 1H), 4.3-4.2 (m, 3H), 3.95 (dd, 1H), 3.0 (m, 2H), 2.0 (m, 1H), 1.7 + 1.6 (2m, 2H), 1.48 + 1.37 (2m, 2H), 0.88 (2d, 6H). HR-ESI+: m/z [M+H]+ = 700.2199 / 700.2202 [measured/theoretical]

### EXAMPLE 5: 2-(chloromethyl)-5-[[(2S)-2-[[(2S)-2-(9H-fluoren-9-ylmethoxycarbonylamino)-3-methyl-butanoyl]amino]propanoyl]amino]benzenesulfonic acid

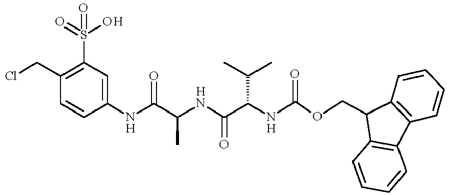

To a solution of 5-[[(2S)-2-[[(2S)-2-(9H-fluoren-9-ylmethoxycarbonylamino)-3-methyl-butanoyl]amino]propanoyl]amino]-2-(hydroxymethyl)benzenesulfonate (sulfonate obtained from **Example 2;** 504.1 mg, 0.816 mmol) in NMP (5 mL), were added 6 times over a 75 minutes period, a solution of SOCl₂ (60 µL 0.816 mmol) in NMP (500 µL). The reaction mixture was stirred at r.t. for 15 minutes. The crude product was purified by direct deposit of the reaction mixture on an Oasis column in using the TFA method to afford (337 mg) as a white powder. IR Wavelength (cm⁻¹): 3600 to 2400, 1688+1648, 1599, 1518, 1022. UPLC-MS: MS (ESI) m/z [M+H]+ = 614.17+616.18 (Cl)

### EXAMPLE 6: Sodium 5-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl] amino]-1-methoxy-2-methyl-3-oxo-propyl] pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonate

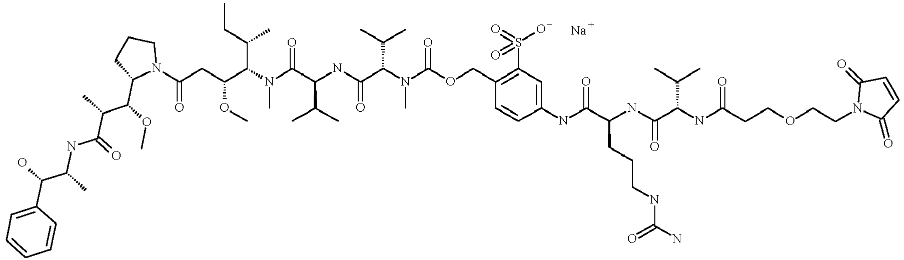

### Step 1: Sodium 5-[[(2S)-2-[[(2S)-2-(9H-fluoren-9-ylmethoxycarbonylamino)-3-methyl-butanoyl]amino]-5-ureido-pentanoyl]amino]-2-[(4-nitrophenoxy)carbonyloxymethyl]benzenesulfonate

To a solution of **Example 1** (450 mg; 0.64 mmol) in DMF (6 mL), were added DIEA (1.34 mL; 7.67 mmol) and bis(4-nitrophenyl)carbonate (778 mg; 2.56 mmol). The solution was stirred at r.t. for 2 hours and bis(4-nitrophenyl)carbonate (390 mg; 1.28 mmol) was added. After 1 hour, the solution was concentrated under reduced pressure and the residue was purified by silica gel chromatography (gradient of methanol and acetic acid in DCM) to give the title compound (523 mg; 0.45 mmol). ¹H NMR (400 MHz, dmso-d6) δ ppm 10.2/8.1/5.95 (m, 3H), 8.3 (d, 2H), 7.95 (s, 1H), 7.9 (d, 2H), 7.75 (dd, 1H), 7.75 (m, 2H), 7.65 (d, 2H), 7.4 (d, 4H), 7.35 (d, 1H), 5.7 (s, 2H), 5.35 (brs, 2H), 4.4 (m, 1H), 4.3 (t, 1H), 4.2 (d, 2H), 3.95 (m, 1H), 3 (m, 2H), 2 (m, 1H), 1.8-1.3 (m, 4H), 0.85 (2d, 6H)

### Step 2: 5-[[(2S)-2-[[(2S)-2-amino-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonic acid, trifluoroacetic acid salt

To a solution of MMAE (200 mg; 0.28 mmol) in DMF (5.6 mL), was added DIEA (0.19 mL; 1.39 mmol) followed by sodium 5-[[(2*S*)-2-[[(2*S*)-2-(9*H*-fluoren-9-ylmethoxycarbonylamino)-3-methyl-butanoyl]amino]-5-ureido-pentanoyl]amino]-2-[(4-nitrophenoxy)carbonyloxymethyl]benzenesulfonate (472 mg; 0.55 mmol) and HOBt (75 mg; 0.56 mmol). The solution was stirred at r.t. for 3.5 hours, then DEA (0.19 mL; 1.39 mmol) was added. After 2 hours, the solution was concentrated under reduced pressure and the residue was purified by Cis reverse phase preparative-HPLC on Xbridge^{®} by TFA method to give the title compound (99 mg; 0.08 mmol). ¹H NMR (400 MHz, dmso-d6) δ ppm 10.2/10 (2s, 1H), 8.56/8.35 (2m, 1H), 8.1 (d, 1H), 7.9 (brs, 1H), 7.88/7.6 (2d, 1H), 7.68 (brs, 1H), 7.35-7.13 (m, 5H), 7.3 (brs, 1H), 6/5.96 (2t, 1H), 5.5 (m, 2H), 5.4 (brs, 2H), 4.8-4.15 (m, 5H), 4.05-3.85 (m, 2H), 3.8-3.6 (5s, 9H), 3.62-3.5 (m, 2H), 3.1-2.8 (m, 5H), 3 (brs, 3H), 2.4/2.3 (d+dd, 2H), 2.2-1.9 (m, 3H), 1.9-1.75 (m, 11H), 1.05/1 (2d, 9H), 1-0.75 (m, 21H)

### Step 3: Example 6

To a solution of 5-[[(2S)-2-[[(2S)-2-amino-3-methyl-butanoyl]amino]-5-ureido-pentanoyl] amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonic acid, TFA salt (22 mg; 0.018 mmol) in DMF (0.18 mL), were added DIEA (6.3 µL mL; 36 µmmol) and 1-[2-[3-(2,5-dioxopyrrolidin-1-yl)-3-oxo-propoxy]ethyl]pyrrole-2,5-dione (6.2 mg; 20 µmol). The solution was stirred at r.t. for 4.5 hours. The reaction was purified by Cis reverse phase preparative-HPLC on Xbridge^{®} column using NH₄HCO₃ method to give **Example 6** (9.6 mg; 6.8 µmol). HRMS (ESI) [M+H]⁺ found = 1420.7041 (δ = -0.3 ppm)

### EXAMPLE 7: Sodium 5-[[(2S)-2-[[(2S)-2-[6-(2,5-dioxopyrrol-1-yl)hexanoylamino]-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl] amino]-1-methoxy-2-methyl-3-oxo-propyl]pyrrolidin-1-yl]-2-methoxy-1-[(1S)-1-methylpropyl]-4-oxobutyl]-methyl-carbamoyl]-2-methyl-propyl]carbamoyl]-2-methyl-propyl]-methylcarbamoyl]oxymethyl]benzenesulfonate

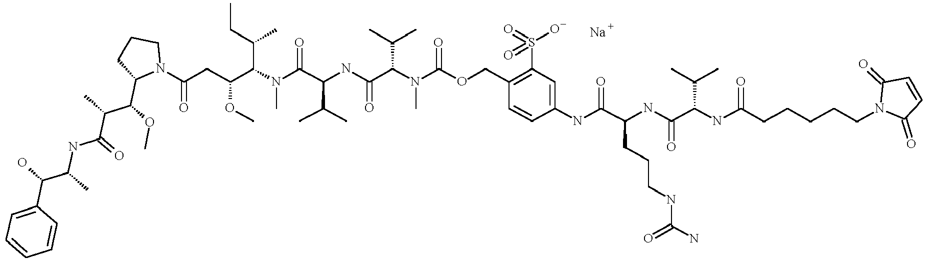

To a solution of 5-[[(2S)-2-[[(2S)-2-amino-3-methyl-butanoyl]amino]-5-ureido-pentanoyl] amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methylpropyl]carbamoyl]-2-methyl-propyl]-methyl-carbamoyl]oxymethyl]benzenesulfonic acid, TFA salt (obtained from Step 2 of **Example 6;** 30 mg; 0.025 mmol) in DMF (0.25 mL) was added DIEA (8.7 µL mL; 36 µmmol) and (2,5-dioxopyrrolidin-1-yl) 6-(2,5-dioxopyrrol-1-yl)hexanoate (8.46 mg; 0.027 mmol). The solution was stirred at r.t. for 3.5 hours. The reaction was purified by Cis reverse phase preparative-HPLC on Xbridge^{®} column using NH₄HCO₃ method to give **Example 7** (20 mg; 14.09 µmol). HRMS (ESI) [M+H]⁺ found = 1418.7253 (δ = 0.1 ppm)

### EXAMPLE 8: sodium 5-[[(2S)-2-[[(2S)-2-[[2-[2-[2-(2azidoethoxy)ethoxy] ethoxy] acetyl] amino] -3-methyl-butanoyl] amino] -5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonate

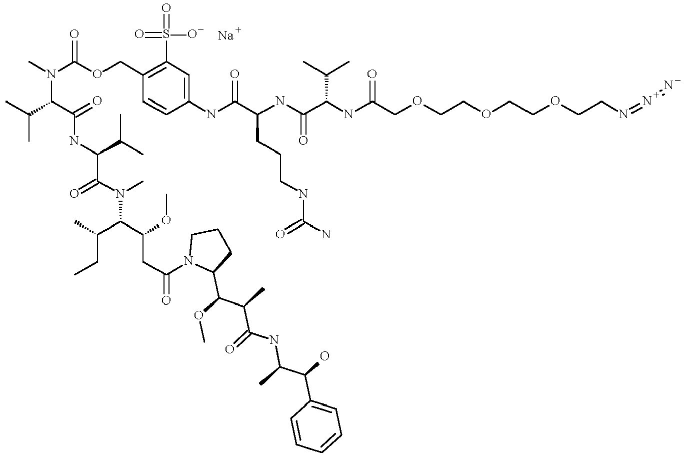

To a solution of 2-[2-[2-(2-azidoethoxy)ethoxy]ethoxy]acetic acid (6.4 mg; 0.027 mmol) in DMF (125 µL), were added DIEA (21.7 µL; 124 µmmol) and TSTU (7.88 mg; 0.026 mmol). After completion of the activation, a solution of 5-[[(2S)-2-[[(2S)-2-amino-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methylcarbamoyl]-2-methyl-propyl]carbamoyl]-2-methyl-propyl]-methylcarbamoyl]oxymethyl]benzenesulfonic acid, TFA salt (30 mg; 0.025 mmol) in DMF (0.15 mL) was added. The solution was stirred at r.t. for 2 hours. The reaction was purified by Cis reverse phase preparative-HPLC on Xbridge^{®} column using NH₄HCO₃ method to give **Example 8** (16 mg; 11.10 µmol). HRMS (ESI) [M+H]⁺ found = 1440.7382 (δ = -2.6 ppm)

### EXAMPLE 9: 5-[[(2S)-2-[[1-[2-[2-(2,5-dioxopyrrol-1-yl)ethoxy] ethylcarbamoyl] cyclobutanecarbonyl] amino] -5-ureido-pentanoyl] amino] -2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonic acid

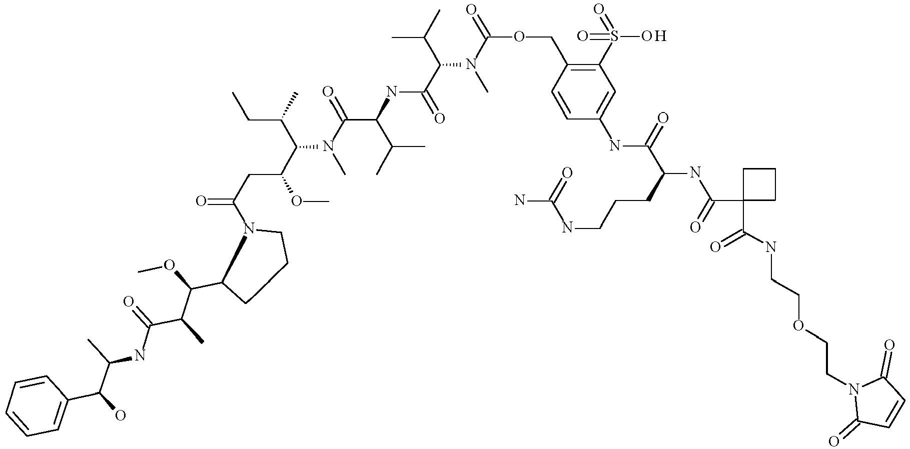

### Step 1: sodium 5-[[(2S)-2-(9H-fluoren-9-ylmethoxycarbonylamino)-5-ureido-pentanoyl]amino]-2-[(4-nitrophenoxy)carbonyloxymethyl]benzenesulfonate

To a solution of sodium 5-[[(2S)-2-(9H-fluoren-9-ylmethoxycarbonylamino)-5-ureido-pentanoyl]amino]-2-(hydroxymethyl)benzenesulfonate (261mg; 0.448mmol) in DMF (5.75 mL), were successively added bis(4-nitrophenyl) carbonate (1.09 g; 3.58 mmol) and DIPEA (1.87 mL; 10.75 mmol). The reaction mixture was stirred at r.t. for 16 hours, then was concentrated to dryness to afford the crude mixture. The crude product was purified by silica gel chromatography (gradient of methanol containing 2% AcOH in DCM) to afford the title compound (137 mg; 0.183 mmol) as a white solid. UPLC-MS: MS(ESI): m/z [M+H]+ = 748.48

### Step 2: sodium 5-[[(2S)-2-(9H-fluoren-9-ylmethoxycarbonylamino)-5-ureidopentanoyl]amino]-2-[[[(1S)-]-[[(1S)-1-[[(1S,2R)-4-[(2S)-2-[(1R,2R)-3-[[(1R,2S)-2-hydroxy-l-methyl-2-phenyl-ethyl]amino]-l-methoxy-2-methyl-3-oxo-propyl]pyrrolidin-l-yl/-2-methoxy-1-[(1S)-1-methylpropyl]-4-oxo-butyl]-methyl-carbamoyl]-2-methylpropyl]carbamoyl]-2-methyl-propyl/-methyl-carbamoyl]oxymethyl]benzenesulfonate

To a solution of sodium 5-[[(2S)-2-(9H-fluoren-9-ylmethoxycarbonylamino)-5-ureidopentanoyl]amino]-2-[(4-nitrophenoxy)carbonyloxymethyl]benzenesulfonic acid (64.5 mg; 0.0863 mmol) in DMF (1.42 mL), were successively added MMAE (61.9 mg; 0.0863 mmol), DIPEA (75.1 µL; 0.431 mmol) and HOBt (23.3 mg; 0.173 mmol). The reaction mixture was stirred at r.t. for 16 hours. The crude product was purified by Cis reverse phase prep-HPLC by direct deposit of the reaction mixture on the Xbridge^{®} column and using the TFA method to afford the title compound (33.5 mg; 0.0253 mmol). UPLC-MS: MS(ESI): m/z [M+H]+ = 1326.88, [M+Na]+ = 1348.09

### Step 3: 5-[[(2S)-2-amino-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonic acid, TFA salt

To a solution of sodium 5-[[(2S)-2-(9H-fluoren-9-ylmethoxycarbonylamino)-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methylpropyl]carbamoyl]-2-methyl-propyl]-methyl-carbamoyl]oxymethyl]benzenesulfonate (36.8 mg; 0.0292 mmol) in DMF (750 µL), was added piperidine (23.1 µL; 0.234 mmol). The reaction mixture was stirred at r.t. for 17 hours. The crude product solution was purified by Cis reverse phase prep-HPLC by direct deposit of the reaction mixture on the Xbridge^{®} column and using the TFA method to afford the title compound (17 mg; 0.0139 mmol). UPLC-MS: MS(ESI): m/z [M+H]+ = 1038.10, [M+Na]+ = 1059.85

### Step 4: Example 9

To a solution of 5-[[(2S)-2-amino-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1 S)-1-methylpropyl]-4-oxo-butyl]-methyl-carbamoyl]-2-methyl-propyl]carbamoyl]-2-methyl-propyl]-methylcarbamoyl]oxymethyl]benzenesulfonic acid, TFA salt (24.5 mg; 0.0215 mmol) in DMF (735 µL), were successively added **Preparation 1** (10.5 mg; 0.0258 mmol) and DIPEA (19 µL; 0.108 mmol). The reaction mixture was stirred at r.t. for 16 hours. The crude product solution was purified by Cis reverse phase prep-HPLC by direct deposit of the reaction mixture on the Xbridge^{®} column and using the TFA method to afford **Example 9** (19.3 mg; 0.0147 mmol) as a white powder. HR-ESI+: m/z [M+H]⁺ = 1396.7040 / 1396.7074

### [measured/theoretical]

### EXAMPLE 10: 5-[[(2S)-2-[[(2S)-2-[3-[2-(2,5-dioxopyrrol-1-yl)ethoxy]propanoylamino]-3-methyl-butanoyl]amino]propanoyl]amino]-2-[[[(1S)-1-[[(1S)-1-[[(1S,2R)-4-[(2S)-2-[(1R,2R)-3-[[(1R,2S)-2-hydroxy-1-methyl-2-phenylethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonic acid

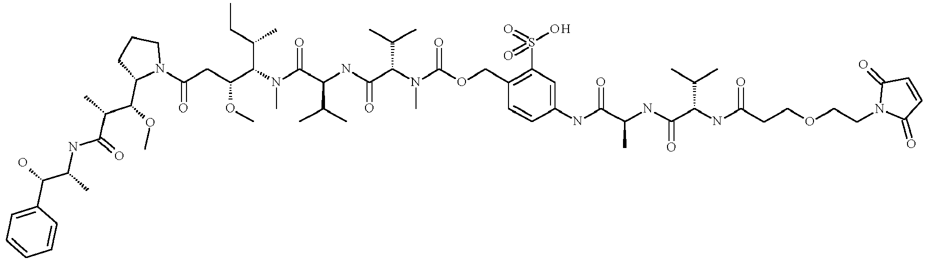

### Step 1: 5-[[(2S)-2-[[(2S)-2-(9H-fluoren-9-ylmethoxycarbonylamino)-3-methyl-butanoyl] amino]propanoyl]amino]-2-[(4-nitrophenoxy)carbonyloxymethyl]benzenesuffonic acid

To a suspension of **Example 2** (1.28 g; 2.07 mmol) in THF (65 mL), was added pyridine (875 µL; 10.8 mmol) followed by 4-nitrophenyl chloroformate (1.09 g; 5.41 mmol). The mixture was stirred overnight at r.t. Then additional 4-nitrophenyl chloroformate (1.09 g; 5.41 mmol; 2.5 eq.) was added. After 5 hours stirring at r.t., the mixture was concentrated to dryness then purified by column chromatography on Cis using water/acetonitrile 90/10 to 0/100 as eluent in 30 minutes. Acetonitrile of the combined tubes was removed, and the rest was lyophilized to afford the title compound (650 mg; 0.83 mmol) as a white solid. ¹H NMR (DMSO): δ 0.88 (m, 6H), 1.31 (d, 3H, J = 4.8 Hz), 1.97-2.03 (m, 1H), 3.92 (t, 1H, J = 6.8 Hz), 4.23 (s, 2H), 4.24-4.34 (m, 1H), 4.42 (t, 1H, J = 5.6 Hz), 5.69 (s, 2H), 7.30-7.48 (m, 6H), 7.62 (d, 2H, J = 8 Hz), 7.72-7.76 (m, 3H), 7.89 (d, 2H, J = 6.4 Hz), 7.94 (s, 1H), 8.18 (d, 1H, J = 5.6 Hz), 8.33 (d, 2H, J = 7.6 Hz), 10.11 (s, 1H). ¹³C NMR (DMSO): δ 18.01, 18.26, 19.21, 30.4, 46.66, 49.05, 59.91, 65.67, 67.82, 117.7, 119.1, 120.06, 122.66, 125.37, 126.33, 127.05, 127.62, 128.0, 138.06, 140.67, 143.77, 143.86, 145.1, 146.23, 151.96, 155.47, 156.12, 171.0, 171.15. LCMS (2-100 ACN/H₂O+0.05% TFA): 90.41 % Tr = 12.7 min. Positiv mode 578.41 detected. Negativ mode 759.17 detected

### Step 2: 5-[[(2S)-2-[[(2S)-2-(9H-fluoren-9-ylmethoxycarbonylamino)-3-methyl-butanoyl] amino]propanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methylpropyl]carbamoyl]-2-methyl-propyl]-methyl-carbamoyl]oxymethyl]benzenesulfonic acid

To a solution of 5-[[(2S)-2-[[(2S)-2-(9H-fluoren-9-ylmethoxycarbonylamino)-3-methyl-butanoyl]amino]propanoyl]amino]-2-[(4-nitrophenoxy)carbonyloxymethyl] benzenesulfonic acid (29.3 mg; 0.0374 mmol) in DMF (1.20 mL), were successively added MMAE (26.9 mg; 0.0374 mmol), DIPEA (32.6 µL; 0.187 mmol) and HOBt (10.1 mg; 0.075 mmol). The reaction mixture was stirred at r.t. for 16 hours. The crude product was purified by C₁₈ reverse phase prep-HPLC by direct deposit of the reaction mixture on the Xbridge^{®} column and using the TFA method to afford the title compound (35.4 mg; 0.0264 mmol). UPLC-MS: MS(ESI): m/z [M+H]+ = 1340.9

### Step 3: 5-[[(2S)-2-[[(2S)-2-amino-3-methyl-butanoyl]amino]propanoyl]amino]-2-[[[(1S)-1-[[(1S)-1-[[(1S,2R)-4-[(2S)-2-[(1R, 2R)-3-[[(1R,2S)-2-hydroxy-1-methyl-2-phenylethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonic acid, TFA salt

To a solution of 5-[[(2S)-2-[[(2S)-2-(9H-fluoren-9-ylmethoxycarbonylamino)-3-methyl-butanoyl]amino]propanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxopropyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl] benzenesulfonic acid (50.1 mg; 0.0374 mmol) in DMF (1.5 mL), was added piperidine (29.6 µL; 0.299 mmol) The reaction mixture was stirred at r.t. for 1 hour. The crude product solution was purified by Cis reverse phase prep-HPLC by direct deposit of the reaction mixture on the Xbridge^{®} column and using the TFA method to afford the title compound (23 mg; 0.0187 mmol) as a white powder. HR-ESI+: m/z [M+H]+ = 1117.6235/1117.6218,

[M+2H]/2+ = 559.3146/559.3148 [measured/theoretical]

### Step 4: Example 10

To a solution of 5-[[(2S)-2-[[(2S)-2-amino-3-methyl-butanoyl]amino]propanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonic acid, TFA salt (10.4 mg; 0.00845 mmol) in DMF (0.34 mL), were successively added (2,5-dioxopyrrolidin-1-yl) 3-[2-(2,5-dioxopyrrol-1-yl)ethoxy]propanoate (2.9 mg; 0.00929 mmol) and DIPEA (7.36 µL; 0.0422 mmol). The reaction mixture was stirred at r.t. for 16 hours. The crude product was purified by Cis reverse phase prep-HPLC by direct deposit of the reaction mixture on the Xbridge^{®} column and using the TFA method to afford **Example 10** (7.7 mg; 0.0059 mmol) as a white powder. HR-ESI+: m/z [M+H]+ = 1312.6701/1312.6750, [measured/theoretical]

### EXAMPLE 11: 5-[[(2S)-2-[[3-[2-[2-(2,5-dioxopyrrol-1-yl)ethoxy] ethylcarbamoyl]oxetane-3-carbonyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonic acid

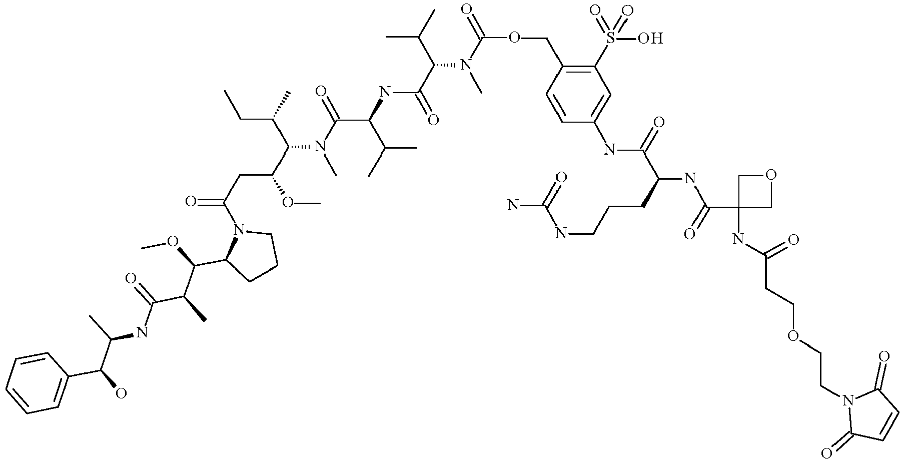

To a solution of 5-[[(2S)-2-amino-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1 S)-1-methylpropyl]-4-oxo-butyl]-methyl-carbamoyl]-2-methyl-propyl]carbamoyl]-2-methyl-propyl]-methylcarbamoyl]oxymethyl]benzenesulfonic acid; 2,2,2-trifluoroacetic acid (16 mg; 0.0131 mmol) in DMF (480 µL), were successively added **Preparation 2** (10.24 mg; 0.0250 mmol) and DIPEA (11.4 µL; 0.0657 mmol). The reaction mixture was stirred at r.t. for 16 hours. The crude product solution was purified by Cis reverse phase prep-HPLC by direct deposit of the reaction mixture on the Xbridge^{®} column and using the TFA method to afford **Example 11** (4.4 mg; 0.0031 mmol) as a white powder. HR-ESI+: m/z [M+2H]2+ = 699.8477 / 699.8472 [measured/theoretical]

### EXAMPLE 12: [5-[[(2S)-2-[[(2S)-2-[3-[2-(2,5-dioxopyrrol-1-yl)ethoxy] propanoylamino]-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]phenyl]methanesulfonic acid

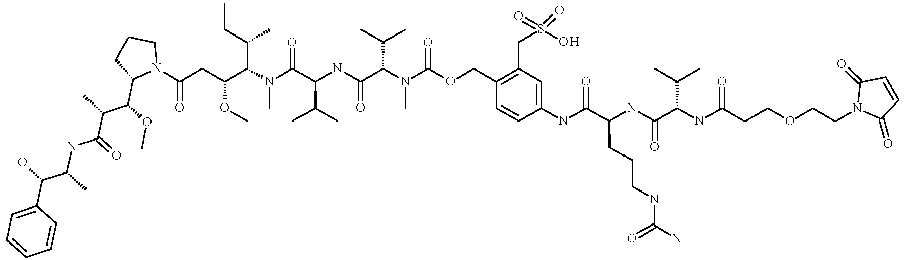

### Step 1: Sodium [5-[[(2S)-2-[[(2S)-2-(9H-fluoren-9-ylmethoxycarbonylamino)-3-methyl-butanoyl]amino]-5-ureido-pentanoyl]amino]-2-[(4-nitrophenoxy)carbonyloxymethyl]phenyl]methanesulfonate

To a solution of **Example 3** (30 mg; 0.0418 mmol) in DMF (0.4 mL) was added DIEA (87 µL; 70.501 mmol) and bis(4-nitrophenyl)carbonate (51 mg; 0.167 mmol). The solution was stirred at r.t. for 2 hours and bis(4-nitrophenyl)carbonate (25 mg; 0.08 mmol) was again added. After 1 hour at r.t., the solution was concentrated under reduced pressure and the residue was purified by silica gel chromatography (gradient of methanol in DCM) to give the title compound (25 mg; 0.030 mmol).

### Step 2: [5-[[(2S)-2-[[(2S)-2-(9H-fluoren-9-ylmethoxycarbonylamino)-3-methyl-butanoyl] amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]phenyl]methanesulfonic acid

To a solution of MMAE (10 mg; 0.0139 mmol) in DMF (0.6 mL), was added DIEA (12 µL; 0.0696 mmol) followed by sodium [5-[[(2S)-2-[[(2S)-2-(9H-fluoren-9-ylmethoxycarbonylamino)-3-methyl-butanoyl]amino]-5-ureido-pentanoyl]amino]-2-[(4-nitrophenoxy)carbonyloxymethyl]phenyl]methanesulfonate (24 mg; 0.028 mmol) and HOBt (3.77 mg; 0.0279 mmol). The solution was stirred at r.t. for 3.5 hours and the solution was directly purified by Cis reverse phase preparative-HPLC on Xbridge^{®} by TFA method to give the title compound (5.4 mg; 0.003 mmol). HRMS (ESI) [M+H]+ found = 1439.7558 (δ = 1.9 ppm)

### Step 3: [5-[[(2S)-2-[[(2S)-2-amino-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]phenyl]methanesulfonic acid, TFA salt

[5-[[(2S)-2-[[(2S)-2-(9H-fluoren-9-ylmethoxycarbonylamino)-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methylpropyl]carbamoyl]-2-methyl-propyl]-methyl-carbamoyl]oxymethyl]phenyl] methanesulfonic acid (5 mg; 3.4 µmol) was disolved in DMF (0.2 mL) then DEA (0.7 µL; 6.8 mmol) was added. The reaction was stirred at r.t. for 1 hour and the solution was purified by Cis reverse phase preparative-HPLC on Xbridge^{®} by TFA method to give the title compound (3.5 mg; 2.6 µmol).

### Step 4: Example 12

To a solution of [5-[[(2S)-2-[[(2S)-2-amino-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl] carbamoyl]-2-methyl-propyl]-methyl-carbamoyl]oxymethyl]phenyl]methanesulfonic acid, TFA salt (3.5 mg; 2.6 µmol) in DMF (15 µL), were added DIEA (2.3 µL; 13 µmmol) and 1-[2-[3-(2,5-dioxopyrrolidin-1-yl)-3-oxo-propoxy]ethyl]pyrrole-2,5-dione (0.9 mg; 2.9 µmol). The solution was stirred at r.t. for 2.5 hours. The reaction was purified by Cis reverse phase preparative-HPLC on Xbridge^{®} column using NH₄HCO₃ method to give **Example 12** (2.8 mg; 1.8 µmol). HRMS (ESI) [M+H]+ found = 1412.7397 (δ = 1.1 ppm)

### EXAMPLE 13: 5-[[(2S)-2-[[(2S)-2-(9H-fluoren-9-ylmethoxycarbonylamino)-3-methyl-butanoyl]amino]-5-ureido-pentanoyl]amino]-2-(iodomethyl)benzenesulfonic acid

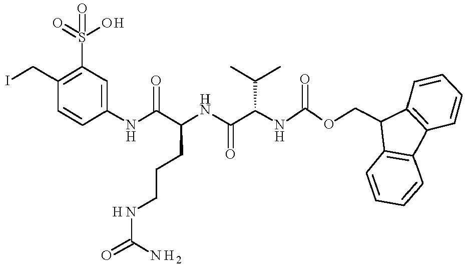

To a solution of **Example 4** (100 mg; 123 µmol) in solution in acetone (6 mL) was added sodium iodide (51 mg; 340 µmol). The reaction mixture was stirred at r.t. for 20 hours. The solvent was evaporated, and the compound was used in the next step without further work-up. UPLC-MS: [M+H]+ 792.65; [M+Na]+ 814.49

### EXAMPLE 14: [4-[[(2S)-2-[[(2S)-2-[3-[2-(2,5-dioxopyrrol-1-yl)ethoxy] propanoylamino]-3-methyl-butanoyl]amino]-5-ureido-pentanoyl]amino]-2-sulfophenyl]methyl-[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-dimethyl-ammonium;2,2,2-trifluoroacetate

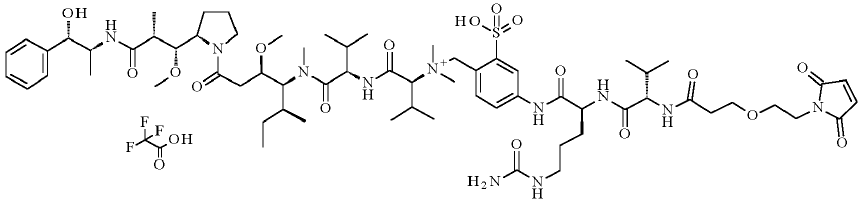

### Step 1: [4-[[(2S)-2-[[(2S)-2-(9H-fluoren-9-ylmethoxycarbonylamino)-3-methyl-butanoyl] amino]-5-ureido-pentanoyl]amino]-2-sulfo-phenyl]methyl-[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-dimethyl-ammonium; iodide

To a solution of (2S)-2-[[(2S)-2-(dimethylamino)-3-methyl-butanoyl]amino]-N-[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-N,3-dimethyl-butanamide (auristatin E) (42.1 mg; 57.5 µmol) in DMF (10 mL), were successively added **Example 13** (152 mg; 95.8 µmol) and DIPEA (83.4 µL; 365 µmol). The reaction was stirred at r.t. for 17 hours. The desired product was observed by UPLC-MS and the solution was used without work-up in the next step. UPLC-MS: [M+H]+ 1396.31; [M+Na]+ 1418.37

### Step 2: [4-[[(2S)-2-[[(2S)-2-amino-3-methyl-butanoyl]amino]-5-ureido-pentanoyl]amino]-2-sulfo-phenyl]methyl-[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-dimethyl-ammonium;2,2,2-trifluoroacetate;2,2,2-trifluoroacetic acid

To the solution of [4-[[(2S)-2-[[(2S)-2-(9H-fluoren-9-ylmethoxycarbonylamino)-3-methyl-butanoyl]amino]-5-ureido-pentanoyl]amino]-2-sulfo-phenyl]methyl-[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-dimethyl-ammonium; iodide in DMF, was added piperidine (45.4 µL; 460 µmol) and the reaction was stirred at r.t. for 1 hour. The crude product was purified by C18 reverse phase prep-HPLC by direct deposit of the reaction mixture on the Xbridge^{®} column and using the TFA method to afford the desired product (15 mg; 10.7 µmol) as a white solid. UPLC-MS: [M+H]+ 1174.51; [M+Na]+ 1197.03

### Step 3: Example 14

To a solution of [4-[[(2S)-2-[[(2S)-2-(9H-fluoren-9-ylmethoxycarbonylamino)-3-methyl-butanoyl]amino]-5-ureido-pentanoyl]amino]-2-sulfo-phenyl]methyl-[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-dimethylammonium; iodide (15 mg; 10.7 µmol) in solution in DMF (450 µL), were successively added (2,5-dioxopyrrolidin-1-yl)-3-[2-(2,5-dioxopyrrol-1-yl)ethoxy]propanoate (3.7 mg; 11.8 µmol) and DIPEA (9.3 µL; 53.5 µmol). The solution was stirred at r.t. for 1 hour. The crude product was purified by C18 reverse phase prep-HPLC by direct deposit of the reaction mixture on the Xbridge^{®} column and using the TFA method to afford **Example 14** (15.7 mg; 12.1 µmol) as a white solid. HRMS (ESI) [M-CF₃CO₂]⁺ 1368.7460 (δ = -1.7 ppm)

### EXAMPLE 23: N-({[4-({N-[6-(2,5-dioxo-2,5-dihydro-1H-pyrrol-1-yl)hexanoyl]-L-valyl-L-alanyl}amino)-2-sulfophenyl]methoxy}carbonyl)-N-methyl-L-valyl-N-[(3R,4S,5S)-1-{(2S)-2-[(1R,2R)-3-{[(1S,2R)-1-hydroxy-1-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamide

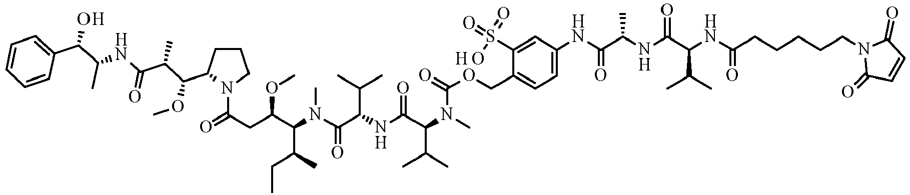

To a solution of 5-[[(2S)-2-[[(2S)-2-amino-3-methyl-butanoyl]amino]propanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonic acid, TFA salt (12.7 mg; 0.0103 mmol; obtained from Step 3 of **Example 10)** in DMF (0.4 mL), were added DIEA (9 µL; 51 µmol) and (2,5-dioxopyrrolidin-1-yl) 6-(2,5-dioxopyrrol-1-yl)hexanoate (3.5 mg; 11.3 µmol). The solution was stirred at r.t. for 6 hours. The reaction was purified by Cis reverse phase preparative-HPLC on Xbridge^{®} column using TFA method to give **Example 23.** IR (cm⁻¹): 3278, 1768/1703, 1631, 1159, 829/696. RMN ¹H (500 MHz, DMSO-d6) δ ppm 9.96 (s, 1H), 8.1 (s, 1H), 7.87 (m, 2H), 7.68 (m, 1H), 7.6 (d, 1H), 7.31 (m, 2H), 7.26 (m, 3H), 7.17 (m, 1H), 6.99 (s, 2H), 5.48 (m, 2H), 4.74 (m, 1H), 4.49 (m, 1H), 4.46 (m, 1H), 4.41 (m, 1H), 4.38 (m, 1H), 4.33 (m, 1H), 4.19 (m, 1H), 3.98 (m, 1H), 3.98 (m, 1H), 3.77 (m, 1H), 3.6 (m, 2H), 3.56 (m, 1H), 3.46 (m, 1H), 3.36 (m, 2H), 3.32 (m, 1H), 3.26 (m, 2H), 3.24 (m, 3H), 3.23 (m, 2H), 3.19 (m, 3H), 3.13 (m, 2H), 3.04 (m, 1H), 2.98 (m, 3H), 2.94 (m, 1H), 2.9 (m, 1H), 2.86 (m, 1H), 2.37 (br dd, 2H), 2.12 (m, 2H), 2.12 (m, 2H), 2.06 (m, 2H), 1.97 (m, 2H), 1.49 (m, 9H), 1.29 (d, 3H), 0.89 (m, 27H). HRMS (ESI)

[M+H]⁺ found = 1310.6893 (δ = -4.5 ppm)

### EXAMPLE 24: N-[6-(2,5-dioxo-2,5-dihydro-1H-pyrrol-1-yl)hexanoyl]-L-valyl-H⁵-carbamoyl-N-(4-{[({[(4S)-4,11-diethyl-9-hydroxy-3,14-dioxo-3,4,12,14-tetrahydro-1H-pyrano[3',4':6,7]indolizino[1,2-b]quinolin-4-yl]oxy}carbonyl)oxy]methyl}-3-sulfophenyl)-L-ornithinamide

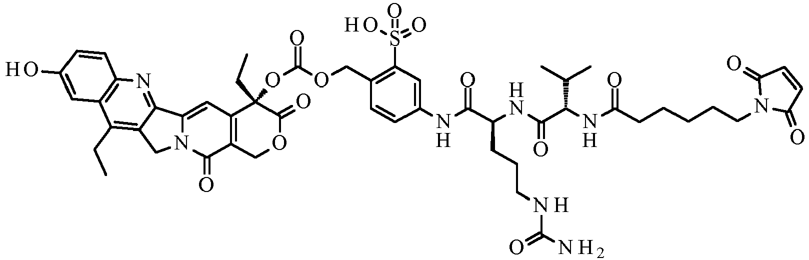

### Step 1: 5-[[(2S)-2-[[(2S)-2-amino-3-methyl-butanoyl]amino]-5-ureido-pentanoyl]amino]-2-(hydroxymethyl)benzenesulfonic acid

To a solution of **Example 1** (200 mg; 0.284 mmol) in DMF (8 mL), was added DEA (212 µL; 2.053 mmol) and the reaction was stirred at r.t. for 1 hour. The excess of DEA was evaporated under vacuum and the solution of crude expected compound in DMF was use directly in the next step.

### Step 2: 5-[[(2S)-2-[[(2S)-2-[6-(2,5-dioxopyrrol-1-yl)hexanoylamino]-3-methyl-butanoyl]amino]-5-ureido-pentanoyl]amino]-2-(hydroxymethyl)benzenesulfonic acid

To the solution (8 mL) of the compound from the previous step, were added DIEA (102 µL; 0.5866 mmol) and (2,5-dioxopyrrolidin-1-yl) 6-(2,5-dioxopyrrol-1-yl)hexanoate (99.4 mg; 0.3226 mmol). The reaction was stirred at r.t. for 18 hours. The reaction was purified by Cis reverse phase preparative-HPLC on Xbridge^{®} column using TFA method to give after lyophilization the expected compound. IR (cm⁻¹): between 3674 and 2999, 1768 (weak) + 1699 + 1641, 1238 / 1141, 825 and 694. RMN ¹H (400 MHz, DMSO-d6) δ ppm 9.97 (s, 1H), 8.04 (d, 1H), 7.85 (d, 1H), 7.77 (d, 1H), 7.65 (dd, 1H), 7.32 (d, 1H), 6.99 (s, 2H), 6.02 (m, 1H), 5.18 (m, 1H), 4.72 (s, 2H), 4.36 (m, 1H), 4.21 (dd, 1H), 3.37 (t, 2H), 2.98 (m, 2H), 2.16 (m, 2H), 1.97 (m, 1H), 1.65 (m, 2H), 1.39 (m, 8H), 0.83 (dd, 6H). HRMS (ESI) [M+H]+ found = 653.2572 (δ = -4.2 ppm)

### Step 3: tert-butyl [(19S)-10,19-diethyl-19-hydroxy-14,18-dioxo-17-oxa-3,13-diazapentacyclo[11.8.0.02,11.04,9.015,20]henicosa-1(21),2,4(9),5,7,10,15(20)-heptaen-7-yl] carbonate

To a suspension of (19S)-10,19-diethyl-7,19-dihydroxy-17-oxa-3,13-diazapentacyclo [11.8.0.02,11.04,9.015,20]henicosa-1(21),2,4(9),5,7,10,15(20)-heptaene-14,18-dione (220 mg; 0.5607 mmol) in DCM (22 mL), were added tert-butoxycarbonyl tert-butyl carbonate (128.5 mg; 0.5887 mmol) as a powder and pyridine (91 µL; 1.121 mmol). The yellow suspension was stirred at r.t. for 18 hours. The solvent and excess of pyridine were evaporated off under vacuum and the crude was purified by chromatography on silica (DCM/MeOH) to give the expected compound as a yellow powder. IR (cm⁻¹): 3700-3000, 1753, 1659, 1254/1142. RMN ¹H (400 MHz, DMSO-d6) δ ppm 8.21 (d, 1H), 8.09 (d, 1H), 7.74 (dd, 1H), 7.33 (s, 1H), 6.5 (s, 1H), 5.38 (2s, 4H), 3.2 (q, 2H), 1.86 (m, 2H), 1.54 (s, 9H), 1.3 (t, 3H), 0.88 (t, 3H)

### Step 4: tert-butyl [(19S)-19-chlorocarbonyloxy-10,19-diethyl-14,18-dioxo-17-oxa-3,13-diazapentacyclo[11.8.0.02,11.04,9.015,20]henicosa-1(21),2,4(9),5,7,10,15(20)-heptaen-7-yl] carbonate

To a solution of tert-butyl [(19S)-10,19-diethyl-19-hydroxy-14,18-dioxo-17-oxa-3,13-diazapentacyclo[11.8.0.02,11.04,9.015,20]henicosa-1(21),2,4(9),5,7,10,15(20)-heptaen-7-yl] carbonate (30 mg; 0.0609 mmol) in DCM (2 mL), were added DMAP (22.3 mg; 0.1827 mmol) followed by triphosgene (7.2 mg; 0.0244 mmol). The reaction was stirred at r.t. for 30 minutes and this solution was used as such in next step.

### Step 5: 2-[[(19S)-7-tert-butoxycarbonyloxy-10,19-diethyl-14,18-dioxo-17-oxa-3,13-diazapentacyclo[11.8.0.02,11.04,9.015,20]henicosa-1(21),2,4(9),5,7,10,15(20)-heptaen-19-yl]oxycarbonyloxymethyl]-5-[[(2S)-2-[[(2S)-2-[6-(2,5-dioxopyrrol-1-yl)hexanoylamino]-3-methyl-butanoyl]amino]-5-ureido-pentanoyl]amino]benzenesulfonic acid

To a solution of tert-butyl [(19S)-19-chlorocarbonyloxy-10,19-diethyl-14,18-dioxo-17-oxa-3,13-diazapentacyclo[11.8.0.02,11.04,9.015,20]henicosa-1(21),2,4(9),5,7,10,15(20)-heptaen-7-yl] carbonate in solution in DCM (2 mL), were added DMAP (22.3 mg; 0.1827 mmol) followed by 5-[[(2S)-2-[[(2S)-2-[6-(2,5-dioxopyrrol-1-yl)hexanoylamino]-3-methyl-butanoyl]amino]-5-ureido-pentanoyl]amino]-2-(hydroxymethyl)benzenesulfonic acid (31.80 mg; 0.04873 mmol; obtained according to Step 2 above). The reaction was stirred at r.t. for 14 hours. The reaction was purified by Cis reverse phase preparative-HPLC on CSH^{®} column using TFA method to give after lyophilization the expected compound.

### Step 6: Example 24

To a solution of 2-[[(19S)-7-tert-butoxycarbonyloxy-10,19-diethyl-14,18-dioxo-17-oxa-3,13-diazapentacyclo[11.8.0.02,11.04,9.015,20]henicosa-1(21),2,4(9),5,7,10,15(20)-heptaen-19-yl]oxycarbonyloxymethyl]-5-[[(2S)-2-[[(2S)-2-[6-(2,5-dioxopyrrol-1-yl)hexanoylamino]-3-methyl-butanoyl]amino]-5-ureido-pentanoyl]amino]benzenesulfonic acid (12.5 mg; 1.66 µmol) in DCM (2.5 mL), was added TFA (0.3 mL) and the solution was stirred at r.t. for 1.5 hours. The reaction was purified by Cis reverse phase preparative-HPLC on CSH^{®} column using TFA method to give after lyophilization **Example 24.** HRMS (ESI)

[M+H]+ found = 1071.3768 (δ = 0.4 ppm)

### EXAMPLE 25: (1S,3S)-3,5,12-trihydroxy-3-(hydroxyacetyl)-10-methoxy-6,11-dioxo-1,2,3,4,6,11-hexahydrotetracen-1-yl 2,3,6-trideoxy-3-[({[4-({N-[6-(2,5-dioxo-2,5-dihydro-1H-pyrrol-1-yl)hexanoyl]-L-valyl-N⁶-carbamoyl-L-ornithyl}amino)-2-sulfophenyl]methoxy}carbonyl)amino]-α-L-lyxo-hexopyranoside

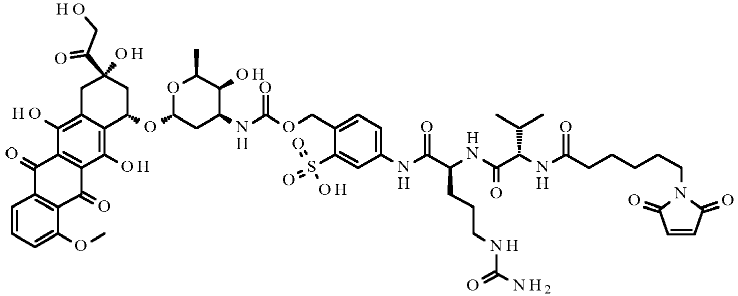

### Step 1: 5-[[(2S)-2-[[(2S)-2-(9H-fluoren-9-ylmethoxycarbonylamino)-3-methyl-butanoyl]amino]-5-ureido-pentanoyl]amino]-2-[[(2S, 3S, 4S, 6R)-3-hydroxy-2-methyl-6-[[(1S,3S)-3,5,12-trihydroxy-3-(2-hydroxyacetyl)-10-methoxy-6,11-dioxo-2,4-dihydro-1H-tetracen-1-yl]oxy]tetrahydropyran-4-yl]carbamoyloxymethyl]benzenesulfonic acid

To a solution of sodium 5-[[(2S)-2-[[(2S)-2-(9H-fluoren-9-ylmethoxycarbonylamino)-3-methyl-butanoyl]amino]-5-ureido-pentanoyl]amino]-2-[(4-nitrophenoxy) carbonyloxymethyl]benzenesulfonate (157 mg; 0.1854 mmol; obtained from Step 1 of **Example 6)** in DMF (11 mL), were added (7S,9S)-7-[(2R,4S,5S,6S)-4-amino-5-hydroxy-6-methyl-tetrahydropyran-2-yl]oxy-6,9,11-trihydroxy-9-(2-hydroxyacetyl)-4-methoxy-8,10-dihydro-7H-tetracene-5,12-dione (107.5 mg; 0.1853 mmol) followed by DIEA (323 µL; 1.854 mmol). The red mixture turned to dark purple and, after 2 hours conversion, was complete. This solution was used as such in the next step.

### Step 2 : 5-[[(2S)-2-[[(2S)-2-amino-3-methyl-butanoyl]amino]-5-ureido-pentanoyl]amino]-2-[[(2S,3S,4S,6R)-3-hydroxy-2-methyl-6-[[(1S,3S)-3,5,12-trihydroxy-3-(2-hydroxyacetyl)-10-methoxy-6,11-dioxo-2,4-dihydro-1H-tetracen-1-yl]oxy]tetrahydropyran-4-yl]carbamoyloxymethyl]benzenesulfonic acid

To the solution of 5-[[(2S)-2-[[(2S)-2-(9H-fluoren-9-ylmethoxycarbonylamino)-3-methyl-butanoyl]amino]-5-ureido-pentanoyl]amino]-2-[[(2S,3S,4S,6R)-3-hydroxy-2-methyl-6-[[(1S,3S)-3,5,12-trihydroxy-3-(2-hydroxyacetyl)-10-methoxy-6,11-dioxo-2,4-dihydro-1H-tetracen-1-yl]oxy]tetrahydropyran-4-yl]carbamoyloxymethyl]benzenesulfonic acid as prepared in previous step, was added DEA (96 µL; 0.9268 mmol). The reaction was stirred at r.t. for 1 hour. Solvent was partially evaporated off and the crude was purified by Cis reverse phase preparative-HPLC on X-Bridge^{®} column using TFA method to give after lyophilization the expected compound. IR (cm⁻¹): 3357, 2661, 1666/1608, 1585/1531, 1201, 1082, 1020, 763/709. RMN ¹H (500 MHz, DMSO-d6) δ ppm 14.06 (s, 1H), 13.29 (s, 1H), 10.15 (s, 1H), 8.62 (d, 1H), 8.04 (m, 3H), 7.93 (d, 1H), 7.93 (t, 1H), 7.89 (d, 1H), 7.66 (dd, 1H), 7.61 (dd, 1H), 7.26 (d, 1H), 6.88 (d, 1H), 5.99 (t, 1H), 5.49 (brs, 1H), 5.37 (m, 2H), 5.25 (brs, 1H), 4.97 (t, 1H), 4.79 (brm, 2H), 4.58 (s, 2H), 4.46 (q, 1H), 4.18 (q, 1H), 3.99 (s, 3H), 3.75 (m, 1H), 3.63 (m, 1H), 3.51 (brs, 1H), 2.99 (m, 2H), 2.99 (m, 2H), 2.16 (m, 2H), 2.07 (m, 1H), 1.87 (td, 1H), 1.72 (m, 1H), 1.61 (m, 1H), 1.5 (dt, 1H), 1.45 (m, 1H), 1.37 (m, 1H), 1.14 (d, 3H), 0.93 (2d, 6H)

### Step 3: Example 25

To a solution of 5-[[(2S)-2-[[(2S)-2-amino-3-methyl-butanoyl]amino]-5-ureido-pentanoyl] amino]-2-[[(2S,3S,4S,6R)-3-hydroxy-2-methyl-6-[[(1S,3S)-3,5,12-trihydroxy-3-(2-hydroxyacetyl)-10-methoxy-6,11-dioxo-2,4-dihydro-1H-tetracen-1-yl]oxy] tetrahydropyran-4-yl]carbamoyloxymethyl]benzenesulfonic acid (5 mg; 4.7 µmol) in DMF (1 mL), were added DIEA (1.6 µL; 5.2 µmmol) and (2,5-dioxopyrrolidin-1-yl) 6-(2,5-dioxopyrrol-1-yl)hexanoate (1.6 mg; 5.2 µmol). The solution was stirred at r.t. for 12 hours. The reaction was purified by Cis reverse phase preparative-HPLC on Xbridge^{®} column using TFA method to give **Example 25.** HRMS (ESI) [M+H]⁺ found = 1222.4153 (δ = 1.7 ppm)

### EXAMPLE 26: sodium N-{[(9H-fluoren-9-yl)methoxy]carbonyl}-L-valyl-N⁵-carbamoyl-N-[4-(hydroxymethyl)-3-(2-sulfonatoethyl)phenyl]-L-ornithinamide

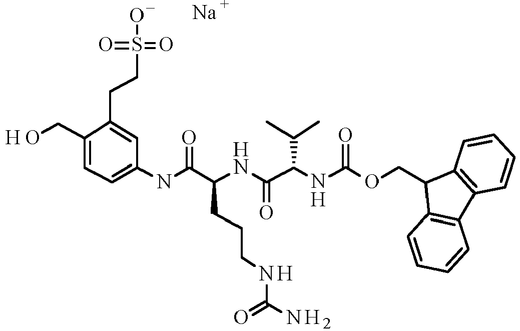

### Step 1: (2-allyl-4-nitro-phenyl)methoxy-tert-butyl-dimethyl-silane

A solution of tert-butyl-[(2-iodo-4-nitro-phenyl)methoxy]-dimethyl-silane (6.56 g; 16.68 mmol) and allyltributyltin (7.76 mL; 25.02 mmol) in 1,4-dioxane (165 mL) was purged with argon (3 times). Tetrakis(triphenylphosphine)palladium(0) (1.93 g; 1.67 mmol) was added and the mixture was purged again with argon (3 times) and stirred at 100°C for 18 hours. The reaction mixture was filtered over Celite^{®} and concentrated to dryness. The residue was dissolved in DCM (150 mL) and washed with a 1M aqueous solution of sodium hydroxyde. The aqueous layer was extracted with DCM and the combined organic layers were washed with brine, dried over sodium sulfate, filtered and concentrated to dryness. The resulting crude was purified by flash chromatography over silica gel using cyclohexane / EtOAc (10/0 to 95/5) as eluent to afford the expected compound (4.91 g; 15.97 mmol) as an orange oil. ¹H NMR (DMSO): δ 0.11 (s, 6H), 0.92 (s, 9H), 3.49 (d, 2H, J = 6.5 Hz), 4.84 (s, 2H), 5.04-5.15 (m, 2H), 5.92-6.02 (m, 1H), 7.69 (d, 1H, J = 8.5 Hz), 8.02 (d, 1H, J = 2.5 Hz), 8.13 (dd, 1H, J = 2.5 and 8.5 Hz)

### Step 2: 3-[2-[[tert-butyl(dimethyl)silyl]oxymethyl]-5-nitro-phenyl]propane-1,2-diol

To a solution of (2-allyl-4-nitro-phenyl)methoxy-tert-butyl-dimethyl-silane (4.91 g; 15.97 mmol) in a mixture of acetone (147 mL) and water (20 mL), were added 4-methylmorpholine *N*-oxide (3.74 g; 31.94 mmol) and potassium osmate (VI) dihydrate (0.29 g; 0.8 mmol) and the mixture was stirred at r.t. for 20 hours. The reaction mixture was quenched with a saturated aqueous solution of sodium thiosulfate (40 mL) and water (40 mL) and the aqueous layer was extracted with EtOAc. The combined organic layers were washed with brine, dried over sodium sulfate, filtered, and concentrated to dryness. The resulting crude was purified by flash chromatography over silica gel using cyclohexane / EtOAc (10/0 to 5/5) as eluent to afford the expected compound (4.65 g; 13.62 mmol) as a yellow oil. ¹H NMR (DMSO): δ 0.11 (s, 6H), 0.93 (s, 9H), 2.58 (dd, 1H, J = 8.8 and 14.2 Hz), 2.9 (dd, 1H, J = 3.5 and 14.2 Hz), 3.26-3.30 (m, 1H), 3.36-3.41 (m, 1H), 3.61-3.68 (m, 1H), 4.7 (t, 1H, J = 5.7 Hz), 4.75 (d, 1H, J = 5.4 Hz), 4.84-4.93 (m, 2H), 7.65-7.67 (m, 1H), 8.08-8.11 (m, 2H)

### Step 3: 2-[2-[[tert-butyl(dimethyl)silyl]oxymethyl]-5-nitro-phenyl]acetaldehyde

To a solution of 3-[2-[[tert-butyl(dimethyl)silyl]oxymethyl]-5-nitro-phenyl]propane-1,2-diol (3.05 g; 8.22 mmol) in a mixture of water (18 mL) and THF (51 mL) at 0°C, was added sodium periodate (5.27 g; 24.65 mmol) portion-wise. The reaction mixture was stirred for 2 hours from 0°C to 10°C, then quenched with a saturated aqueous solution of sodium thiosulfate (50 mL) and water (50 mL). The aqueous layer was extracted with EtOAc. The combined organic layers were washed with brine, dried over sodium sulfate, filtered and concentrated to dryness at 30°C to afford the expected compound (2.61 g; 8.22 mmol) as a yellow oil used as such for the next step. ¹H NMR (DMSO): δ 0.08 (s, 6H), 0.90 (s, 9H), 4.04 (s, 2H), 4.72 (s, 2H), 7.7 (d, 1H, J = 8.8 Hz), 8.13 (d, 1H, J = 2.5 Hz), 8.18 (dd, 1H, J = 2.4 and 8.5 Hz), 9.69 (s, 1H)

### Step 4: 2-[2-[[tert-butyl(dimethyl)silyl]oxymethyl]-5-nitro-phenyl]ethanol

To a solution of 2-[2-[[tert-butyl(dimethyl)silyl]oxymethyl]-5-nitro-phenyl]acetaldehyde (2.61 g; ) in MeOH (40 mL), was added at 0°C sodium borohydride (0.47g; 12.33 mmol). The reaction mixture was warmed to r.t. and stirred for 1 hour, then quenched by addition of brine (25 mL) and water (25 mL). The aqueous layer was extracted with EtOAc then the combined organic layers were washed with brine, dried over sodium sulfate, filtered, and concentrated to dryness. The resulting crude was purified by flash chromatography over silica gel using cyclohexane / EtOAc (10/0 to 6/4) to afford the expected compound (1.36 g; 4.36 mmol) as a yellow solid. ¹H NMR (DMSO): δ 0.11 (s, 6H), 0.92 (s, 9H), 2.82 (t, 2H, J = 6.4 Hz), 3.67 (q, 2H, J = 5.9 Hz), 4.74 (t, 1H, J = 5.1 Hz), 4.87 (d, 2H), 7.66 (d, 1H, J = 8.6 Hz), 8.08-8.11 (m, 2H)

### Step 5: [2-(2-bromoethyl)-4-nitro-phenyl]methoxy-tert-butyl-dimethyl-silane

To a solution of 2-[2-[[tert-butyl(dimethyl)silyl]oxymethyl]-5-nitro-phenyl]ethanol (1.26 g; 4.05 mmol) in THF (25 mL) at 0°C, were added TEA (1.12 mL; 8.09 mmol) and mesyl chloride (0.47 mL; 6.07 mmol). The reaction mixture was allowed to warm slowly to r.t., stirred for 5 hours, then filtered and rinsed with THF. The filtrate was added to a solution of lithium bromide (1.76 g; 20.23 mmol) in THF (25 mL) at 0°C. The reaction mixture was allowed to warm slowly to r.t., stirred for 4 days, then quenched with a saturated aqueous solution of ammonium chloride (50 mL). The aqueous layer was extracted with EtOAc. The combined organic layers were washed with brine, dried over sodium sulfate, filtered and concentrated to dryness to afford the expected compound (1.38 g; 4.03 mmol) as a yellow oil used as such for the next step. ¹H NMR (DMSO): δ 0.12 (s, 6H), 0.92 (s, 9H), 3.27 (t, 2H, J = 7.2 Hz), 3.81 (t, 2H, J = 7.2 Hz), 4.87 (s, 2H), 7.68 (d, 1H, J = 8.4 Hz), 8.13-8.16 (m, 1H), 8.18-8.19 (m, 1H)

### Step 6: [2-(2-bromoethyl)-4-nitro-phenyl]methanol

To a solution of [2-(2-bromoethyl)-4-nitro-phenyl]methoxy-tert-butyl-dimethyl-silane (1.38 g) in THF (28 mL) and water (14 mL) at 0°C, was added acetic acid (42 mL; 737.28 mmol). The reaction mixture was allowed to warm slowly to r.t., stirred for 4 days, then diluted with water (100 mL). The aqueous layer was extracted with EtOAc. The combined organic layers were washed with brine, dried over sodium sulfate, filtered and concentrated to dryness. The resulting crude was purified by flash chromatography over silica gel using cyclohexane / EtOAc (10/0 to 6/4) as eluent to afford the expected compound (0.73 g; 2.81 mmol) as a beige solid. ¹H NMR (DMSO): δ 3.27 (t, 2H, J = 7.2 Hz), 3.8 (t, 2H, J = 7.2 Hz), 4.67 (s, 2H), 5.51 (brs, 1H), 7.7 (d, 1H, J = 8.4 Hz), 8.11-8.14 (m, 1H), 8.15-8.16 (m, 1H)

### Step 7: 2-[2-(hydroxymethyl)-5-nitro-phenyl]ethylsulfonyloxysodium

To a solution of [2-(2-bromoethyl)-4-nitro-phenyl]methanol (188 mg; 0.72 mmol) in EtOH (750 µL), were successively added a solution of sodium sulfite (273 mg; 2.17 mmol) in water (1.1 mL) and tetrabutylammonium iodide (13 mg; 0.036 mmol). The reaction mixture was heated at 70°C under micro-waves irradiation for 35 minutes. The reaction mixture was diluted with water (20 mL) and washed with EtOAc. The aqueous layer was concentrated to dryness and the resulting crude was purified by flash chromatography over silica gel using DCM / MeOH (10/0 to 6/4) as eluent and then by flash chromatography over Cis using ACN / water (2/98 to 34/66) as eluent to afford the expected compound (80 mg; 0.28 mmol) as a white solid. ¹H NMR (DMSO): δ 2.66-2.70 (m, 2H), 2.94-2.98 (m, 2H), 4.64 (d, 2H, J = 5.3 Hz), 5.47 (t, 1H, J = 5.3 Hz), 7.68 (d, 1H, J = 8.5 Hz), 7.99 (d, 1H, J = 2.3 Hz), 8.06 (dd, 1H, J = 2.4 and 8.4 Hz)

### Step 8: 2-[5-amino-2-(hydroxymethyl)phenyl]ethylsulfonyloxysodium

To a solution of [2-(2-bromoethyl)-4-nitro-phenyl]methanol (293 mg; 1.03 mmol) in MeOH (11 mL) and water (2 mL), was added Pd/C 10% (55 mg; 0.052 mmol) under argon. The reaction mixture was purged with hydrogen and stirred at r.t. under atmospheric pressure of hydrogen for 16 hours. The reaction mixture was filtered over a 40 µm PTFE filter, rinsed with a mixture of MeOH / water (1/1) and concentrated to dryness to afford the expected compound (274 mg; 1.03 mmol) as a yellow solid used as such for the next step. ¹H NMR (DMSO): δ 2.55-2.59 (m, 2H), 2.75-2.79 (m, 2H), 4.32 (d, 2H, J = 5.3 Hz), 5.66 (t, 1H, J = 5.2 Hz), 4.86 (brs, 2H), 6.33-6.35 (m, 2H), 6.93 (d, 1H, J = 7.8 Hz)

### Step 9: Example 26

To a solution of 2-[5-amino-2-(hydroxymethyl)phenyl]ethylsulfonyloxysodium (304 mg; 1.15 mmol) in DMF (6 mL), were added (2S)-2-[[(2S)-2-(9H-fluoren-9-ylmethoxycarbonylamino)-3-methyl-butanoyl]amino]-5-ureido-pentanoic acid (474 mg; 0.96 mmol), HATU (472 mg; 1.24 mmol) and sodium hydrogenocarbonate (160 mg; 1.91 mmol). The reaction mixture was stirred at r.t. for 20 hours, diluted with water (50 mL) and washed with EtOAc. The aqueous layer was concentrated to dryness and purified by flash chromatography over Cis using ACN / water + TFA (0.1%) (2/98 to 50/50) as eluent to afford after freeze-drying, **Example 26** (83 mg; 0.11 mmol) as a pale yellow solid. ¹H NMR (DMSO): δ 0.84-0.88 (m, 6H), 1.32-1.49 (m, 2H), 1.54-1.74 (m, 2H), 1.94-2.04 (m, 1H), 2.58-2.62 (m, 2H), 2.83-2.87 (m, 2H), 2.90-3.05 (m, 2H), 3.93 (t, 1H, J = 8 Hz), 4.23-4.34 (m, 3H), 4.37-4.43 (m, 1H), 4.45 (s, 2H), 5.34 (brs, 2H), 5.98 (brs, 1H), 7.25 (d, 1H, J = 8.1 Hz), 7.30-7.34 (m, 2H), 7.39-7.44 (m, 4H), 7.74 (t, 2H, J = 7.2 Hz), 7.89 (d, 2H, J = 7.5 Hz), 8.08 (d, 1H, J = 7.5 Hz), 9.90 (s, 1H). LCMS (2-100 ACN / H₂O + 0.1% TFA) : 76.60% Tr = 8.1 min. Negative mode 708.45 detected (M-H)

### EXAMPLE 27: N-{3-[2-(2,5-dioxo-2,5-dihydro-1H-pyrrol-1-yl)ethoxy]propanoyl}-L-valyl-N-{4-[(5S,8S,11S,12R)-11-[(2S)-butan-2-yl]-12-(2-{(2S)-2-[(1R,2R)-3-{[(1S,2R)-1-hydroxy-1-phenylpropan-2-yl]amino}-1-methoxy-2-methyl-3-oxopropyl]pyrrolidin-1-yl}-2-oxoethyl)-4,10-dimethyl-3,6,9-trioxo-5,8-di(propan-2-yl)-2,13-dioxa-4,7,10-triazatetradecan-1-yl]-3-(2-sulfoethyl)phenyl}-N⁵-carbamoyl-L-ornithinamide

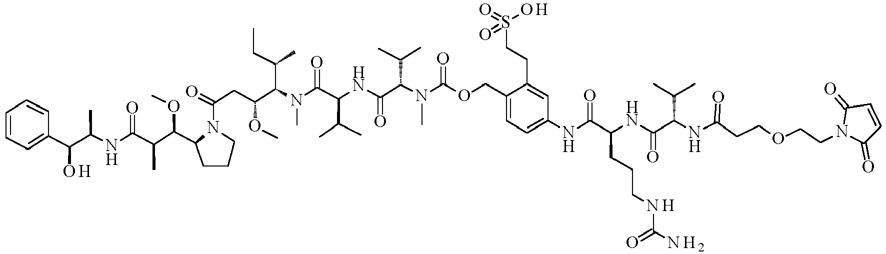

### Step 1: 2-[5-[[(2S)-2-[[(2S)-2-(9H-fluoren-9-ylmethoxycarbonylamino)-3-methyl-butanoyl]amino]-5-ureido-pentanoyl]amino]-2-[(4-nitrophenoxy)carbonyloxymethyl]phenyl]ethanesulfonic acid

To a solution of **Example 26** (32 mg, 0.0436 mmol) in DMF (0.5 mL), were added bis(4-nitrophenyl) carbonate (53.1 mg; 0.1747 mmol) and DIEA (91 µL; 0.5240 mmol). The solution was stirred at r.t. for 4 hours. After evaporation to dryness under vacuum the crude was purified by chromatography on silica gel to lead to the expected compound.

### Step 2: 2-[5-[[(2S)-2-[[(2S)-2-(9H-fluoren-9-ylmethoxycarbonylamino)-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methylcarbamoyl]oxymethyl]phenyl]ethanesulfonic acid

To a solution of MMAE (17 mg, 0.02368 mmol) in DMF (6 mL), were added 2-[5-[[(2S)-2-[[(2S)-2-(9H-fluoren-9-ylmethoxycarbonylamino)-3-methyl-butanoyl]amino]-5-ureido-pentanoyl]amino]-2-[(4-nitrophenoxy)carbonyloxymethyl]phenyl]ethanesulfonic acid (41.4 mg; 0.0473 mmol), DIEA (20 µL; 0.1184 mmol) and HOBt (6.4 mg; 0.047 mmol). The reaction was stirred at r.t. overnight and this solution was engaged as such in the next step.

### Step 3: 2-[5-[[(2S)-2-[[(2S)-2-amino-3-methyl-butanoyl]amino]-5-ureido-pentanoyl] amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl] carbamoyl]-2-methyl-propyl]-methyl-carbamoyl]oxymethyl]phenyl]ethanesulfonic acid, TFA

To the solution of 2-[5-[[(2S)-2-[[(2S)-2-(9H-fluoren-9-ylmethoxycarbonylamino)-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl] oxymethyl]phenyl]ethanesulfonic acid was added DEA (24 µL; 0.2338 mmol) and the solution was stirred at r.t. for 1.5 hours. The reaction was purified by Cis reverse phase preparative-HPLC on CSH^{®} column using TFA method to give after lyophilization the expected compound.

### Step 4: Example 27

To a solution of 2-[5-[[(2S)-2-[[(2S)-2-amino-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]phenyl]ethanesulfonic acid, TFA (9.5 mg; 7.1 mmol) in DMF (0.2 mL), was added DIEA (2.5 µL; 0.014 mmol) followed by (2,5-dioxopyrrolidin-1-yl) 3-[2-(2,5-dioxopyrrol-1-yl)ethoxy]propanoate (2.4 mg; 7.8 µmol). The reaction was stirred at r.t. overnight. The reaction was purified by Cis reverse phase preparative-HPLC on CSH^{®} column using TFA method to give after lyophilization **Example 27.** HRMS (ESI) [M+H]⁺ found = 1426.7443 (δ = -6.7 ppm)

### EXAMPLE 28: 5-[[(2S)-2-[[(2S)-2-[6-(3,4-dibromo-2,5-dioxo-pyrrol-1-yl)hexanoylamino]-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonic acid

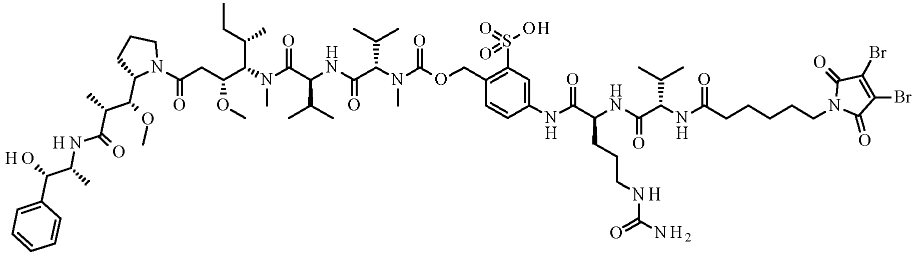

To a solution of 6-(3,4-dibromo-2,5-dioxo-pyrrol-1-yl)hexanoic acid in solution in DMF (0.2 mL) were added HATU (11.3 mg; 29.8 µmol) as a powder. The reaction was stirred at r.t. for 10 minutes and lutidine (6.3 µL; 54.2 mmol) was added. The reaction was stirred at r.t. for 2 hours. Then 5-[[(2S)-2-[[(2S)-2-amino-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonic acid (10 mg; 8.3 µmol; obtained from Step 2 of **Example 6)** was added and the reaction was stirred at r.t. for 15 hours. The reaction was purified by Cis reverse phase preparative-HPLC on X-Bridge^{®} column using TFA method to give after lyophilization the expected compound. HRMS (ESI) [M+H]⁺ found = 1552.5595 (δ = -2.5 ppm)

### EXAMPLE 34: N-{[(9H-fluoren-9-yl)methoxy]carbonyl}-L-valyl-N⁵-carbamoyl-N-[4-(hydroxymethyl)-3-(3-sulfopropyl)phenyl]-L-ornithinamide

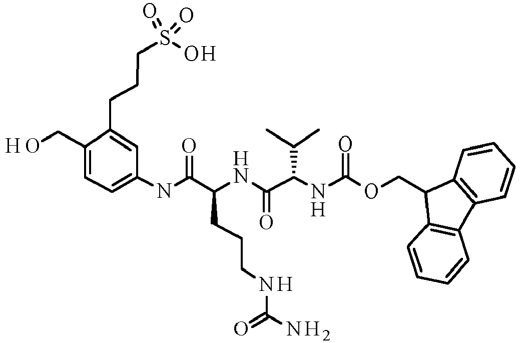

### Step 1: 2-iodo-4-nitrobenzoic acid

To a solution of 2-amino-4-nitrobenzoic acid (10.0 g; 54.9 mmol) in ACN (280 mL), was added p-toluenesulfonic acid monohydrate (32.0 g; 168 mmol). The mixture was stirred at r.t. for 15 minutes, then a solution of sodium nitrite (8.00 g; 115.9 mmol) and potassium iodide (24.0 g; 144.6 mmol) in water (140 mL) were added dropwise in 15 minutes. The reaction mixture was stirred for 19 hours. After the completion of the reaction, the mixture was quenched with sodium thiosulfate (13.02 g; 82.36 mmol) and acidified with an aqueous solution of hydrogen chloride 3N (25 mL). The aqueous layer was extracted with DCM and the combined organic layers were washed with an aqueous solution of hydrogen chloride 1N, dried over sodium sulfate, filtered and concentrated to dryness to afford compound of the title (15.0 g; 51.2 mmol) as an orange powder. ¹H NMR (DMSO): 7.86 (d, 1H, J = 8.4 Hz), 8.27 (d, 1H, J = 8.4 Hz), 8.64 (s, 1H), 13.8 (brs, 1H)

### Step 2: (2-iodo-4-nitrophenyl)methanol

To a solution of 2-iodo-4-nitrobenzoic acid (5.00 g; 17.1 mmol) in THF (70 mL), was added a solution of borane 1N in THF (85 mL; 85.0 mmol). The reaction mixture was stirred at 65°C for 4 hours. After the completion of the reaction, the reaction mixture was cooled down to r.t. and quenched with MeOH (200 mL). The mixture was stirred at r.t. for 30 minutes then was concentrated to dryness. The resulting residue was purified by column chromatography on silica gel using cyclohexane / EtOAc (80/20 to 50/50) as eluent to afford compound of the title (3.38 g; 12.1 mmol) as a yellow solid. ¹H NMR (DMSO): δ 4.47 (d, 2H, J = 5.2 Hz), 5.82 (t, 1H, J = 5.2 Hz), 7.70 (d, 1H, J = 8.8 Hz), 8.29 (dd, 1H, J = 8.8 and 2.0 Hz), 8.54 (d, 1H, J = 2.0 Hz)

### Step 3: (4-amino-2-iodophenyl)methanol

To a solution of (2-iodo-4-nitrophenyl)methanol (3.70 g; 13.3 mmol) in EtOH (100 mL) and water (25 mL), were successively added iron (3.70 g; 66.3 mmol) and ammonium chloride (800 mg; 15.0 mmol). The reaction mixture was stirred for 3 hours at 80°C. After the completion of the reaction, the reaction mixture was filtered over Celite^{®}, washed with EtOH and concentrated to dryness. The resulting residue was taken up in EtOAc and washed with a saturated solution of sodium hydrogen carbonate. The organic layer was dried over sodium sulfate, filtered and concentrated to dryness to afford compound of the title (2.48 g; 9.95 mmol) as a yellow oil. ¹H NMR (DMSO): δ 4.28 (d, 2H, J = 5.2 Hz), 4.97 (t, 1H, J = 5.2 Hz), 5.16 (s, 2H), 6.57 (d, 1H, J = 8.4 Hz), 7.02-7.10 (m, 2H)

### Step 4: 4-({[tert-butyl(dimethyl)silyl]oxy)methyl)-3-iodoaniline

To a solution of (4-amino-2-iodophenyl)methanol (3.51 g; 13.4 mmol) in DCM (150 mL), was added imidazole (0.95 g; 14.0 mmol). The mixture was cooled down to 0°C then a solution of tert-butylchlorodimethylsilane (2.40 mL; 13.85 mmol) in DCM (150 mL) was added dropwise over 15 minutes. The ice bath was removed and the reaction mixture was stirred at r.t. for 16 hours. After the completion of the reaction, the reaction mixture was quenched with MeOH (20 mL) and concentrated to dryness. The resulting residue was purified by column chromatography on silica gel using cyclohexane / EtOAc (100/0 to 90/10) as eluent to afford compound of the title (3.64 g; 10.0 mmol) as a yellow oil. ¹H NMR (DMSO): δ 0.06 (s, 6H), 0.88 (s, 9H), 4.46 (s, 2H), 5.24 (s, 2H), 6.55 (d, 1H, J = 8.4 Hz), 7.03 (d, 1H, J = 8.4 Hz), 7.05 (s, 1H)

### Step 5: N-[4-({[tert-butyl(dimethyl)silyl]oxy}methyl)-3-iodophenyl]-N⁵-carbamoyl-N2-{[(9H-fluoren-9-yl)methoxy]carbonyl)-L-ornithinamide

To a solution of 4-({[tert-butyl(dimethyl)silyl]oxy}methyl)-3-iodoaniline (10.0 g; 27.5 mmol) in MeOH (70 mL) and DCM (140 mL) were successively added Fmoc-Cit-OH (12.0 g; 30.28 mmol) and EEDQ (8.17 g ; 33.0 mmol). The reaction mixture was stirred for 14 hours at r.t. After the completion of the reaction, the resulting residue was purified by column chromatography on silica gel using DCM / MeOH (100/0 to 88/12) as eluent to afford compound of the title (17.09 g; 22.0 mmol) as a white solid. ¹H NMR (DMSO): δ 0.09 (s, 6H), 0.91 (s, 9H), 1.38-1.48 (m, 2H), 1.59-1.68 (m, 2H), 2.93-3.05 (m, 2H), 4.06-4.15 (m, 1H), 4.20-4.29 (m, 3H), 4.56 (s, 2H), 5.41 (s, 2H), 5.98 (t, 1H, J = 5.5 Hz), 7.30-7.43 (m, 5H), 7.55 (dd, 1H, J = 8.8 and 2.1 Hz), 7.69 (d, 1H, J = 7.8 Hz), 7.74 (dd, 2H, J = 7.2 and 3.4 Hz), 7.89 (d, 2H, J = 7.5 Hz), 8.25 (d, 1H, J = 1.5 Hz), 10.12 (s, 1H)

### Step 6: N-[4-({[tert-butyl(dimethyl)silyl]oxy}methyl)-3-iodophenyl]-N⁵-carbamoyl-L-ornithinamide

To a solution of *N*-[4-({[*tert*-butyl(dimethyl)silyl]oxy}methyl)-3-iodophenyl]-*N*⁵-carbamoyl-*N*²-{[(9*H*-fluoren-9-yl)methoxy]carbonyl}-L-ornithinamide (17.1 g; 23.0 mmol) in THF (120 mL), was added dimethylamine 2M in THF (44.5 mL; 89.0 mmol). The reaction mixture was stirred for 15 hours at r.t. After concentration to dryness, the resulting residue was purified by column chromatography on Cis using water / ACN (98/02 to 0/100) as eluent to afford compound of the title (5.47 g; 10.5 mmol) as a white solid. ¹H NMR (DMSO): δ 0.0 (s, 6H), 0.81 (s, 9H), 1.27-1.38 (m, 3H), 1.47-1.53 (m, 1H), 2.83-2.89 (m, 2H), 3.16-3.19 (m, 1H), 4.46 (s, 2H), 5.26 (s, 2H), 5.82 (t, 1H, J = 5.6 Hz), 7.24 (d, 1H, J = 8.5 Hz), 7.50 (dd, 1H, J = 8.3 and 2.0 Hz), 8.17 (d, 1H, J = 2.0 Hz)

### Step 7: N-{[(9H-fluoren-9-yl)methoxy]carbonyl}-L-valyl-N-[4-({[tert-butyl(dimethyl)silyl]oxy}methyl)-3-iodophenyl]-N⁵-carbamoyl-L-ornithinamide

To a solution of *N*-[4-({[*tert*-butyl(dimethyl)silyl]oxy}methyl)-3-iodophenyl]-*N*⁵-carbamoyl-L-ornithinamide (3.00 g; 5.76 mmol) in 2-methyltetrahydrofuran (240 mL), were successively added Fmoc-Val-OSu (8.65 g; 8.65 mmol) and DIPEA (1.90 mL; 11.5 mmol). The reaction mixture was stirred for 15 hours at r.t.. The reaction mixture was filtered through a sintered funnel and the recovered solid was washed with 2-methyltetrahydrofuran, then dried under high vacuum to afford compound of the title (3.57 g; 4.24 mmol) as a white solid. ¹H NMR (DMSO): δ 0.10 (s, 6H), 0.83-0.95 (m, 15H), 1.27-1.52 (m, 2H), 1.52-1.75 (m, 2H), 1.93-2.07 (m, 1H), 2.88-3.09 (m, 2H), 3.93 (t, 1H, J = 8.0 Hz), 4.17-4.49 (m, 4H), 4.56 (s, 2H), 5.40 (s, 2H), 5.96 (t, 1H, J = 5.6 Hz), 7.27-7.37 (m, 3H), 7.37-7.48 (m, 3H), 7.54 (d, 1H, J = 8.0 Hz), 7.74 (t, 2H, J = 7.2 Hz), 7.88 (d, 2H, J = 7.6 Hz), 8.13 (d, 1H, J = 7.6 Hz), 8.22 (s, 1H), 10.11 (s, 1H)

### Step 8: N-{[(9H-fluoren-9-yl)methoxy]carbonyl}-L-valyl-N-[4-({[tert-butyl(dimethyl)silyl] oxy}melhyl)-3-(3-hydroxyprop-1-yn-1-yl)phenyl]-N⁵-carbamoyl-L-ornilhinamide

To a solution of *N*-{[(9*H*-fluoren-9-yl)methoxy]carbonyl}-L-valyl-*N*-[4-({[*tert-*butyl(dimethyl)silyl]oxy}methyl)-3-iodophenyl]-*N*⁵-carbamoyl-L-ornithinamide (4.0 g; 4.55 mmol) and 2-propyn-1-ol (0.54 mL; 9.10 mmol) in dry DMF (36 mL), was added DIPEA (2.26 mL; 13.66 mmol; 3.0 eq.). The reaction mixture was purged with Argon, then bis(triphenylphosphine)palladium(II) dichloride (0.64 g; 0.91 mmol) and copper(I) iodide (0.17 g; 0.91 mmol) were added. The reaction mixture was purged again with Argon, and stirred at r.t. for 16 hours. A saturated solution of ammonium chloride (80 mL) was added and the aqueous layer was extracted with EtOAc. The combined organic layers were washed with brine, dried over sodium sulfate, filtered and concentrated to dryness. The crude was purified by flash chromatography over silica gel using DCM / MeOH (10/0 to 85/15) as eluent to afford the product of the title (2.7 g; 3.52 mmol) as a brown solid. ¹H NMR (DMSO-d₆): 0.08 (s, 6H), 0.85-0.89 (m, 6H), 0.90 (s, 9H), 1.32-1.48 (m, 2H), 1.54-1.74 (m, 2H), 1.94-2.03 (m, 1H), 2.89-3.06 (m, 2H), 3.93 (dd, 1H, J = 7.0 and 9.1 Hz), 4.22-4.33 (m, 5H), 4.36-4.41 (m, 1H), 4.74 (s, 2H), 5.35 (t, 1H, J = 5.9 Hz), 5.40 (s, 2H), 5.96 (t, 1H, J = 6.0 Hz), 7.32 (t, 2H, J = 7.4 Hz), 7.36-7.43 (m, 4H), 7.48 (dd, 1H, J = 2.3 and 8.8 Hz), 7.74 (t, 2H, J = 7.7 Hz), 7.78 (s, 1H), 7.89 (d, 2H, J = 7.8 Hz), 8.14 (d, 1H, J = 7.1 Hz), 10.09 (s, 1H)

### Step 9: N-{[(9H-fluoren-9-yl)methoxy]carbonyl}-L-valyl-N-[4-({[tert-butyl(dimethyl)silyl] oxy}methylj-3-{3-[(methanesulfonyl)oxy]prop-1-yn-1-yl}phenyl]-N⁵-carbamoyl-L-ornithinamide

To a solution of *N-*{[(9*H*-fluoren-9-yl)methoxy]carbonyl}-L-valyl-*N-*[4-({[*tert-*butyl(dimethyl)silyl]oxy}methyl)-3-(3-hydroxyprop-1-yn-1-yl)phenyl]-*N*⁵-carbamoyl-L-ornithinamide (1.0 g; 1.3 mmol) in dry DMF (9 mL), were successively added at 0°C DIPEA (0.64 mL; 3.9 mmol) and methanesulfonyl chloride (0.15 mL; 1.95 mmol). The reaction mixture was allowed to warm slowly to r.t. and stirred for 1 hour. A saturated solution of ammonium chloride (25 mL) was added, and the aqueous layer was extracted with EtOAc. The combined organic layers were washed with brine, dried over sodium sulfate, filtered and concentrated to dryness to afford the product of the title (1.1 g; 1.3 mmol) as an orange gum used as such for the next step. ¹H NMR (DMSO-d₆): 0.08 (s, 6H), 0.84-0.89 (m, 6H), 0.90 (s, 9H), 1.32-1.49 (m, 2H), 1.54-1.74 (m, 2H), 1.94-2.03 (m, 1H), 2.91-3.05 (m, 2H), 3.29 (s, 3H), 3.93 (dd, 1H, J = 7.1 and 9.0 Hz), 4.20-4.33 (m, 3H), 4.37-4.42 (m, 1H), 4.75 (s, 2H), 5.23 (s, 2H), 5.4 (s, 2H), 5.96 (t, 1H, J = 5.8 Hz), 7.32 (t, 2H, J = 7.2 Hz), 7.39-7.43 (m, 4H), 7.56 (dd, 1H, J = 2.3 and 8.8 Hz), 7.74 (t, 2H, J = 7.4 Hz), 7.83 (d, 1H, J = 2.3 Hz), 7.89 (d, 2H, J = 7.3 Hz), 8.14 (d, 1H, J = 7.3 Hz), 10.15 (s, 1H)

### Step 10: N-{[(9H-fluoren-9-yl)methoxy]carbonyl)-L-valyl-N-{3-[3-(acetylsuffanyl)prop-1-yn-1-yl]-4-({[tert-butyl(dimethyl)silyl]oxy}methyl)phenyl}-N⁵-carbamoyl-L-ornithinamide

To a solution of *N-*{[(9*H*-fluoren-9-yl)methoxy]carbonyl}-L-valyl-*N-*[4-({[*tert-*butyl(dimethyl)silyl]oxy}methyl)-3-{3-[(methanesulfonyl)oxy]prop-1-yn-1-yl}phenyl]-*N*⁵-carbamoyl-L-ornithinamide (1.1 g; 1.3 mmol) in dry DMF (10 mL), was added potassium thioacetate (0.3 g; 2.6 mmol). The reaction mixture was stirred at r.t. for 16 hours. A saturated solution of sodium bicarbonate (35 mL) was added, and the aqueous layer was extracted with EtOAc. The combined organic layers were washed with brine, dried over sodium sulfate, filtered, and concentrated to dryness. The crude was purified by flash chromatography over silica gel using DCM / MeOH (10/0 to 9/1) as eluent to afford the product of the title (0.52 g; 0.62 mmol) as a yellowish solid. ¹H NMR (DMSO-d₆): 0.08 (s, 6H), 0.84-0.89 (m, 6H), 0.90 (s, 9H), 1.31-1.49 (m, 2H), 1.54-1.73 (m, 2H), 1.95-2.02 (m, 1H), 2.4 (s, 3H), 2.89-3.06 (m, 2H), 3.93 (dd, 1H, J = 6.9 and 9.1 Hz), 3.97 (s, 2H), 4.20-4.33 (m, 3H), 4.36-4.41 (m, 1H), 4.69 (s, 2H), 5.39 (s, 2H), 5.96 (t, 1H, J = 5.8 Hz), 7.32 (t, 2H, J = 7.2 Hz), 7.35-7.43 (m, 4H), 7.49 (dd, 1H, J = 2.2 and 8.5 Hz), 7.72-7.76 (m, 3H), 7.89 (d, 2H, J = 7.5 Hz), 8.12 (d, 1H, J = 7.4 Hz), 10.08 (s, 1H)

### Step 11: N-{[(9H-fluoren-9-yl)methoxy]carbonyl}-L-valyl-N⁵-carbamoyl-N-[4-(hydroxymethyl)-3-(3-sulfoprop-1-yn-1-yl)phenyl]-L-ornithinamide

To a 35% solution of hydrogen peroxide (1.08 mL; 12.57 mmol), was added formic acid (9.68 mL; 256.5 mmol) at 0°C. This solution was stirred for 1 hour, then added to *N-{[(9H-*fluoren-9-yl)methoxy]carbonyl}-L-valyl-*N*-{3-[3-(acetylsulfanyl)prop-1-yn-1-yl]-4-({[*tert*-butyl(dimethyl)silyl]oxy}methyl)phenyl}-*N*⁵-carbamoyl-L-ornithinamide (367 mg; 0.44 mmol) at r.t. The reaction mixture was stirred for 2 hours, diluted with water (20 mL) and concentrated to dryness at 35°C. The residue was suspended in hydrochloric acid 1N (25 mL) and washed with DCM / MeOH (7/3). The aqueous layer was concentrated to dryness at 35°C. The crude was purified by flash chromatography over Cis using ACN / water + TFA (0.1%) (2/98 to 50/50) as eluent to afford after freeze-drying product of the title (117 mg; 0.13 mmol) as a white solid. ¹H NMR (DMSO-d₆): δ 0.84-0.88 (m, 6H), 1.33-1.50 (m, 2H), 1.55-1.75 (m, 2H), 1.95-2.02 (m, 1H), 2.89-3.05 (m, 2H), 3.58 (s, 2H), 3.91-3.95 (m, 1H), 4.20-4.33 (m, 3H), 4.37-4.42 (m, 1H), 4.55 (s, 2H), 5.22 (brs, 1H), 5.38 (s, 2H), 5.96 (t, 1H, J = 5.6 Hz), 7.32 (t, 2H, J = 7.2 Hz), 7.36-7.43 (m, 4H), 7.48 (dd, 1H, J = 2.4 and 9.2 Hz), 7.65 (d, 1H, J = 1.6 Hz), 7.74 (t, 2H, J = 6.8 Hz), 7.89 (d, 2H, J = 7.4 Hz), 8.12 (d, 1H, J = 7.6 Hz), 10.03 (s, 1H). LCMS (2-100 ACN/H₂O + 0.05% TFA): 77.27%, Rt = 8.3 min. Positive mode 720.26 detected (M+H⁺)

### Step 12: Example 34

To a suspension of *N*-{[(9*H*-fluoren-9-yl)methoxy]carbonyl}-L-valyl-*N*⁵-carbamoyl-*N*-[4-(hydroxymethyl)-3-(3-sulfoprop-1-yn-1-yl)phenyl]-L-ornithinamide (10 mg; 0.014 mmol) in THF (600 µL) and acetic acid (60 µL), was added under argon Pt/C dry 5% (7 mg; 0.0018 mmol). The reaction mixture was purged with hydrogen three times, then stirred at r.t. for 64 hours. The reaction mixture was filtered over a 45 µm PTFE filter, washed with MeOH and MeOH / water (1/1), then concentrated to dryness to afford **Example 34** (7 mg) as an orange oil. LCMS (2-100 ACN/H₂O + 0.05% FA): 6.93%, Rt = 8.8 min. Negative mode 722.19 detected (M-H)

As performed for Examples 6 to 12, Examples 14 to 25, Example 27 or Example 28, **Example 34,** a para-amino-benzyl linker compound of Formula (I), can be used for the preparation of Linker-Drug compound of Formula (II) and for the preparation of an antibody-drug conjugate of Formula (III) according to the invention.

### EXAMPLE A: Conjugation and analytical characterization of ADCs

As used throughout this application, antibody drug conjugates can be identified using a naming convention in the general format of "target antigen/antibody-linker-drug". For example only, if an antibody-drug conjugate is referred to as "Target X-Example Y", such a conjugate would comprise an antibody that binds Target X, and a linker-drug exemplified in Example Y.

### 1. Conjugation

Exemplary ADCs were synthesized using one of the methods described below. Antibodies used in the exemplary ADC synthesis were defined by the abbreviations Ab T TG and Ab T (Table 1).

**Table 1. Antibodies used for the purpose of the invention**

| **Antibody abbreviation** | **Antibody** | **Mutation** | **Sequence (VL/VH)** | **Patent** | **Allotype** |
|---|---|---|---|---|---|
| Ab T TG | Trastuzumab | N297S | SEQ 1 / 2 | US6870034B2 | G1m17 |
| Ab T | Trastuzumab | WT | SEQ 1 / 2 | US6870034B2 | G1m17,1 |

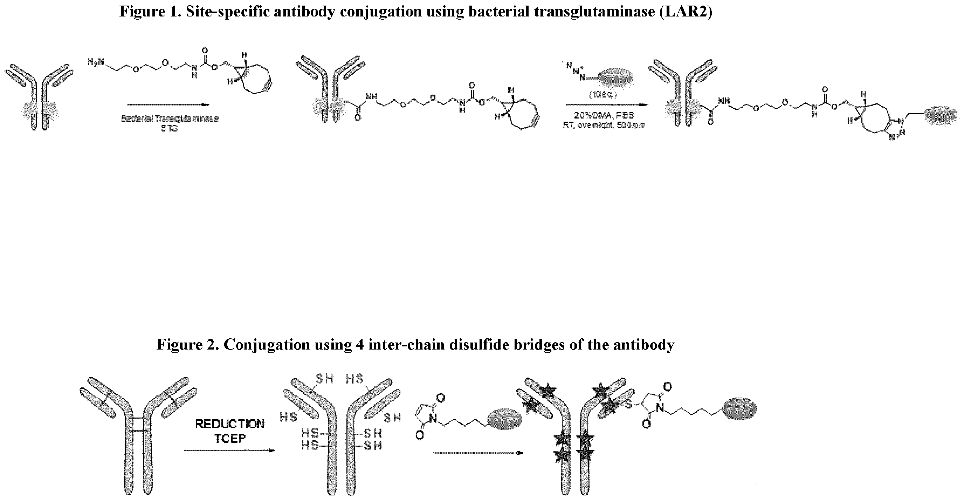

Antibody T TG was endowed with a bacterial transglutaminase (BTG) - reactive glutamines that were specifically functionalized with amine containing cyclooctyne BCN (or *N-*[(1*R*,8*S*,*9s*)-Bicyclo[6.1.0]non-4-yn-9-ylmethyloxycarbonyl]-1,8-diamino-3,6-dioxaoctane). The site specific antibody conjugation to BCN moiety using bacterial transglutaminase was performed as described in Innate Pharma 2013 (presentation at ADC Summit, San Francisco, California, Oct. 15, 2013), WO 2017/059160A1 and WO 2016/144608A1. These modifications allowed conjugating the described azide containing precursors using the following method A (Figure 1).

The conjugates of Ab T were obtained by full reduction of the four inter-chain disulfide bonds followed by conjugation with an excess amount of drug (Figure 2). For the purpose of the invention a water-soluble tertiary phosphine such as Tris-(2-carboxyethyl) phosphine (TCEP) was used as a reducing agent. Full reduction and conjugation produce primarily the fully loaded species with 8 drugs per antibody and eventually a small proportion of 6 drug loaded mAb explaining average DAR value of 7.1 and 7.7 (Table 2).

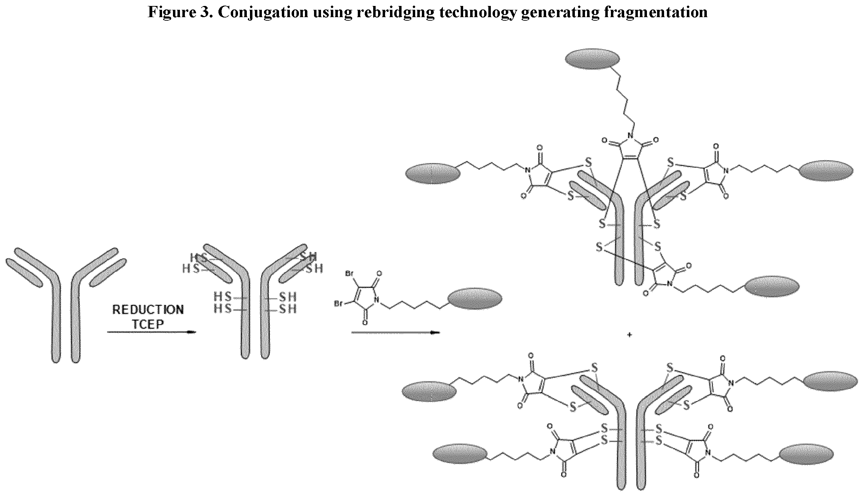

Some conjugates of Ab T were produced by rebridging technology described in Method D below. The four inter-chain disulfide bonds of the Ab T were fully reduced with TCEP and reformed using dibromo maleimide functionalized linker-payload (Figure 3). The conjugation results in two conformations due to the proximity of the cysteine implicated in interchain disulfide bridges on each heavy chain of the antibody.

### Method A (DAR2)

Dimethyl sulfoxide (DMSO, 619 µl, 20% of the coupling volume) was added to the Ab solution (1.8 mg/mL; 2.5 mL, 4.5 mg). The mixture was stirred for 30 seconds in vortex followed by the addition of 4-fold molar excess of linker-warhead payload (20 mM, 6 µL in DMSO). The reaction was stirred at r.t. overnight at 64 rpm. To remove the unconjugated linker-payload, it was added 10-fold molar excess of DBCO-containing Tentagel resin (0.1-0.2 mmol/g, Iris Biotech, CS-0477.0500) and the mixture was stirred for 6 hours at r.t. The buffer was exchanged with PBS 1X (Sigma Life Science, P3813, 10PAK) by 3 filtration cycles using Vivaspin 20, 50KD, PES (Sartorius Stedim, VS2031), then filtered sterilely through 0.2 µm sterile PES Filter, 25 mm (Whatmann, G896-2502) and stored at 4°C.

### Method B (DAR8)

The Ab solution (10.3 mg/mL; 0.5mL) was diluted in EDTA containing PBS buffer pH 7.4 (10 mM; 0.5 mL) to fix a final EDTA concentration in the coupling reaction of 5 mM. Then TCEP (1 mM in PBS buffer pH 7.4, 849 µL) was added to the antibody followed by incubation at 37°C for 2 hours. After reduction, the antibody solution was cooled down to 2-8°C and 20-fold molar excess of the linker-payload (5 mM, 141 µL in DMSO) was added. The reaction was incubated at 4°C for 1.5 hours. The solution was centrifuged (14000G at 4°C) for 20 minutes and it was loaded on HiLoad 26/600 Superdex 200 pg (GE Healthcare, 28989336) SEC chromatography column. The ADC was eluted with 20% DMA in PBS (Sigma Life Science, P3813, 10PAK) followed by 2 cycle's dialysis (16 and 4 hours) in PBS 1X pH 7.4 (Sigma Life Science, P3813, 10PAK). The conjugate was concentrated using Vivaspin 20, 50KD, PES (Sartorius Stedim, VS2031), filtered sterilely through 0.2 µm sterile PES Filter, 25 mm (Whatmann, G896-2502) and stored at 4°C.

### Method C (DAR8)

The Ab solution (10.3 mg/mL; 0.5 mL) was diluted in EDTA containing PBS buffer pH 7.4 (10 mM; 0.5 mL) to fix a final EDTA concentration in the coupling reaction of 5 mM. Then TCEP (1 mM in PBS buffer pH 7.4; 849 µL) was added to the antibody followed by incubation at 37°C for 2 hours. After reduction, the antibody solution is cooled down to 2-8°C and 20-fold molar excess of the linker-payload (5 mM; 141 µL in DMSO) was added. The reaction was incubated at 4°C for 1.5 hours. Then the conjugate was purified on rmp protein A resin (GE Healthcare, 17-5138-01) followed by 2 cycle's dialysis (16 and 4 hours) in PBS 1X pH 7.4 (Sigma Life Science, P3813, 10PAK). The ADC was concentrated using Vivaspin 20, 50KD, PES (Sartorius Stedim, VS2031), filtered sterilely through 0.2 µm sterile PES Filter, 25 mm (Whatmann, G896-2502) and stored at 4°C.

### Method D (DAR4)

To Ab solution (5 mg/ml, 0.5 ml) in BBS buffer pH 8 (prepared as described below), was added 8-fold molar excess of TCEP (1 mM in BBS pH 8, 137 µl) and the reaction was incubated at 37°C for 2 hours. Then, 7.5-fold molar excess of the linker-payload (1 mM, 129 µL in DMF) was added and the resulting solution was mixed at r.t. for 1 hour at 600 rpm. The resulting ADC was dialyzed in PBS 1X pH 7.4 (Sigma Life Science, P3813, 10PAK) for 16 hours at 4°C. Then the conjugate was purified on rmp protein A resin (GE Healthcare, 17-5138-01) followed by 2 hours dialysis at r.t. in PBS 1X pH 7.4 (Sigma Life Science, P3813, 10PAK). The ADC was concentrated using Vivaspin 20, 50KD, PES (Sartorius Stedim, VS2031), filtered sterilely through 0.2 µm sterile PES Filter, 25mm (Whatmann, G896-2502) and stored at 4°C.

*Preparation of BBS buffer pH 8*: Na₂B₄O₇. 10 H₂O (528 mg) was dissolved in deionized water (27.7 mL). Sodium chloride (63 mg, 25 mM) and EDTA (16 mg, 1 mM) were solubilized in 22.3 mL of hydrochloric acid 0.1 M. Then, the two solutions were mixed together, and the resulting solution is directly used for the conjugation step.

### 2. LC-MS General Methodology

Drug-to-antibody ratio (DAR) of exemplary ADCs was determined by liquid chromatography hyphenated with mass spectrometry (LC-MS) with the following method LC-I or LC-II. For the LC-I method, mobile phase A was purified by MS grade water (Biosolve, Dieuze, France, 00232141B1BS), mobile phase B by MS grade acetonitrile (Biosolve, Dieuze, France, 0001204101BS) and mobile phase D purified by MS grade water supplemented with 1% of formic acid (FA) (Honeywell/Fluka, Bucharest, Romania, 56302). Mobile phase D was fixed at 10% in order to maintain a 0.1% FA mobile phase composition and column temperature was set at 80°C. A general MS method was optimized for all ADCs synthesized (Table 2). For the LC-II method, mobile phase A was purified by MS grade water (Biosolve, Dieuze, France, 00232141B1BS) and mobile phase B by MS grade acetonitrile (Biosolve, Dieuze, France, 0001204101BS), both containing 0.1% formic acid (FA) (Honeywell/Fluka, Bucharest, Romania, 56302). The column temperature was set at 80°C. A general MS method was optimized for all ADCs synthesized (Table 2).

Method LC-I: ADC was loaded onto a Bioresolve RP mAb Polyphenyl, 450A, 2.7 µm, 2.1x150 mm (Waters, Saint-Quentin-en-Yvelines, France, 186008946). For analysis in both intact and reduced conditions, a desalting step was performed for 1.5 minutes at 20% of B with a flow rate of 0.6 mL/min. Elution step was performed with a gradient from 1.5 min at 20% B to 16.5 minutes at 70% B with a flow rate of 0.3 mL/min. A wash step was set from 16.8 minutes to 18.8 minutes at 90% B with a flow rate of 0.6 mL/min. Finally, a conditioning step was used at 19.1 minutes for 1.9 minutes at 20% B with a flow rate of 0.6 mL/min (Total run time = 21 minutes).

Method LC-II: ADC was loaded onto a Bioresolve RP mAb Polyphenyl, 450A, 2.7 µm, 2.1x150mm (Waters, Saint- Quentin-en-Yvelines, France, 186008946). For analysis in both intact and reduced conditions, a desalting step was performed for 1.5 minutes at 20% of B with a flow rate of 0.6ml/min. Then the elution step was carried out at the same flow rate with a gradient from 1.5 minutes at 20% B to 16.5 min at 50% B. The following washing step was set from 16.8 minutes to 18.8 minutes at 100% B with flow 0.6 mL/min. Finally, a conditioning step was used from 18.8 minutes to 19.2 minutes from 100% to 20% B stabilizing the column with additional 1.9 minutes at 20% B with a flow rate mentioned above (Total run time=21min).

LC-MS analysis was performed using a Waters UPLC H-Class Bio chromatography system hyphenated with a Xevo G2 XS Q-TOF ESI mass spectrometer (Waters, Manchester, UK). The ADC was either analyzed in intact condition (no preliminary treatment) or following reduction with 5 mM (final concentration) of dithiothreitol DTT (Thermo Scientific, Rockford, IL, 20291). Subsequently, treated ADC was analyzed using the aforementioned LC-I and LC-II (Table 2). Electrospray-ionization time-of-flight mass spectra of the analytes were acquired using MassLynx^{™} acquisition software (Waters, Manchester, UK). Then, the extracted intensity vs. m/z spectrum was deconvoluted using Maximum Entropy (MaxEnt) method of MassLynx^{™} software in order to determine the mass of each intact antibody species or each reduced antibody fragment depending on the treatment used. Finally, DAR was determined from the deconvoluted spectra or UV chromatogram by summing the integrated MS (total ion current) or UV (280 nm) peak area of unconjugated and conjugated given species (mAb or associated fragment). For the DAR determination by UV chromatogram, relative area percentage of every specie was multiplied by the number of drugs attached. The summed, weighted areas of every specie were divided by the sum of total relative area percentage and the results produced an estimation of the final average DAR value for the full ADC. For the DAR determination by deconvoluted spectra, the percentage of every specie identified was calculated by intensity peak value from deconvoluted spectra. The percentage obtained, was multiplied by the number of drugs attached. The summed results produced an estimation of the final average DAR value for the full ADC.

### 3. Size Exclusion Chromatography

Size exclusion chromatography (SEC) was performed to determine the quality of the ADCs, its aggregation percentage after purification. The analysis was performed on analytical column Superdex 200 Increase 5/150 GL (GE Healthcare, 28990945) in isocratic conditions 100% PBS pH 7.4 (Sigma Life Science, P3813, 10PAK), flow 0.45 mL/min for 12 minutes. The percentage of aggregate fraction in the conjugate sample was quantified based on the peak area absorbance at 280 nm. Its calculation was based on the ratio between the high molecular weight eluent at 280 nm divided by the sum of peak area absorbance at the same wavelength of the high molecular weight and monomeric eluents multiplied by 100.

### 4. Hydrophobic Interaction Chromatography

Hydrophobic Interaction Chromatography (HIC) was carried out to determine the hydrophobicity impact of the linker-payload and the bioconjugation technique on the antibody. The analysis was performed using TSKgel Butyl-NPR column (Tosoh Bioscience, 0014947) with mobile phase A (1.5 M ammonium sulfate (NH₄)₂SO₄, 25 mM potassium phosphate dibasic (K₂HPO₄), adjusted at pH 7) and B (25 mM potassium phosphate dibasic (K₂HPO₄), 20% isopropanol, adjusted at pH 7). The elution was started with flow of 0.6 mL/min at 5% B. The gradient raised from 5% to 100% B in 17 minutes, followed by washing step at 100% B for 5 minutes. Finally, a conditioning step was carried out 100%-5% B for 2 minutes followed by 2 minutes at 5% B.

For the purpose of the invention, a relative retention time (RRT) of each ADC was calculated using the ADC retention time (RT) divided by the antibody RT (Table 3).

### 5. Results

Characterization of the exemplary ADCs was summarized in Table 2 (named as **Examples 15 to 22** and **Examples 29 to 33;** coupling and LC-MS method, aggregation status and DAR). The average DAR values were determined using the above LC-MS method LC-I or LC-II (point 2) and the percentage of aggregates was measured by size exclusion chromatography (SEC) described above (point 3).

**Table 2. ADC Analytical characterization and coupling methodology**

| **Ex.** | **ADC** | **Coupling Method** | **LC-MS Method** | **DAR (by MS)** | **% Agg (by SEC)** |
|---|---|---|---|---|---|
| **15** | Ab T TG-Example 8 | A | LC-I | 1.9 | 1.2 |
| **16** | Ab T-Example 6 | B | LC-I | 8 | 0.2 |
| **17** | Ab T-Example 7 | C | LC-I | 8 | 0.2 |
| **18** | Ab T-Example 10 | C | LC-I | 7.3 | 0.2 |
| **19** | Ab T-Example 9 | C | LC-I | 7.3 | 0.2 |
| **20** | Ab T-Example 11 | C | LC-I | 8 | 0.2 |
| **21** | Ab T-Example 12 | C | LC-II | 7.5 | 0.2 |
| **22** | Ab T-Example 14 | C | LC-II | 7.7 | 0.2 |
| **29** | Ab T-Example 23 | C | LC-II | 7.5 | 0.2 |
| **30** | Ab T-Example 27 | C | LC-II | 7.1 | 1.4 |
| **31** | Ab T-Example 24 | C | LC-II | 7.6 | 2.5 |
| **32** | Ab T-Example 25 | C | LC-II | 7.2 | 2.7 |
| **33** | Ab T-Example 28 | D | LC-II | 4 | 1.7 |

The hydrophobicity of the exemplified ADC was compared by the calculation of the relative retention time using HIC chromatography described above (point 4). Table 3 summarized RRT values of the conjugates.

**Table 3. Comparison of ADC hydrophobicity using the RRT by HIC chromatography**

| **Ex.** | **ADC** | **RRT** | **RT ADC (HIC)** | **RT mAb (HIC)** |
|---|---|---|---|---|
| **15** | Ab T TG-Example 8 | 0.97 | 5.84 | 6.01 |
| **16** | Ab T-Example 6 | 2.02 | 11.21 | 5.54 |
| **17** | Ab T-Example 7 | 2.31 | 12.06 | 5.22 |
| **18** | Ab T-Example 10 | 2.18 | 11.47 | 5.25 |
| **19** | Ab T-Example 9 | 2.15 | 11.31 | 5.25 |
| **20** | Ab T-Example 11 | 2.20 | 11.74 | 5.33 |
| **21** | Ab T-Example 12 | 2.65 | 13.01 | 4.91 |
| **22** | Ab T-Example 14 | 2.33 | 10.03 | 4.75 |
| **29** | Ab T-Example 23 | 2.83 | 14.02 | 4.96 |
| **30** | Ab T-Example 27 | 2.56 | 14.16 | 5.54 |
| **31** | Ab T-Example 24 | 1.45 | 8.05 | 5.54 |
| **32** | Ab T-Example 25 | 1.28 | 6.83 | 5.32 |
| **33** | Ab T-Example 28 | 1.85 | 8.58 | 4.63 |

Stability study was performed for all exemplified ADCs, where the conjugates were incubated in conditions of accelerated degradation in PBS buffer at +37°C over 1 week. SEC chromatography was exploited to measure the aggregation status of the conjugates. The obtained results were illustrated in Table 4.

**Table 4. Stability studies of the ADCs at conditions of accelerated degradation in PBS buffer**

| **Ex.** | **ADC** | **% Agg (by SEC) T₀** | **% Agg (by SEC) 1 week 37°C** |
|---|---|---|---|
| **15** | Ab T TG-Example 8 | 1.2 | 1.2 |
| **16** | Ab T-Example 6 | 0.2 | 0.2 |
| **17** | Ab T-Example 7 | 0.2 | 0.2 |
| **18** | Ab T-Example 10 | 0.2 | 5.2 |
| **19** | Ab T-Example 9 | 0.2 | 4.3 |
| **20** | Ab T-Example 11 | 0.2 | 1.8 |
| **21** | Ab T-Example 12 | 0.2 | 2.7 |
| **22** | Ab T-Example 14 | 0.2 | 1.7 |
| **29** | Ab T-Example 23 | 0.2 | 1.1 |
| **30** | Ab T-Example 27 | 1.4 | 8.3 |
| **31** | Ab T-Example 24 | 2.5 | 11.6 |
| **32** | Ab T-Example 25 | 2.7 | 21.6 |
| **33** | Ab T-Example 28 | 1.7 | 1.9 |

### EXAMPLE B: Effect of exemplified ADCs on cell viability using CTG assay

HCC1954 (HER+) and MOLT4 (HER-) cell lines were cultivated in RPMI supplemented with 10% heat inactivated fetal bovine serum, penicillin (100 IU/ml), streptomycin (100 µg/ml) and L-glutamine (2 mM). Cell lines were cultured at 37°C in a humidified atmosphere containing 5% CO₂. Cells were seeded in 96 microwell plates and exposed to the ADCs for 120 hours (5 fold serially diluted; 9 concentrations each, triplicates). Effects of ADCs on cell viability were assessed after 5 days of incubation at 37°C/5% CO₂ by quantification of cellular ATP levels using CellTiterGlo at 75 µL reagent/well. All the conditions were tested in triplicates. Luminescence was quantified on a multipurpose plate reader. IC₅₀s were calculated using standard four-parametric curve fitting. IC₅₀ is defined as the compound concentration at which the CTG signal is reduced to 50% of that measured for the control. Each experiment was performed at least twice, with results being reproducible.

As shown in Table 5, all exemplified ADC are active in antigen-positive cells in CTG assay.

**Table 5. Effect of exemplified ADCs on cell viability using CTG assay**

| **Ex.** | **HCC1954 (HER2+)** | | | **MOLT4 (HER2-)** | | |
|---|---|---|---|---|---|---|
| | IC₅₀ n1 (M) | IC₅₀ n2 (M) | **Mean (M)** | IC₅₀ n1 (M) | IC₅₀ n2 (M) | **Mean (M)** |
| **15** | 2.38E-10 | 3.46E-10 | **2.92E-10** | >3.00E-07 | >3.00E-07 | **>3.00E-07** |
| **16** | 1.22E-11 | 5.94E-11 | **3.58E-11** | 5.40E-08 | 6.27E-08 | **5.84E-08** |
| **17** | 2.53E-11 | 3.86E-11 | **3.20E-11** | 2.25E-07 | 1.47E-07 | **1.86E-07** |
| **18** | 4.24E-11 | 9.28E-11 | **6.76E-11** | 1.41E-07 | 2.83E-07 | **2.12E-07** |
| **19** | 4.55E-11 | 7.52E-11 | **6.04E-11** | 4.74E-08 | 2.21E-07 | **1.34E-07** |
| **20** | 4.53E-11 | 6.93E-11 | **5.73E-11** | 3.34E-08 | 6.21E-08 | **4.78E-08** |
| **21** | 7.73E-11 | 9.14E-11 | **8.44E-11** | 4.38E-08 | 9.78E-08 | **7.08E-08** |
| **22** | 2.25E-11 | 3.64E-11 | **2.94E-11** | 1.30E-08 | 1.20E-08 | **1.25E-08** |
| **29** | 4.80E-11 | 5.70E-11 | **5.25E-11** | 1.03E-07 | 9.02E-08 | **9.65E-08** |
| **30** | 1.37E-11 | 1.39E-11 | **1.38E-11** | 5.81E-08 | 4.78E-08 | **5.30E-08** |

## Claims

1. A para-amino-benzyl linker compound of Formula (I): 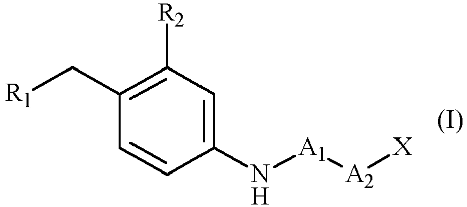 wherein:
• R₁ represents a hydroxy group or a halogen atom;
• R₂ represents a -S(O)₂(OH) group, a -S(O)₂(O⁻M⁺) group, a linear or branched -(C₁-C₄)alkyl-S(O)₂(OH) group, a linear or branched -(C₁-C₄)alkyl-S(O)₂(O⁻M⁺) group, a linear or branched -halo(C₁-C₄)alkyl-S(O)₂(OH) group, or a linear or branched -halo(C₁-C₄)alkyl-S(O)₂(O⁻M⁺) group;
• A₁ represents a -C(O)-CH(R₃)-NH- group;
• A₂ represents a -C(O)-CH(R₄)-NH- group, a 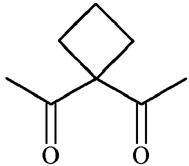 group, a 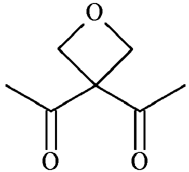 group, or a 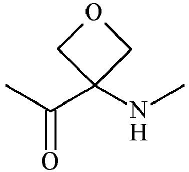 group;
• R₃ and R₄, independently of one another, represent the side chain of an amino acid;
• X represents a hydrogen atom, a hydroxy group, or a protecting group;
• M⁺ represents a pharmaceutically acceptable monovalent cation.

2. A para-amino-benzyl linker compound according to claim 1, wherein R₁ represents a hydroxy group, a bromine atom, a chlorine atom, or an iodine atom.

3. A para-amino-benzyl linker compound according to claim 1, wherein R₂ represents a -S(O)₂(OH) group, a -S(O)₂(O⁻M⁺) group, a -CH₂-S(O)₂(OH) group, a -CH₂-S(O)₂(O⁻M⁺) group, a -CH₂-CH₂-S(O)₂(OH) group, a -CH₂-CH₂-S(O)₂(O⁻M⁺) group, a -CH₂-CH₂-CH₂-S(O)₂(OH) group, or a -CH₂-CH₂-CH₂-S(O)₂(O⁻M⁺) group.

4. A para-amino-benzyl linker compound according to claim 1, wherein A₁ represents a -C(O)-CH(R₃)-NH- group in which R₃ represents a -(CH₂)₃-NH-CO-NH₂ group or a methyl group.

5. A para-amino-benzyl linker compound according to claim 1, wherein A₂ represents a -C(O)-CH(R₄)-NH- group in which R₄ represents an isopropyl group; a 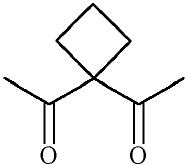 group; a 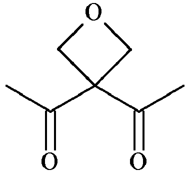 group; or a 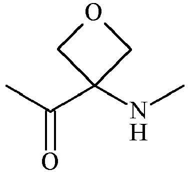 group.

6. A para-amino-benzyl linker compound according to claim 1, wherein A₁ represents a -C(O)-CH(R₃)-NH- group and A₂ represents a -C(O)-CH(R₄)-NH- group, in which R₃ and R₄, independently of one another, represent the side chain of an amino acid..

7. A para-amino-benzyl linker compound according to claim 1, which are:
- sodium 5-[[(2S)-2-[[(2S)-2-(9H-fluoren-9-ylmethoxycarbonylamino)-3-methyl-butanoyl]amino]-5-ureido-pentanoyl]amino]-2-(hydroxymethyl)benzenesulfonate;
- sodium 5-[[(2S)-2-[[(2S)-2-(9H-fluoren-9-ylmethoxycarbonylamino)-3-methyl-butanoyl]amino]propanoyl]amino]-2-(hydroxymethyl)benzenesulfonate;
- 5-[[(2S)-2-[[(2S)-2-(9H-fluoren-9-ylmethoxycarbonylamino)-3-methyl-butanoyl]amino]propanoyl]amino]-2-(hydroxymethyl)benzenesulfonic acid;
- sodium [5-[[(2S)-2-[[(2S)-2-(9H-fluoren-9-ylmethoxycarbonylamino)-3-methyl-butanoyl]amino]-5-ureido-pentanoyl]amino]-2-(hydroxymethyl)phenyl]methanesulfonate;
- 2-(chloromethyl)-5-[[(2S)-2-[[(2S)-2-(9H-fluoren-9-ylmethoxycarbonylamino)-3-methyl-butanoyl]amino]-5-ureido-pentanoyl]amino]benzenesulfonic acid;
- 2-(chloromethyl)-5-[[(2S)-2-[[(2S)-2-(9H-fluoren-9-ylmethoxycarbonylamino)-3-methyl-butanoyl]amino]propanoyl]amino]benzenesulfonic acid;
- 5-[[(2S)-2-[[(2S)-2-(9H-fluoren-9-ylmethoxycarbonylamino)-3-methyl-butanoyl]amino]-5-ureido-pentanoyl]amino]-2-(iodomethyl)benzenesulfonic acid;
- sodium *N*-{[(9*H*-fluoren-9-yl)methoxy]carbonyl}-L-valyl-*N*⁵-carbamoyl-*N*-[4-(hydroxymethyl)-3-(2-sulfonatoethyl)phenyl]-L-ornithinamide;
- *N-*{[(9*H*-fluoren-9-yl)methoxy]carbonyl}-L-valyl-*N*⁵-carbamoyl-*N-*[4-(hydroxymethyl)-3-(3-sulfopropyl)phenyl]-L-ornithinamide.

8. A para-amino-benzyl linker compound of Formula (I) according to any one of claims 1 to 7 for use in the preparation of an antibody-drug conjugate.

9. A Linker-Drug compound of Formula (II): 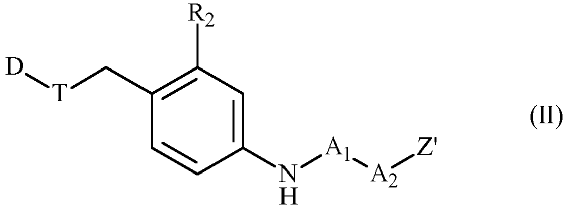 wherein:
• D represents a drug moiety;
• T is a bond, -O-C(O)-N(CH₃)-CH₂-CH₂-N(CH₃)-C(O)-*, -O-*, -NR₅-*, -NRs-C(O)-*, or -O-C(O)-*, wherein the * indicates the point of attachment to D;
• R₂ represents a -S(O)₂(OH) group, a -S(O)₂(O⁻M⁺) group, a linear or branched -(C₁-C₄)alkyl-S(O)₂(OH) group, a linear or branched -(C₁-C₄)alkyl-S(O)₂(O⁻M⁺) group, a linear or branched -halo(C₁-C₄)alkyl-S(O)₂(OH) group, or a linear or branched -halo(C₁-C₄)alkyl-S(O)₂(O⁻M⁺) group;
• A₁ represents a -C(O)-CH(R₃)-NH- group;
• A₂ represents a -C(O)-CH(R₄)-NH- group, a 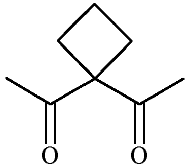 group, a 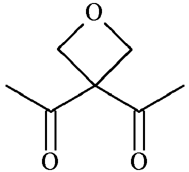 group, or a 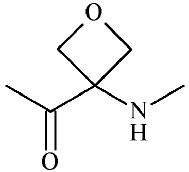 group;
• R₃ and R₄, independently of one another, represent the side chain of an amino acid;
• R₅ represents a hydrogen atom or a (C₁-C₄)alkyl group;
• Z' represents a Spacer unit precursor;
• M⁺ represents a pharmaceutically acceptable monovalent cation.

10. A Linker-Drug compound according to claim 9, wherein R₂ represents a -S(O)₂(OH) group, a -S(O)₂(O⁻M⁺) group, a -CH₂-S(O)₂(OH) group, a -CH₂-S(O)₂(O⁻M⁺) group, a -CH₂-CH₂-S(O)₂(OH) group, a -CH₂-CH₂-S(O)₂(O⁻M⁺) group, a -CH₂-CH₂-CH₂-S(O)₂(OH) group, or a -CH₂-CH₂-CH₂-S(O)₂(O⁻M⁺) group.

11. A Linker-Drug compound according to claim 9, wherein A₁ represents a -C(O)-CH(R₃)-NH- group in which R₃ represents a -(CH₂)₃-NH-CO-NH₂ group or a methyl group.

12. A Linker-Drug compound according to claim 9, wherein A₂ represents a -C(O)-CH(R₄)-NH- group in which R₄ represents an isopropyl group; a 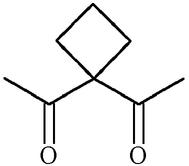 group; a 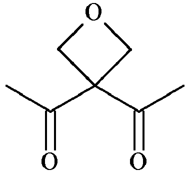 group; or a 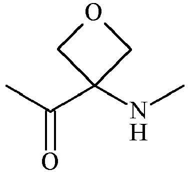 group.

13. A Linker-Drug compound according to claim 9, wherein T represents a bond or -O-C(O)-*, wherein the * indicates the point of attachment to D.

14. A Linker-Drug compound according to claim 9, wherein Z' represents a group selected from: 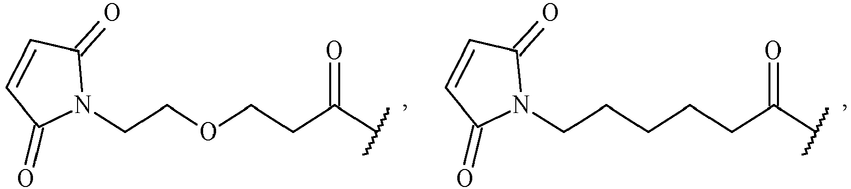 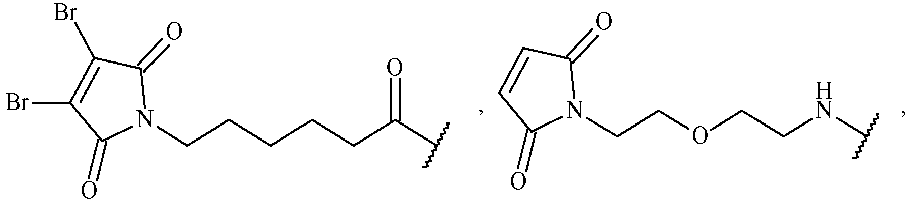 or 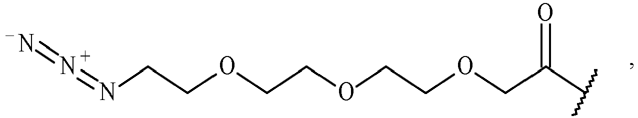 wherein the wavy line indicates the covalent attachment site to the N-terminus or the carbonyl group of A₂ group.

15. A Linker-Drug compound according to claim 9, which are: 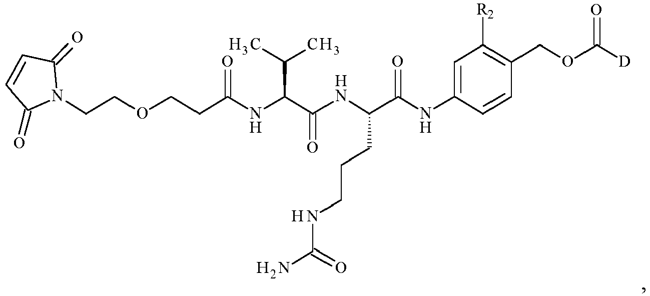 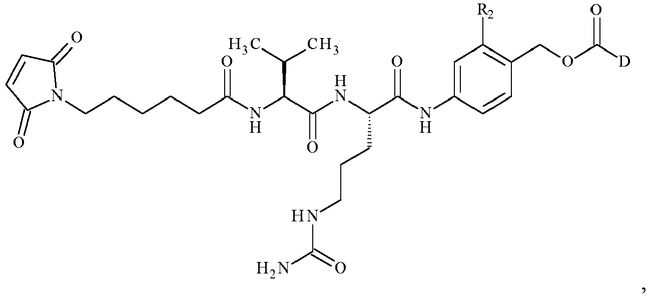 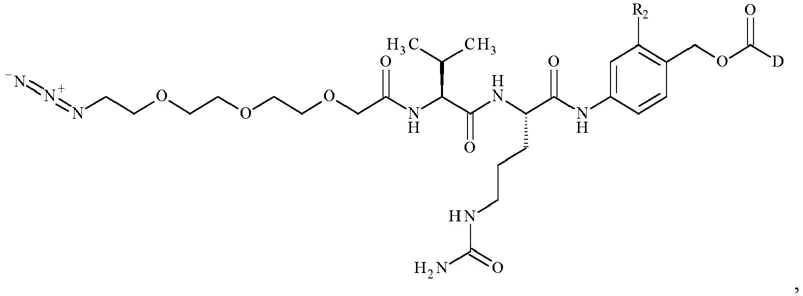 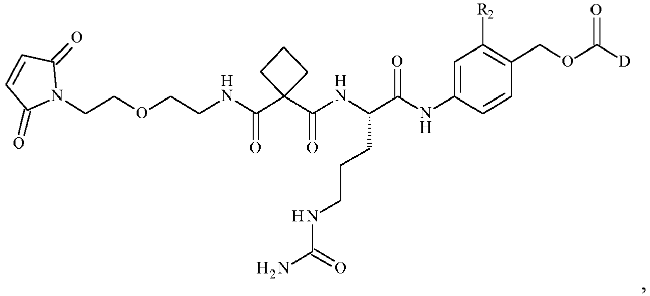 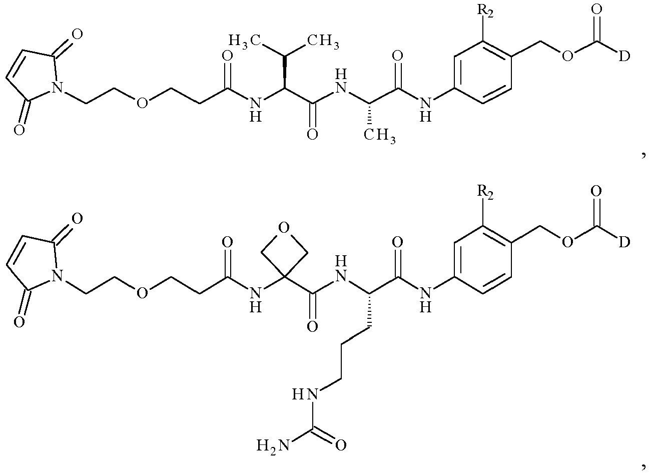 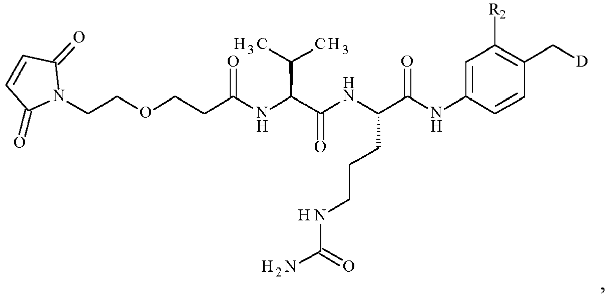 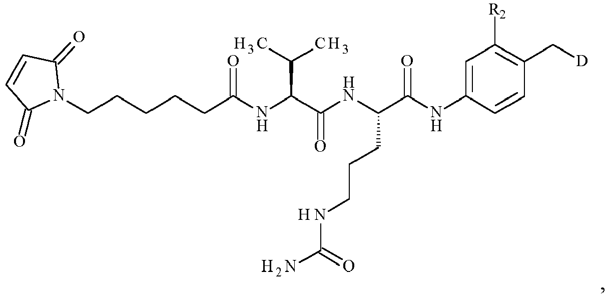 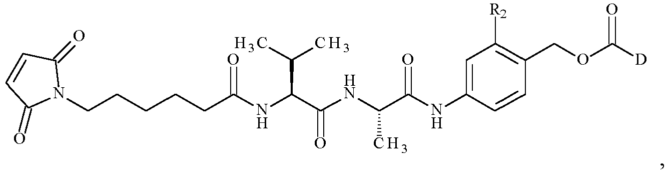 and 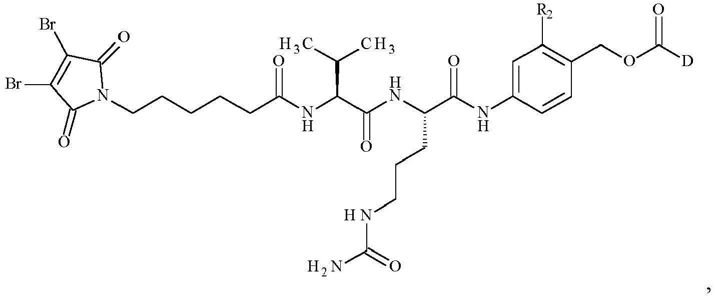 wherein R₂ and D are as defined in claim 9.

16. A Linker-Drug compound according to claim 9, which are:
- sodium 5-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl] carbamoyl]-2-methyl-propyl]-methyl-carbamoyl]oxymethyl]benzenesulfonate;
- sodium5-[[(2S)-2-[[(2S)-2-[6-(2,5-dioxopyrrol-1-yl)hexanoylamino]-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonate;
- sodium 5-[[(2S)-2-[[(2S)-2-[[2-[2-[2-(2azidoethoxy)ethoxy]ethoxy]acetyl]amino]-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl] carbamoyl]-2-methyl-propyl]-methyl-carbamoyl]oxymethyl]benzenesulfonate;
- 5-[[(2S)-2-[[1-[2-[2-(2,5-dioxopyrrol-1-yl)ethoxy]ethylcarbamoyl] cyclobutanecarbonyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl] carbamoyl]-2-methyl-propyl]-methyl-carbamoyl]oxymethyl]benzenesulfonic acid;
- sodium 5-[[(2S)-2-[[(2S)-2-[3-[2-(2,5-dioxopyrrol-1-yl)ethoxy]propanoylamino]-3-methyl-butanoyl]amino]propanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methylcarbamoyl]oxymethyl]benzenesulfonate;
- 5-[[(2S)-2-[[(2S)-2-[3-[2-(2,5-dioxopyrrol-1-yl)ethoxy]propanoylamino]-3-methyl-butanoyl]amino]propanoyl]amino]-2-[[[(1S)-1-[[(1S)-1-[[(S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methylcarbamoyl]oxymethyl]benzenesulfonic acid;
- 5-[[(2S)-2-[[3-[2-[2-(2,5-dioxopyrrol-1-yl)ethoxy]ethylcarbamoyl]oxetane-3-carbonyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzenesulfonic acid;
- [5-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenylethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methylpropyl]carbamoyl]-2-methyl-propyl]-methylcarbamoyl]oxymethyl]phenyl]methanesulfonic acid;
- [4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]-2-sulfo-phenyl]methyl-[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1 S)-1-methylpropyl]-4-oxo-butyl]-methyl-carbamoyl]-2-methyl-propyl]carbamoyl]-2-methyl-propyl]-dimethyl-ammonium; 2,2,2-trifluoroacetate;
- *N*-({[4-({*N*-[6-(2,5-dioxo-2,5-dihydro-1*H*-pyrrol-1-yl)hexanoyl]-L-valyl-L-alanyl}amino)-2-sulfophenyl]methoxy}carbonyl)-*N*-methyl-L-valyl-*N* [(3*R*,4*S*,5*S*)-1-{(2*S*)-2-[(1*R*,2*R*)-3-{[(1*S*,2*R*)-1-hydroxy-1-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamide;
- *N*-[6-(2,5-dioxo-2,5-dihydro-1*H*-pyrrol-1-yl)hexanoyl]-L-valyl-*N*⁵-carbamoyl-*N-*(4-{[({[(4*S*)-4,11-diethyl-9-hydroxy-3,14-dioxo-3,4,12,14-tetrahydro-1*H-*pyrano[3',4':6,7]indolizino[1,2-*b*]quinolin-4-yl]oxy}carbonyl)oxy]methyl}-3-sulfophenyl)-L-ornithinamide;
- (1*S*,3*S*)-3,5,12-trihydroxy-3-(hydroxyacetyl)-10-methoxy-6,11-dioxo-1,2,3,4,6,11-hexahydrotetracen-1-yl2,3,6-trideoxy-3-[({[4-({*N*-[6-(2,5-dioxo-2,5-dihydro-1*H-*pyrrol-1-yl)hexanoyl]-L-valyl-*N*⁵-carbamoyl-L-ornithyl}amino)-2-sulfophenyl]methoxy}carbonyl)amino]-α-L-*lyxo*-hexopyranoside;
- *N*-{3-[2-(2,5-dioxo-2,5-dihydro-1*H*-pyrrol-1-yl)ethoxy]propanoyl}-L-valyl-*N*-{4-[(5*S,*8*S,*11*S,*12*R*)-11-[(2*S*)-butan-2-yl]-12-(2-{(2*S*)-2-[(1*R*,2*R*)-3-{[(1*S*,2*R*)-1-hydroxy-1-phenylpropan-2-yl]amino} -1 -methoxy-2-methyl-3 - oxopropyl]pyrrolidin-1-yl}-2-oxoethyl)-4,10-dimethyl-3,6,9-trioxo-5,8-di(propan-2-yl)-2,13-dioxa-4,7,10-triazatetradecan-1-yl]-3-(2-sulfoethyl)phenyl}-*N*⁵-carbamoyl-L-ornithinamide;
- 5-[[(2S)-2-[[(2S)-2-[6-(3,4-dibromo-2,5-dioxo-pyrrol-1-yl)hexanoylamino]-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenylethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methylpropyl]carbamoyl]-2-methyl-propyl]-methylcarbamoyl]oxymethyl]benzenesulfonic acid.

17. An antibody-drug conjugate of Formula (III): 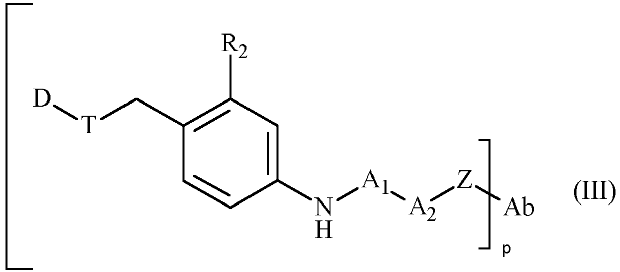 wherein
• Ab represents an antibody or an antigen-binding fragment thereof;
• D is a drug moiety;
• T is a bond, -O-C(O)-N(CH₃)-CH₂-CH₂-N(CH₃)-C(O)-*, -O-*, -NR₅-*, -NR₅-C(O)-*, or -O-C(O)-*, wherein the * indicates the point of attachment to D;
• Z represents a Spacer Unit;
• A₁ represents a -C(O)-CH(R₃)-NH- group;
• A₂ represents a -C(O)-CH(R₄)-NH- group, a 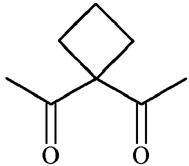 group, a 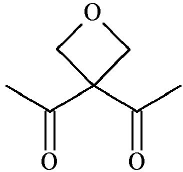 group, or a 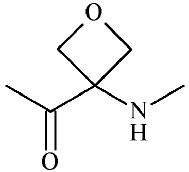 group;
• R₂ represents a -S(O)₂(OH) group, a -S(O)₂(O⁻M⁺) group, a linear or branched -(C₁-C₄)alkyl-S(O)₂(OH) group, a linear or branched -(C₁-C₄)alkyl-S(O)₂(O⁻M⁺) group, a linear or branched -halo(C₁-C₄)alkyl-S(O)₂(OH) group, or a linear or branched -halo(C₁-C₄)alkyl-S(O)₂(O⁻M⁺) group;
• R₃ and R₄, independently of one another, represent the side chain of an amino acid;
• R₅ represents a (C₁-C₄)alkyl group;
• M⁺ represents a pharmaceutically acceptable monovalent cation; and
• p is an integer from 1 to 8.

18. The antibody-drug conjugate according to claim 17, wherein T is a bond or -O-C(O)-*, wherein the * indicates the point of attachment to D.

19. The antibody-drug conjugate according to claim 17, wherein R₂ represents a -S(O)₂(OH) group, a -S(O)₂(O⁻M⁺) group, a -CH₂-S(O)₂(OH) group, a -CH₂-S(O)₂(O⁻M⁺) group, a -CH₂-CH₂-S(O)₂(OH) group, a -CH₂-CH₂-S(O)₂(O⁻M⁺) group, a -CH₂-CH₂-CH₂-S(O)₂(OH) group, or a -CH₂-CH₂-CH₂-S(O)₂(O⁻M⁺) group.

20. The antibody-drug conjugate according to claim 17, wherein A₁ represents a -C(O)-CH(R₃)-NH- group in which R₃ represents a -(CH₂)₃-NH-CO-NH₂ group or a methyl group.

21. The antibody-drug conjugate according to claim 17, wherein A₂ represents a -C(O)-CH(R₄)-NH- group in which R₄ represents an isopropyl group; a 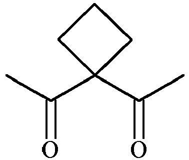 group; a 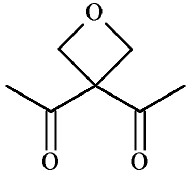 group; or a 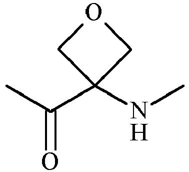 group.

22. The antibody-drug conjugate according to claim 17, wherein the said antibody-drug conjugate is formed from a Linker-Drug compound of Formula (II) selected from: 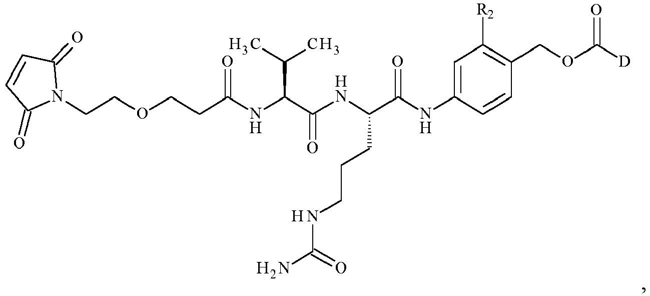 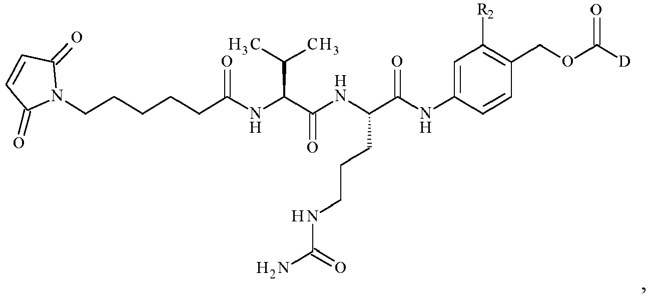 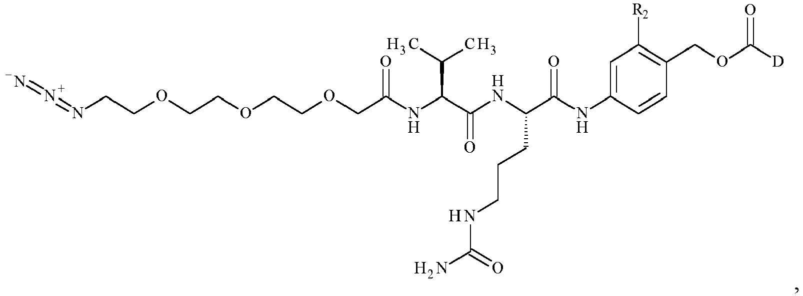 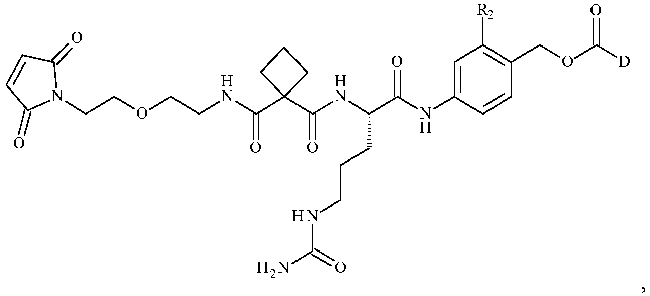 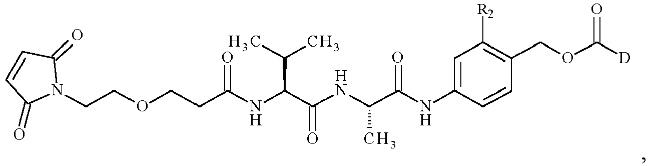 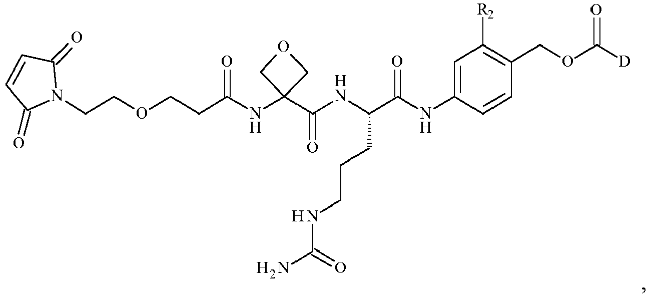 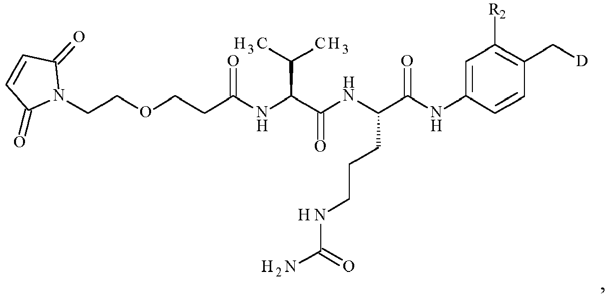 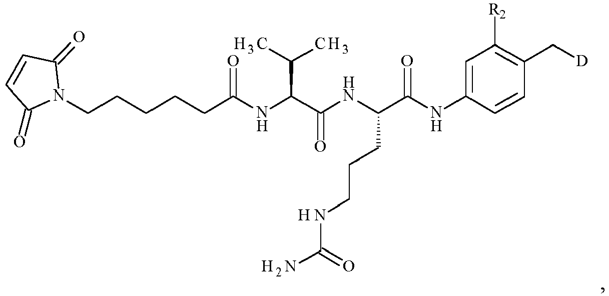 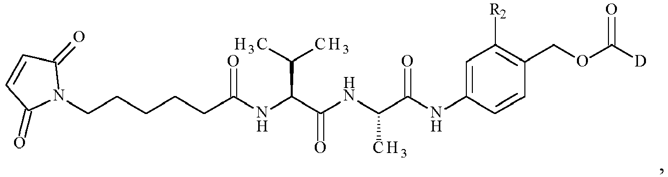 and 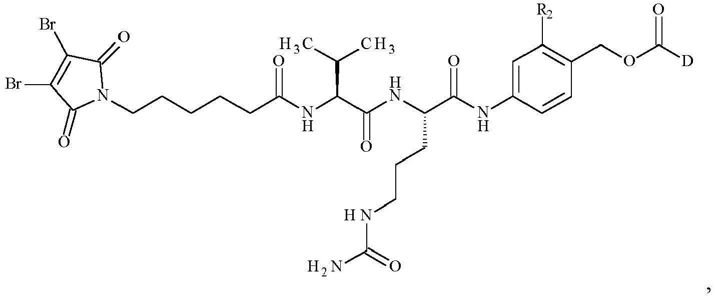 wherein R₂ and D are as defined in claim 17.

23. The antibody-drug conjugate according to claim 17, wherein the said antibody-drug conjugate comprises a Formula selected from: 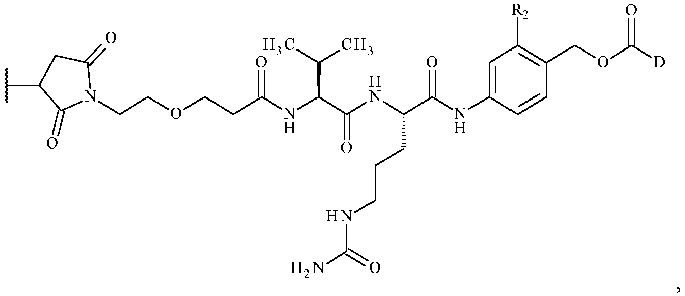 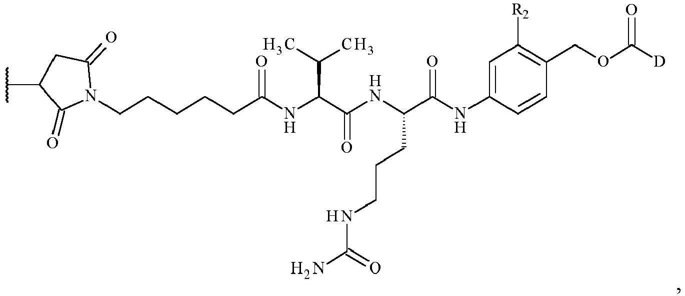 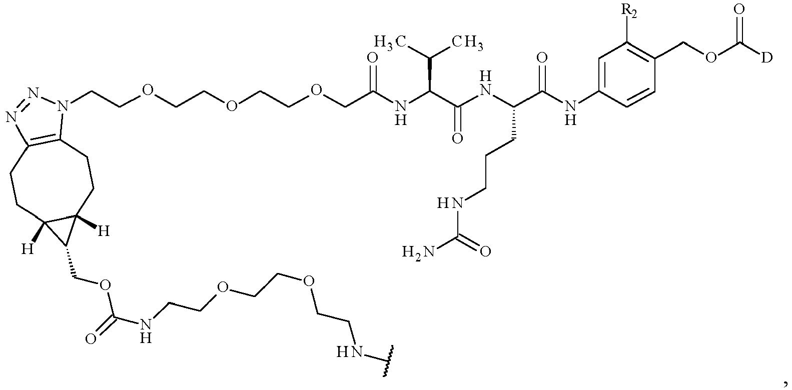 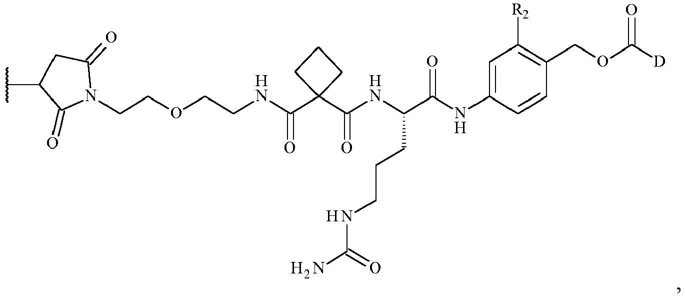 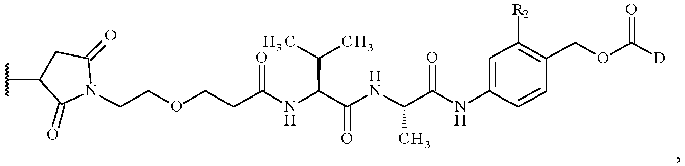 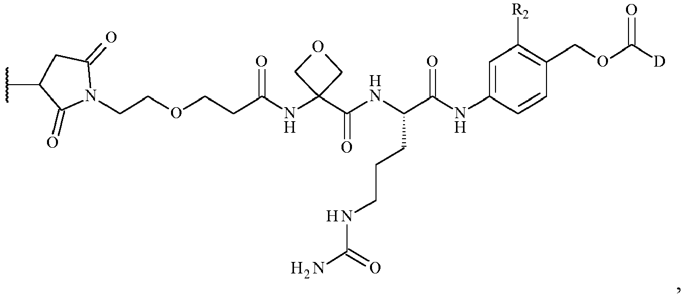 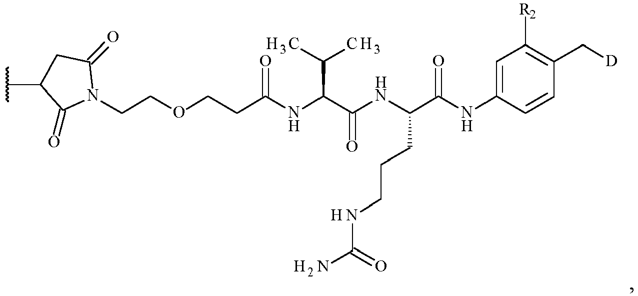 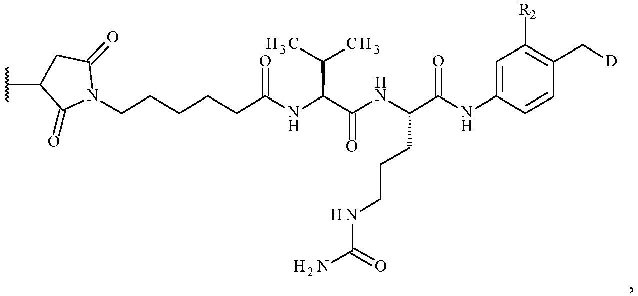 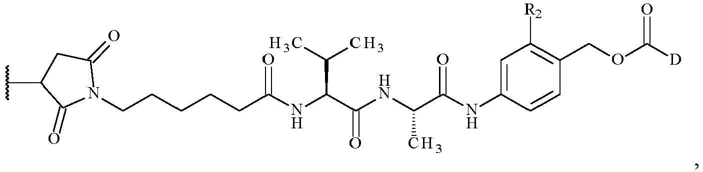 and 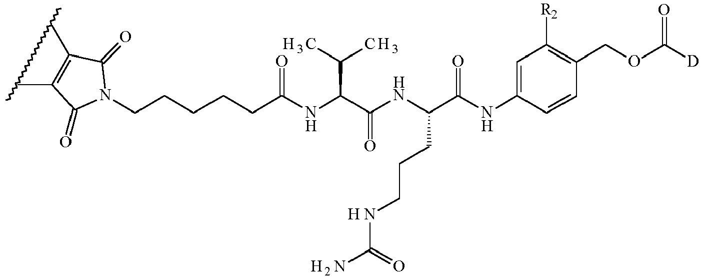 , wherein wavy line indicates the covalent attachment site to the antibody or antigen-binding fragment thereof and, D and R₂ are as defined in claim 17.

24. A pharmaceutical composition comprising the antibody-drug conjugate according to any one of claims 17 to 23, and a pharmaceutically carrier.

25. The antibody-drug conjugate according to any one of claims 17 to 23, for use in the treatment of a mammal being in need thereof.

## Patentansprüche

1. para-Amino-Benzyl-Linker-Verbindung der Formel (I): 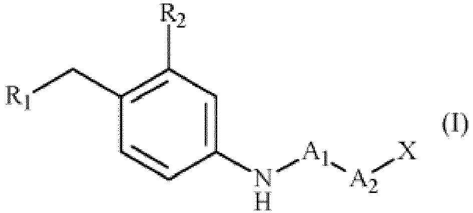 wobei:
• R₁ eine Hydroxygruppe oder ein Halogenatom darstellt;
• R₂ eine -S(O)₂(OH)-Gruppe, eine -S(O)₂(O⁻M⁺)-Gruppe, eine lineare oder verzweigte -(C₁-C₄)Alkyl-S(O)₂(OH)-Gruppe, eine lineare oder verzweigte -(C₁-C₄)Alkyl-S(O)₂(O⁻M⁺)-Gruppe, eine lineare oder verzweigte -Halogen(C₁-C₄)Alkyl-S(O)₂(OH)-Gruppe oder a lineare oder verzweigte -Halogen(C₁-C₄)-Alkyl-S(O)₂(O⁻M⁺)-Gruppe darstellt;
• A₁ eine -C(O)-CH(R₃)-NH-Gruppe darstellt;
• A₂ eine -C(O)-CH(R₄)-NH-Gruppe, eine 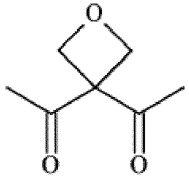 Gruppe, eine 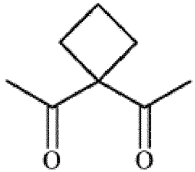 Gruppe oder eine 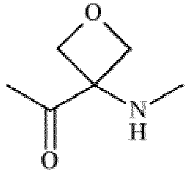 Gruppe darstellt;
• R₃ und R₄ unabhängig voneinander die Seitenkette einer Aminosäure darstellen;
• X ein Wasserstoffatom, eine Hydroxygruppe oder eine Schutzgruppe darstellt;
• M⁺ ein pharmazeutisch verträgliches einwertiges Kation darstellt.

2. para-Amino-Benzyl-Linker-Verbindung nach Anspruch 1, wobei R₁ eine Hydroxygruppe, ein Bromatom, ein Chloratom oder ein Iodatom darstellt.

3. para-Amino-Benzyl-Linker-Verbindung nach Anspruch 1, wobei R₂ eine -S(O)₂(OH)-Gruppe, eine -S(O)₂(O⁻M⁺)-Gruppe, eine -CH₂-S(O)₂-Gruppe, eine -CH₂-S(O)₂(O⁻M⁺)-Gruppe, eine -CH₂-CH₂-S(O)₂(OH)-Gruppe, eine -CH₂-CH₂-S(O)₂(O⁻M⁺)-Gruppe, eine -CH₂-CH₂-CH₂-S(O)₂(OH)-Gruppe oder eine -CH₂-CH₂-CH₂-S(O)₂(O⁻M⁺)-Gruppe darstellt.

4. para-Amino-Benzyl-Linker-Verbindung nach Anspruch 1, wobei A₁ eine -C(O)-CH(R₃)-NH-Gruppe darstellt, in der R₃ eine -(CH₂)₃-NH-CO-NH₂-Gruppe oder eine Methylgruppe darstellt.

5. para-Amino-Benzyl-Linker-Verbindung nach Anspruch 1, wobei A₂ eine -C(O)-CH(R₄)-NH-Gruppe darstellt, in der R₄ eine Isopropylgruppe; eine 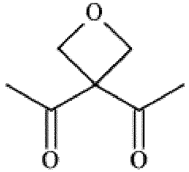 Gruppe; eine 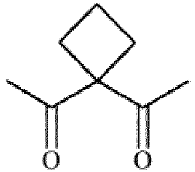 Gruppe oder eine 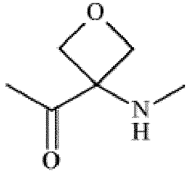 Gruppe darstellt.

6. para-Amino-Benzyl-Linker-Verbindung nach Anspruch 1, wobei A₁ eine - C(O)-CH(R₃)-NH-Gruppe darstellt und A₂ eine -C(O)-CH(R₄)-NH-Gruppe darstellt, in denen R₃ und R₄ unabhängig voneinander die Seitenkette einer Aminosäure darstellen.

7. para-Amino-Benzyl-Linker-Verbindung nach Anspruch 1, welche die folgenden sind:
- Natrium 5-[[(2S)-2-[[(2S)-2-(9H-fluoren-9-ylmethoxycarbonylamino)-3-methyl-butanoyl]amino]-5-ureido-pentanoyl]amino]-2-(hydroxymethyl)benzolsulfonat;
- Natrium 5-[[(2S)-2-[[(2S)-2-(9H-fluoren-9-ylmethoxycarbonylamino)-3-methyl-butanoyl]amino]propanoyl]amino]-2-(hydroxymethyl)benzolsulfonat;
- 5-[[(2S)-2-[[(2S)-2-(9H-fluoren-9-ylmethoxycarbonylamino)-3-methylbutanoyl]amino]propanoyl]amino]-2-(hydroxymethyl)benzolsulfonsäure;
- Natrium [5-[[(2S)-2-[[(2S)-2-(9H-fluoren-9-ylmethoxycarbonylamino)-3-methyl-butanoyl]amino]-5-ureido-pentanoyl]amino]-2-(hydroxymethyl)phenyl]methansulfonat;
- 2-(Chlormethyl)-5-[[(2S)-2-[[(2S)-2-(9H-fluoren-9-ylmethoxycarbonylamino)-3-methyl-butanoyl]amino]-5-ureido-pentanoyl]amino]benzolsulfonsäure;
- 2-(Chlormethyl)-5-[[(2S)-2-[[(2S)-2-(9H-fluoren-9-ylmethoxycarbonylamino)-3-methyl-butanoyl]amino]propanoyl]amino]benzolsulfonsäure;
- 5-[[(2S)-2-[[(2S)-2-(9H-fluoren-9-ylmethoxycarbonylamino)-3-methyl-butanoyl]amino]-5-ureido-pentanoyl]amino]-2-(iodmethyl)benzolsulfonsäure;
- Natrium *N*-{[(9*H*-fluoren-9-yl)methoxy]carbonyl}-L-valyl-*N*⁵-carbamoyl-*N*-[4-(hydroxymethyl)-3-(2-sulfonatoethyl)phenyl]-L-ornithinamid;
- *N*-{[(9*H*-fluoren-9-yl)methoxy]carbonyl}-L-valyl-*N*⁵-carbamoyl-*N*-[4-(hydroxymethyl)-3-(3-sulfopropyl)phenyl]-L-ornithinamid.

8. para-Amino-Benzyl-Linker-Verbindung der Formel (I) nach einem der Ansprüche 1 bis 7 zur Verwendung bei der Herstellung eines Antikörper-Arzneimittel-Konjugats.

9. Linker-Arzneimittel-Verbindung der Formel (II): 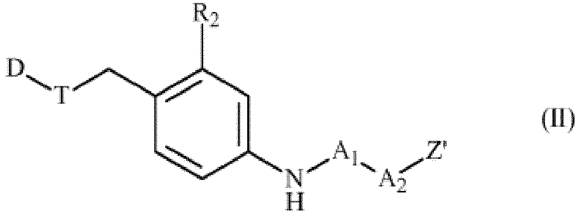 wobei:
• D eine Arzneimittel-Einheit darstellt;
• T eine Bindung, -O-C(O)-N(CH₃)-CH₂-CH₂-N(CH₃)-C(O)-*, -O-*, -NR₅-*, -NR₅-C(O)-* oder -O-C(O)-* ist, wobei das * den Bindungspunkt an D angibt;
• R₂ eine -S(O)₂(OH)-Gruppe, eine -S(O)₂(O⁻M⁺)-Gruppe, eine lineare oder verzweigte -(C₁-C₄)Alkyl-S(O)₂(OH)-Gruppe, eine lineare oder verzweigte -(C₁-C₄)-Alkyl-S(O)₂(O⁻M⁺)-Gruppe, eine lineare oder verzweigte -Halogen(C₁-C₄)-Alkyl-S(O)₂(OH)-Gruppe oder eine lineare oder eine verzweigte -Halogen(C₁-C₄)-Alkyl-S(O)₂(O⁻M⁺)-Gruppe darstellt;
• A₁ eine -C(O)-CH(R₃)-NH-Gruppe darstellt;
• A₂ eine -C(O)-CH(R₄)-NH-Gruppe, eine 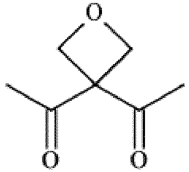 Gruppe, eine 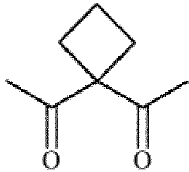 Gruppe oder eine 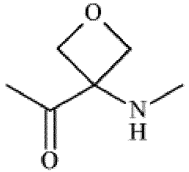 Gruppe darstellt;
• R₃ und R₄ unabhängig voneinander die Seitenkette einer Aminosäure darstellen;
• R₅ ein Wasserstoffatom oder eine (C₁-C₄)Alkylgruppe darstellt;
• Z' einen Vorläufer einer Spacer-Einheit darstellt;
• M⁺ ein pharmazeutisch verträgliches einwertiges Kation darstellt.

10. Linker-Arzneimittel-Verbindung nach Anspruch 9, wobei R₂ eine -S(O)₂(OH)-Gruppe, eine -S(O)₂(O⁻M⁺)-Gruppe, eine -CH₂-S(O)₂(OH)-Gruppe, eine -CH₂-S(O)₂(O⁻M⁺)-Gruppe, eine -CH₂-CH₂-S(O)₂(OH)-Gruppe, eine -CH₂-CH₂-S(O)₂(O⁻M⁺)-Gruppe , eine -CH₂-CH₂-CH₂-S(O)₂(OH)-Gruppe oder eine -CH₂-CH₂-CH₂-S(O)₂(O⁻M⁺)-Gruppe darstellt.

11. Linker-Arzneimittel-Verbindung nach Anspruch 9, wobei A₁ eine -C(O)-CH(R₃)-NH-Gruppe darstellt, in der R₃ eine -(CH₂)₃-NH-CO-NH₂-Gruppe oder eine Methylgruppe darstellt.

12. Linker-Arzneimittel-Verbindung nach Anspruch 9, wobei A₂ eine -C(O)-CH(R₄)-NH-Gruppe darstellt, in der R₄ eine Isopropylgruppe; eine 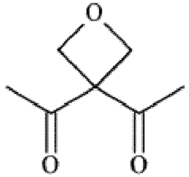 Gruppe; eine 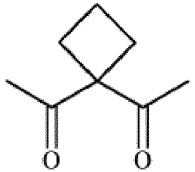 Gruppe oder eine 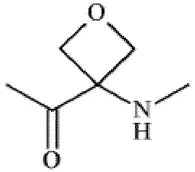 Gruppe darstellt.

13. Linker-Arzneimittel-Verbindung nach Anspruch 9, wobei T eine Bindung oder -O-C(O)-* darstellt, wobei das * den Bindungspunkt an D angibt.

14. Linker-Arzneimittel-Verbindung nach Anspruch 9, wobei Z' eine Gruppe darstellt, ausgewählt aus: 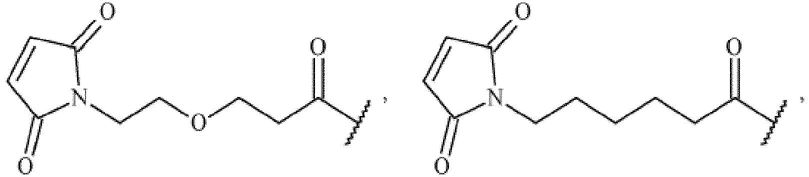 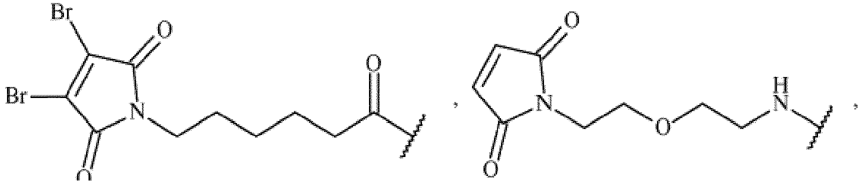 oder 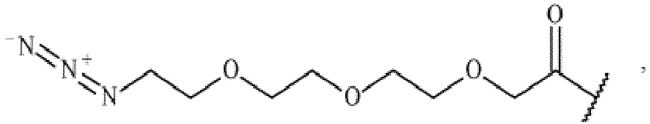 wobei die Wellenlinie die kovalente Bindungsstelle an den N-Terminus oder die Carbonylgruppe der A₂-Gruppe anzeigt.

15. Linker-Arzneimittel-Verbindung nach Anspruch 9, welche die folgenden sind: 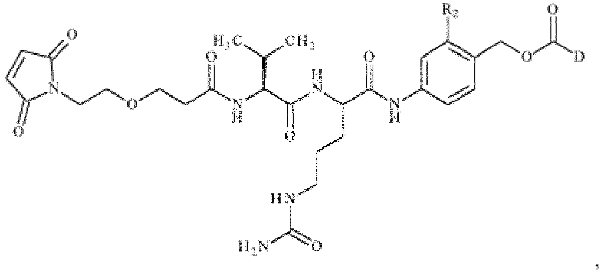 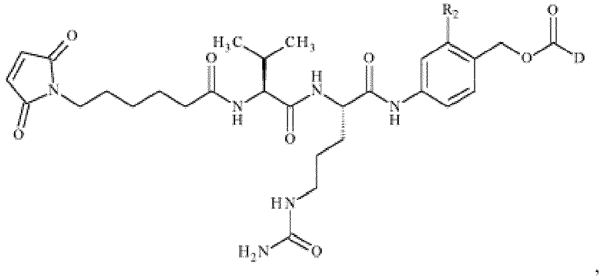 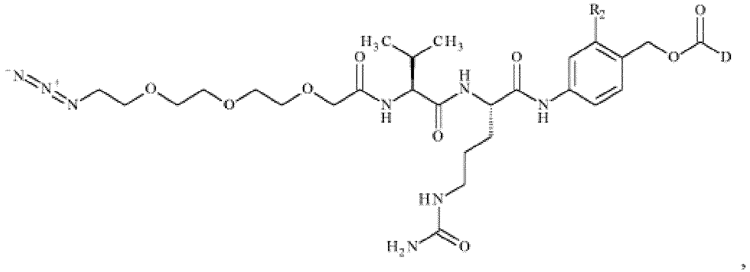 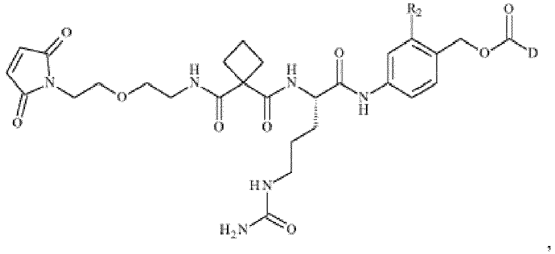 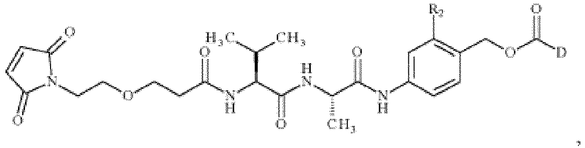 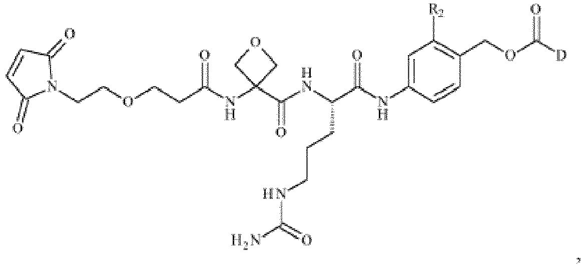 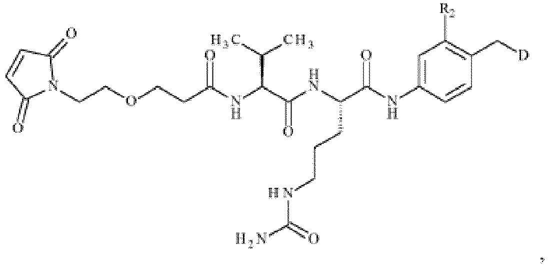 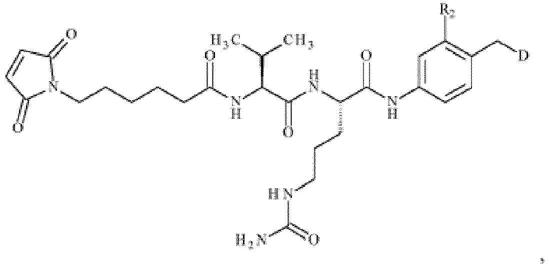 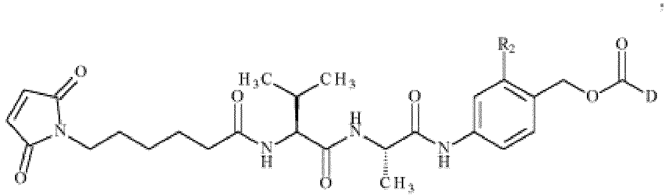 und 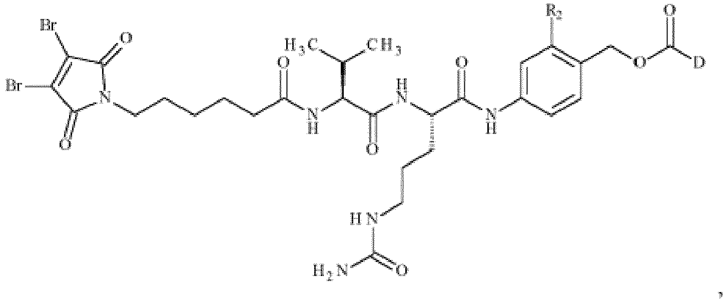 wobei R₂ und D wie in Anspruch 9 definiert sind.

16. Linker-Arzneimittel-Verbindung nach Anspruch 9, welche die folgenden sind:
- Natrium 5-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]-carbamoyl]-2-methyl-propyl]-methylcarbamoyl]oxymethyl]benzolsulfonat;
- Natrium 5-[[(2S)-2-[[(2S)-2-[6-(2,5-dioxopyrrol-1-yl)hexanoylamino]-3-methylbutanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenylethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methylpropyl]carbamoyl]-2-methyl-propyl]-methylcarbamoyl]oxymethyl]benzolsulfonat;
- Natrium 5-[[(2S)-2-[[(2S)-2-[[2-[2-[2-(2azidoethoxy)ethoxy]ethoxy]acetyl]amino]-3-methylbutanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]-carbamoyl]-2-methyl-propyl]-methylcarbamoyl]oxymethyl]benzolsulfonat;
- 5-[[(2S)-2-[[1-[2-[2-(2,5-dioxopyrrol-1-yl)ethoxy]ethylcarbamoyl]cyclobutancarbonyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1 S)-1-methylpropyl]-4-oxo-butyl]-methyl-carbamoyl]-2-methyl-propyl]-carbamoyl]-2-methyl-propyl]-methylcarbamoyl]oxymethyl]benzolsulfonsäure;
- Natrium-5-[[(2S)-2-[[(2S)-2-[3-[2-(2,5-dioxopyrrol-1-yl)ethoxy]propanoylamino]-3-methyl-butanoyl]amino]propanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1 S)-1-methylpropyl]-4-oxo-butyl]-methyl-carbamoyl]-2-methyl-propyl]carbamoyl]-2-methyl-propyl]-methylcarbamoyl]oxymethyl]benzolsulfonat;
- 5-[[(2S)-2-[[(2S)-2-[3-[2-(2,5-dioxopyrrol-1-yl)ethoxy]propanoylamino]-3-methylbutanoyl]amino]propanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzolsulfonsäure;
- 5-[[(2S)-2-[[3-[2-[2-(2,5-dioxopyrrol-1-yl)ethoxy]ethylcarbamoyl]oxetan-3-carbonyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxymethyl]benzolsulfonsäure;
- [5-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenylethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methylpropyl]carbamoyl]-2-methyl-propyl]-methylcarbamoyl]oxymethyl]phenyl]methansulfonsäure;
- [4-[[(2S)-2-[[(2S)-2-[3-[2-(2,5-dioxopyrrol-1-yl)ethoxy]propanoylamino]-3-methyl-butanoyl]amino]-5-ureido-pentanoyl]amino]-2-sulfo-phenyl]methyl-[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1 S)-1-methylpropyl]-4-oxo-butyl]-methyl-carbamoyl]-2-methylpropyl]carbamoyl]-2-methyl-propyl]-dimethyl-ammonium; 2,2,2-Trifluoracetat;
- *N*-({[4-({*N*-[6-(2,5-dioxo-2,5-dihydro-1*H*-pyrrol-1-yl)hexanoyl]-L-valyl-L-alanyl}amino)-2-sulfophenyl]methoxy}carbonyl)-*N*-methyl-L-valyl-*N-*[(3*R*,4*S*,5*S*)-1-{(2*S*)-2-[(1*R*,2*R*)-3-{[(1*S*,2*R*)-1-hydroxy-1-phenylpropan-2-yl]amino}-1-methoxy-2-methyl-3-oxopropyl]pyrrolidin-1-yl}-3-methoxy-5-methyl-1-oxoheptan-4-yl]-*N-*methyl-L-valinamid;
- *N*-[6-(2,5-dioxo-2,5-dihydro-1*H*-pyrrol-1-yl)hexanoyl]-L-valyl-*N*⁵-carbamoyl-*N-*(4-{[({[(4S)-4,11-diethyl-9-hydroxy-3,14-dioxo-3,4,12,14-tetrahydro-1H-pyrano[3',4':6,7]indolizino[1,2-*b*]chinolin-4-yl]oxy}carbonyl)oxy]methyl}-3-sulfophenyl)-L-ornithinamid;
- (1*S*,3*S*)-3,5,12-trihydroxy-3-(hydroxyacetyl)-10-methoxy-6,11-dioxo-1,2,3,4,6,11-hexahydrotetracen-1-yl-2,3,6-tridesoxy-3-[({[4-({*N-*[6-(2,5-dioxo-2,5-dihydro-1*H*-pyrrol-1-yl)hexanoyl]-L-valyl-*N*⁵-carbamoyl-L-ornithyl}amino)-2-sulfophenyl]methoxy}carbonyl)amino]-α-L-*lyxo+*-hexopyranosid;
- *N*-{3-[2-(2,5-dioxo-2,5-dihydro-1*H*-pyrrol-1-yl)ethoxy]propanoyl}-L-valyl-*N-*{4-[(5*S*,8*S*,11*S*,12*R*)-11-[(2*S*)-butan-2-yl]-12-(2-{(2*S*)-2-[(1*R*,2*R*)-3-{[(1*S*,2*R*)-1-hydroxy-1-phenylpropan-2-yl]amino}-1-methoxy-2-methyl-3 - oxopropyl]pyrrolidin-1-yl}-2-oxoethyl)-4,10-dimethyl-3,6,9-trioxo-5,8-di(propan-2-yl)-2,13-dioxa-4,7,10-triazatetradecan-1-yl]-3-(2-sulfoethyl)phenyl} -*N*⁵-carbamoyl-L-ornithinamid;
- 5-[[(2S)-2-[[(2S)-2-[6-(3,4-dibrom-2,5-dioxo-pyrrol-1-yl)hexanoylamino] -3-methyl-butanoyl]amino]-5-ureido-pentanoyl]amino]-2-[[[(1S)-1-[[(1S)-1-[[(1S,2R)-4-[(2S)-2-[(1R,2R)-3-[[(1R,2S)-2-hydroxy-1-methyl-2-phenylethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methylpropyl]carbamoyl]-2-methyl-propyl]-methylcarbamoyl]oxymethyl]benzolsulfonsäure.

17. Antikörper-Wirkstoff-Konjugat der Formel (III): 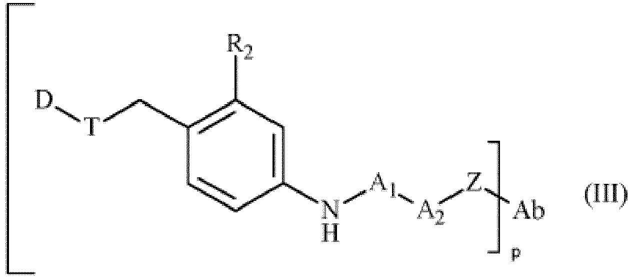 wobei:
• Ab einen Antikörper oder ein Antigen-bindendes Fragment davon darstellt;
• D ein Arzneimittelrest ist;
• T eine Bindung, -O-C(O)-N(CH₃)-CH₂-CH₂-N(CH₃)-C(O)-*, -O-*, -NR₅-*, -NR₅-C(O)-* oder -O-C(O)-* ist, wobei das * den Bindungspunkt an D angibt;
• Z eine Spacer-Einheit darstellt;
• A₁ eine -C(O)-CH(R₃)-NH-Gruppe darstellt;
• A₂ eine -C(O)-CH(R₄)-NH-Gruppe, eine 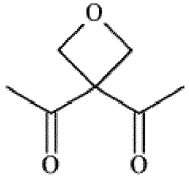 Gruppe, eine 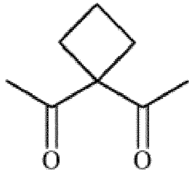 Gruppe oder eine 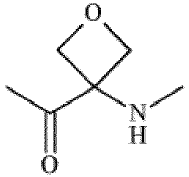 Gruppe darstellt;
• R₂ eine -S(O)₂(OH)-Gruppe, eine -S(O)₂(O⁻M⁺)-Gruppe, eine lineare oder verzweigte -(C₁-C₄)Alkyl-S(O)₂(OH)-Gruppe, eine lineare oder verzweigte -(C₁-C₄)-Alkyl-S(O)₂(O⁻M⁺)-Gruppe, eine lineare oder verzweigte -Halogen(C₁-C₄)-Alkyl-S(O)₂(OH)-Gruppe oder eine lineare oder eine verzweigte -Halogen(C₁-C₄)-Alkyl-S(O)₂(O⁻M⁺)-Gruppe darstellt;
• R₃ und R₄ unabhängig voneinander die Seitenkette einer Aminosäure darstellen;
• R₅ eine (C₁-C₄)Alkylgruppe darstellt;
• M⁺ ein pharmazeutisch verträgliches einwertiges Kation darstellt; und
• p eine ganze Zahl von 1 bis 8 ist.

18. Antikörper-Wirkstoff-Konjugat nach Anspruch 17, wobei T eine Bindung oder -O-C(O)-* ist, wobei das * den Bindungspunkt an D angibt.

19. Antikörper-Wirkstoff-Konjugat nach Anspruch 17, wobei R₂ eine -S(O)₂(OH)-Gruppe, eine -S(O)₂(O⁻M⁺)-Gruppe, eine -CH₂-S(O)₂(OH)-Gruppe eine -CH₂-S(O)₂(O⁻M⁺)-Gruppe, eine -CH₂-CH₂-S(O)₂(OH)-Gruppe, eine -CH₂-CH₂-S(O)₂(O⁻M⁺)-Gruppe, eine -CH₂-CH₂-CH₂-S(O)₂(OH)-Gruppe oder eine -CH₂-CH₂-CH₂-S(O)₂(O⁻M⁺)-Gruppe darstellt.

20. Antikörper-Wirkstoff-Konjugat nach Anspruch 17, wobei A₁ eine -C(O)-CH(R₃)-NH-Gruppe darstellt, in der R₃ eine -(CH₂)₃-NH-CO-NH₂-Gruppe oder eine Methylgruppe darstellt.

21. Antikörper-Wirkstoff-Konjugat nach Anspruch 17, wobei A₂ eine -C(O)-CH(R₄)-NH-Gruppe darstellt, in der R₄ eine Isopropylgruppe; eine 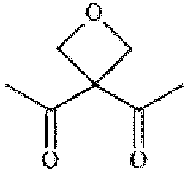 Gruppe; eine 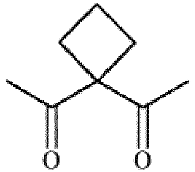 Gruppe oder eine 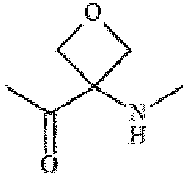 Gruppe darstellt.

22. Antikörper-Wirkstoff-Konjugat nach Anspruch 17, wobei das Antikörper-Wirkstoff-Konjugat aus einer Linker-Wirkstoff-Verbindung der Formel (II) gebildet wird, ausgewählt aus: 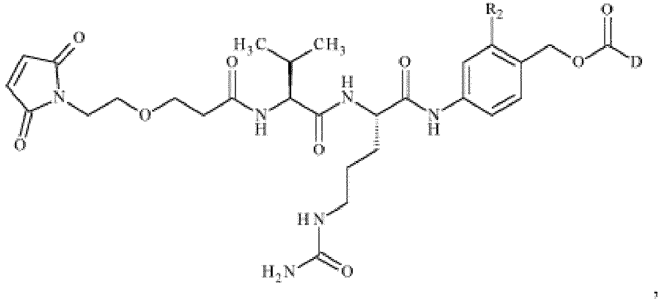 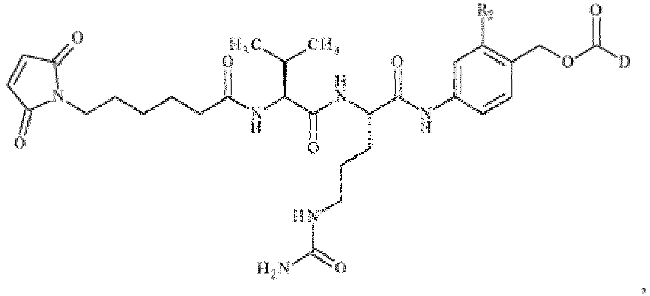 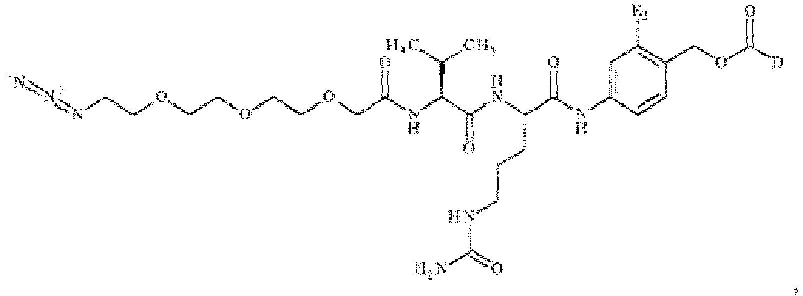 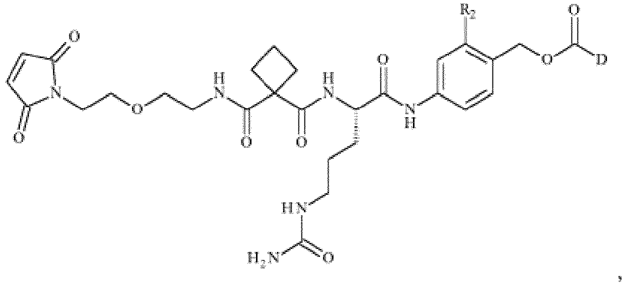 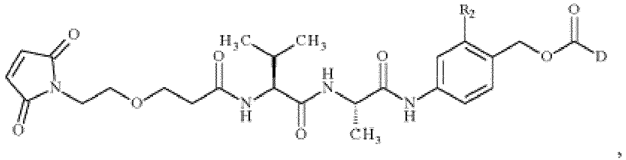 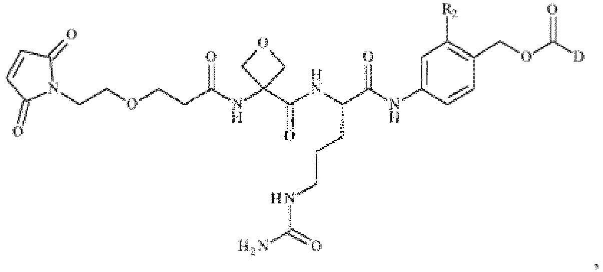 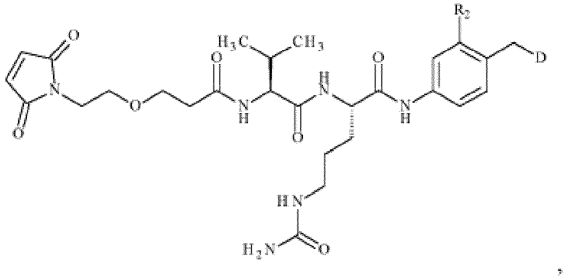 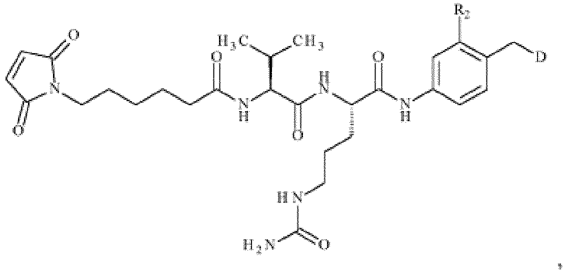 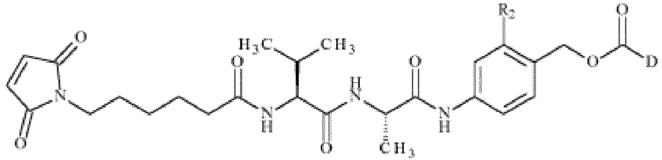 und 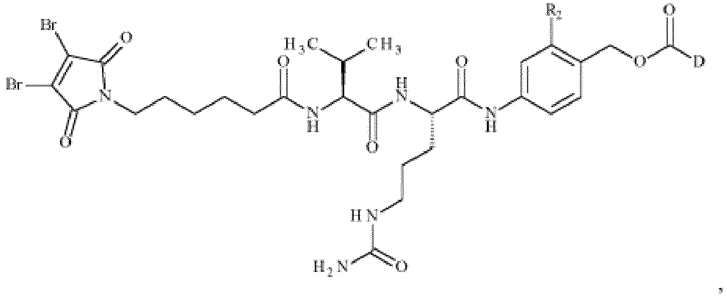 wobei R₂ und D wie in Anspruch 17 definiert sind.

23. Antikörper-Wirkstoff-Konjugat nach Anspruch 17, wobei das Antikörper-Wirkstoff-Konjugat eine Formel umfasst, ausgewählt aus: 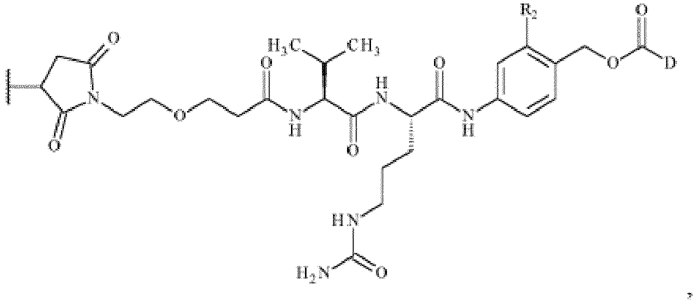 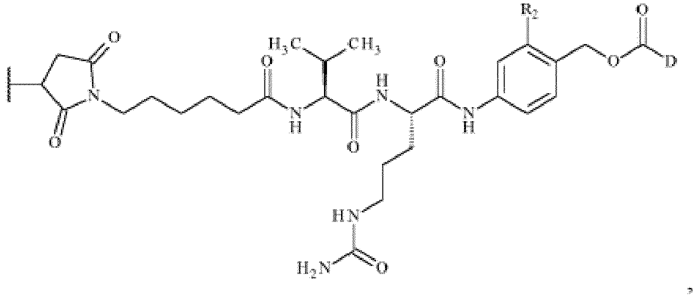 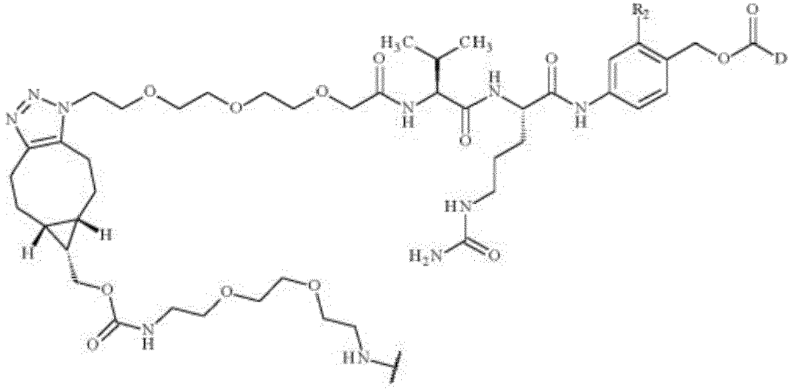 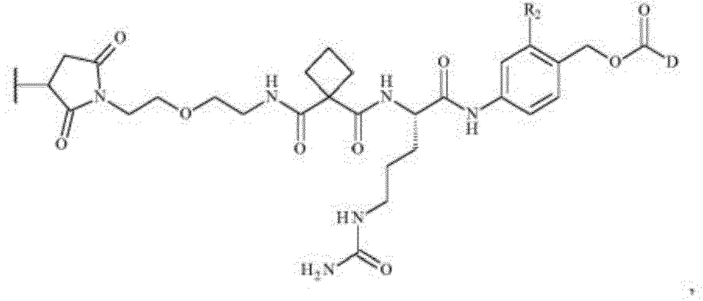 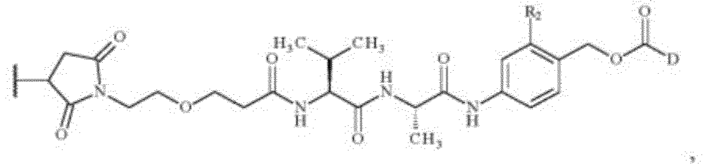 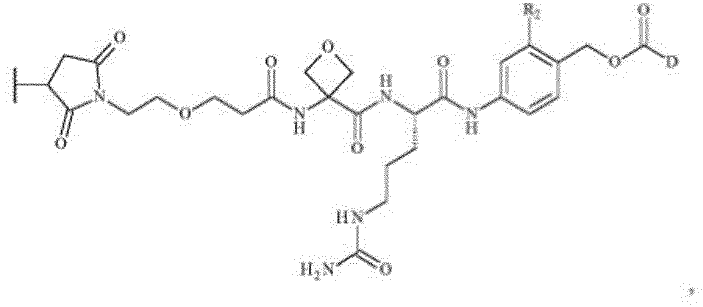 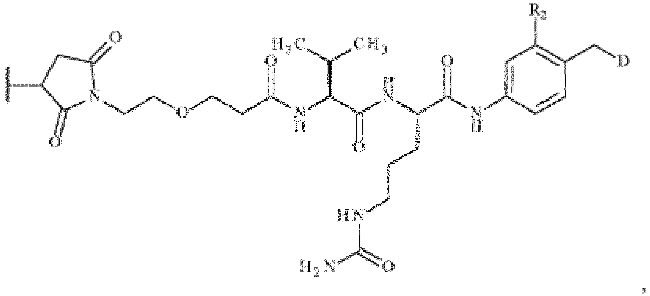 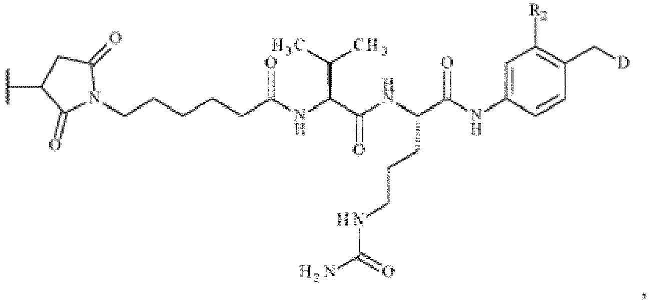 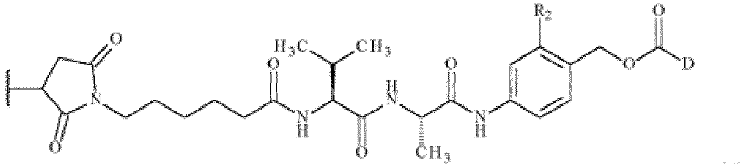 und 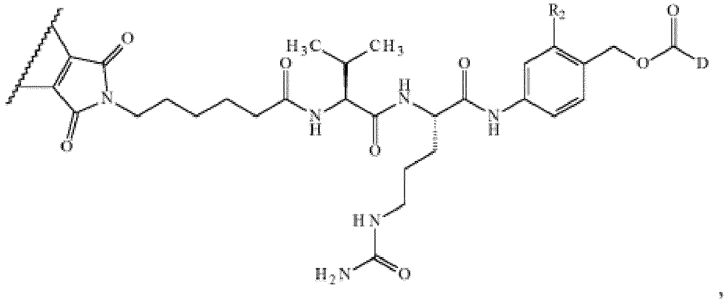 wobei die Wellenlinie die kovalente Bindungsstelle an den Antikörper oder das antigenbindende Fragment davon anzeigt und D und R₂ wie in Anspruch 17 definiert sind.

24. Pharmazeutische Zusammensetzung, umfassend das Antikörper-Wirkstoff-Konjugat nach einem der Ansprüche 17 bis 23 und einen pharmazeutischen Träger.

25. Antikörper-Wirkstoff-Konjugat nach einem der Ansprüche 17 bis 23 zur Verwendung bei der Behandlung eines Säugetiers, das dieses benötigt.

## Revendications

1. Composé de liaison para-amino-benzyle de formule (I) : 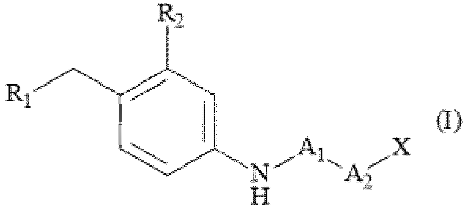 dans lequel :
• R₁ représente un groupe hydroxyle ou un atome d'halogène ;
• R₂ représente un groupe -S(O)₂(OH), un groupe -S(O)₂(O⁻M⁺), un groupe -(alkyl en C₁-C₄)-S(O)₂(OH) linéaire ou ramifié, un groupe -(alkyl en C₁-C₄)-S(O)₂(O⁻M⁺)linéaire ou ramifié, un groupe -halogéno(alkyl en C₁-C₄)-S(O)₂(OH) linéaire ou ramifié, ou un groupe -halogéno(alkyl en C₁-C₄)-S(O)₂(O⁻M⁺) linéaire ou ramifié ;
• A₁ représente un groupe -C(O)-CH(R₃)-NH- ;
• A₂ représente un groupe -C(O)-CH(R₄)-NH-, un groupe 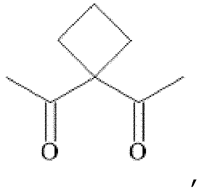 un groupe 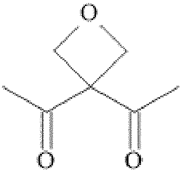 ou un groupe 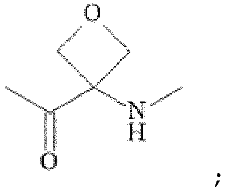
• R₃ et R₄, indépendamment l'un de l'autre, représentent la chaîne latérale d'un acide aminé ;
• X représente un atome d'hydrogène, un groupe hydroxyle ou un groupe protecteur ;
• M⁺ représente un cation monovalent pharmaceutiquement acceptable.

2. Composé de liaison para-amino-benzyle selon la revendication 1, dans lequel R₁ représente un groupe hydroxyle, un atome de brome, un atome de chlore ou un atome d'iode.

3. Composé de liaison para-amino-benzyle selon la revendication 1, dans lequel R₂ représente un groupe -S(O)₂(OH), un groupe -S(O)₂(O⁻M⁺), un groupe -CH₂-S(O)₂(OH), un groupe -CH₂-S(O)₂(O⁻M⁺), un groupe -CH₂-CH₂-S(O)₂(OH), un groupe -CH₂-CH₂-S(O)₂(O⁻M⁺), un groupe -CH₂-CH₂-CH₂-S(O)₂(OH) ou un groupe -CH₂-CH₂-CH₂-S(O)₂(O⁻M⁺).

4. Composé de liaison para-amino-benzyle selon la revendication 1, dans lequel A₁ représente un groupe -C(O)-CH(R₃)-NH- dans lequel R₃ représente un groupe -(CH₂)₃-NH-CO-NH₂ ou un groupe méthyle.

5. Composé de liaison para-amino-benzyle selon la revendication 1, dans lequel A₂ représente un groupe -C(O)CH(R₄)-NH-, dans lequel R₄ représente un groupe isopropyle ; un groupe 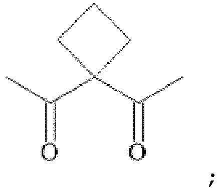 un groupe 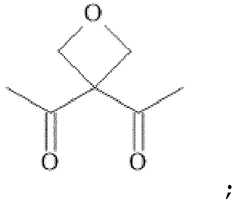 ou un groupe

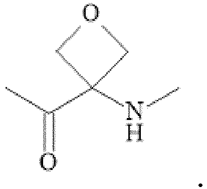

6. Composé de liaison para-amino-benzyle selon la revendication 1, dans lequel A₁ représente un groupe -C(O)-CH(R₃)-NH- et A₂ représente un groupe -C(O)-CH(R₄)-NH-, dans lequel R₃ et R₄, indépendamment l'un de l'autre, représentent la chaîne latérale d'un acide aminé.

7. Composé de liaison para-amino-benzyle selon la revendication 1, qui est :
- le 5-[[(2S)-2-[[(2S)-2-(9H-fluorén-9-ylméthoxycarbonylamino)-3-méthyl-butanoyl]amino]-5-uréido-pentanoyl]amino]-2-(hydroxyméthyl)benzènesulfonate de sodium ;
- le 5-[[(2S)-2-[[(2S)-2-(9H-fluorén-9-ylméthoxycarbonylamino)-3-méthyl-butanoyl]amino]propanoyl]amino]-2-(hydroxyméthyl)benzènesulfonate de sodium ;
- l'acide 5-[[(2S)-2-[[(2S)-2-(9H-fluorén-9-ylméthoxycarbonylamino)-3-méthyl-butanoyl]amino]propanoyl]amino]-2-(hydroxyméthyl)benzènesulfonique ;
- le [5-[[(2S)-2-[[(2S)-2-(9H-fluorén-9-ylméthoxycarbonylamino)-3-méthyl-butanoyl]amino]-5-uréido-pentanoyl]amino]-2-(hydroxyméthyl)phényl]méthanesulfonate de sodium ;
- l'acide 2-(chlorométhyl)-5-[[(2S)-2-[[(2S)-2-(9H-fluorén-9-ylméthoxycarbonylamino)-3-méthyl-butanoyl]amino]-5-uréido-pentanoyl]amino]benzènesulfonique;
- l'acide 2-(chlorométhyl)-5-[[(2S)-2-[[(2S)-2-(9H-fluorén-9-ylméthoxycarbonylamino)-3-méthyl-butanoyl]amino]propanoyl]amino]benzènesulfonique ;
- l'acide 5-[[(2S)-2-[[(2S)-2-(9H-fluorén-9-ylméthoxycarbonylamino)-3-méthyl-butanoyl]amino]-5-uréido-pentanoyl]amino]-2-(iodométhyl)benzènesulfonique ;
- le *N*-{[(9*H*-fluorén-9-yl)méthoxy]carbonyl}-L-valyl-*N*⁵-carbamoyl-*N*-[4-(hydroxyméthyl)-3-(2-sulfonatoéthyl)phényl]-L-ornithinamide de sodium ;
- *le N*-{[(9*H*-fluorén-9-yl)méthoxy]carbonyl}-L-valyl-*N*⁵-carbamoyl- *N*-[4-(hydroxyméthyl)-3-(3-sulfopropyl)phényl]-L-ornithinamide.

8. Composé de liaison para-amino-benzyle de formule (I) selon l'une quelconque des revendications 1 à 7 destiné à être utilisé dans la préparation d'un conjugué anticorps-médicament.

9. Composé de liaison-médicament de formule (II) : 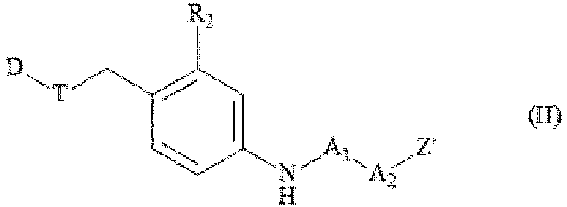 dans lequel :
• D représente un groupement médicamenteux ;
• T est une liaison, -O-C(O)-N(CH₃)-CH₂-CH₂-N(CH₃)-C(O)-*, - O-*, -NR₅-*, -NR₅-C(O)-*, ou -O-C(O)-*, dans lequel * indique le point de fixation à D ;
• R₂ représente un groupe -S(O)₂(OH), un groupe -S(O)₂(O⁻M⁺), un groupe -(alkyl en C₁-C₄)-S(O)₂(OH) linéaire ou ramifié, un groupe -(alkyl en C₁-C₄)-S(O)₂(O⁻M⁺)linéaire ou ramifié, un groupe -halogéno(alkyl en C₁-C₄)-S(O)₂(OH) linéaire ou ramifié, ou un groupe -halogéno(alkyl en C₁-C₄)-S(O)₂(O⁻M⁺) linéaire ou ramifié ;
• A₁ représente un groupe -C(O)-CH(R₃)-NH- ;
• A₂ représente un groupe -C(O)-CH(R₄)-NH-, un groupe 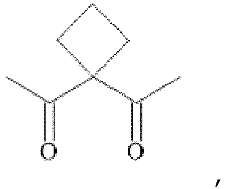 un groupe 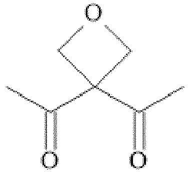 ou un groupe 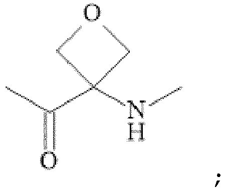
• R₃ et R₄, indépendamment l'un de l'autre, représentent la chaîne latérale d'un acide aminé ;
• R₅ représente un atome d'hydrogène ou un groupe alkyle en C₁-C₄ ;
• Z' représente un précurseur de motif espaceur ;
• M⁺ représente un cation monovalent pharmaceutiquement acceptable.

10. Composé de liaison-médicament selon la revendication 9, dans lequel R₂ représente un groupe -S(O)₂(OH), un groupe - S(O)₂(O-M⁺), un groupe -CH₂-S(O)₂(OH), un groupe -CH₂-S(O)₂(O-M⁺), un groupe -CH₂-CH₂-S(O)₂(OH), un groupe -CH₂-CH₂-S(O)₂(O⁻M⁺), un groupe -CH₂-CH₂-CH₂-S(O)₂(OH) ou un groupe -CH₂-CH₂-CH₂-S(O)₂(O⁻M⁺).

11. Composé de liaison-médicament selon la revendication 9, dans lequel A₁ représente un groupe -C(O)-CH(R₃)-NH- dans lequel R₃ représente un groupe -(CH₂)₃-NH-CO-NH₂ ou un groupe méthyle.

12. Composé de liaison-médicament selon la revendication 9, dans lequel A₂ représente un groupe -C(O)CH(R₄)-NH-, dans lequel R₄ représente un groupe isopropyle ; un groupe 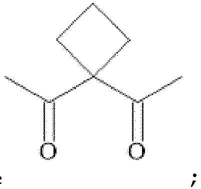 un groupe 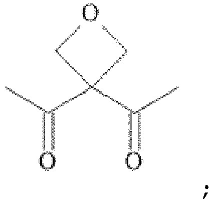 ou un groupe

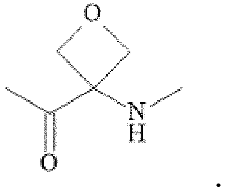

13. Composé de liaison-médicament selon la revendication 9, dans lequel T représente une liaison ou -O-C(O)-*, dans lequel * indique le point de fixation à D.

14. Composé de liaison-médicament selon la revendication 9, dans lequel Z' représente un groupe choisi parmi : 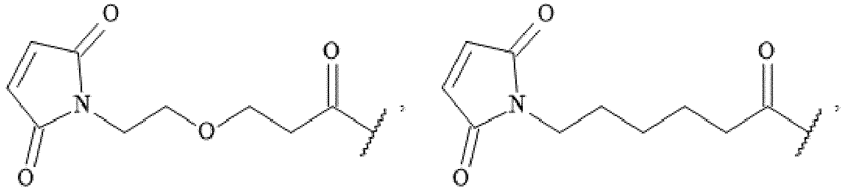 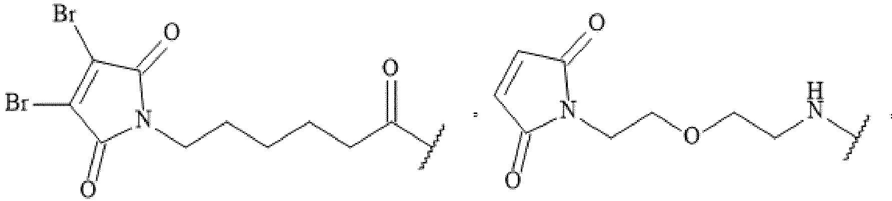 ou 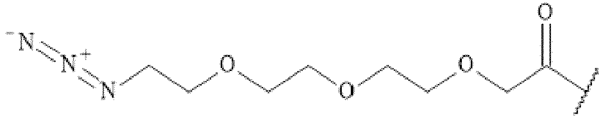 dans lequel la ligne ondulée indique le site de fixation covalent à la terminaison N ou au groupe carbonyle du groupe A₂.

15. Composé de liaison-médicament selon la revendication 9, qui est : 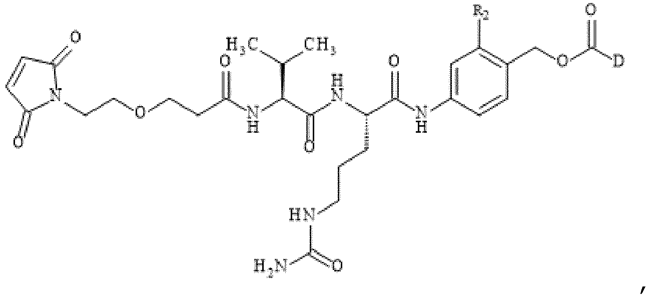 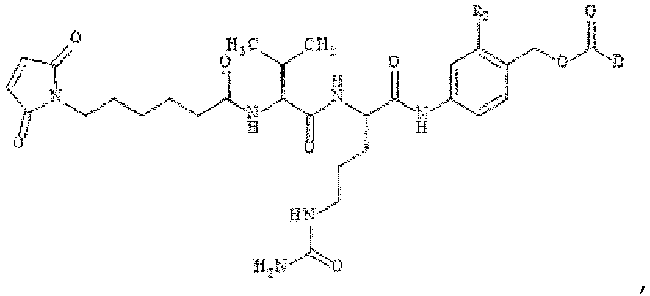 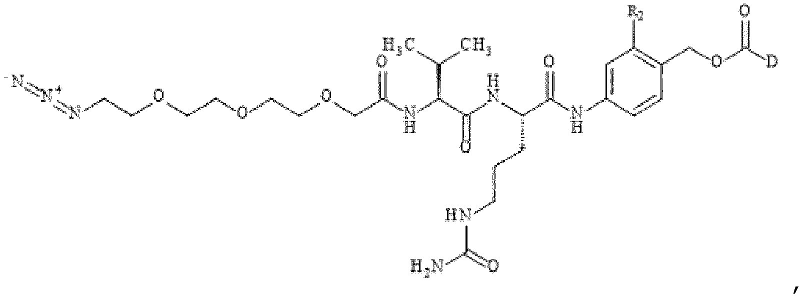 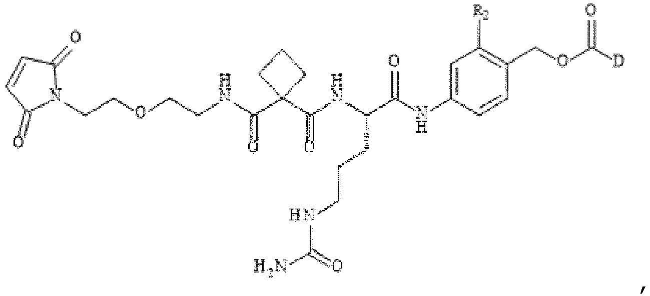 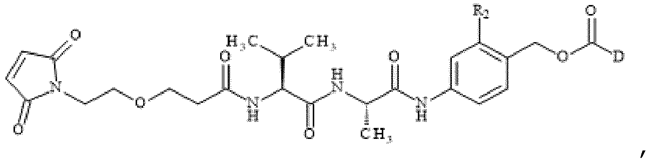 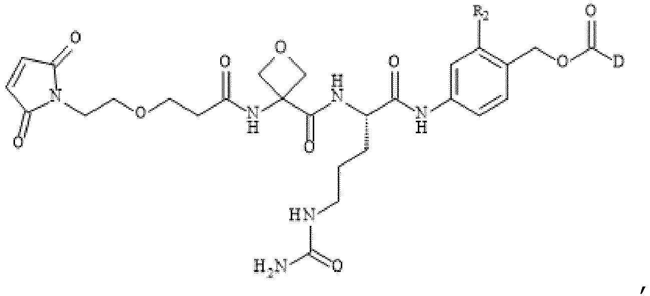 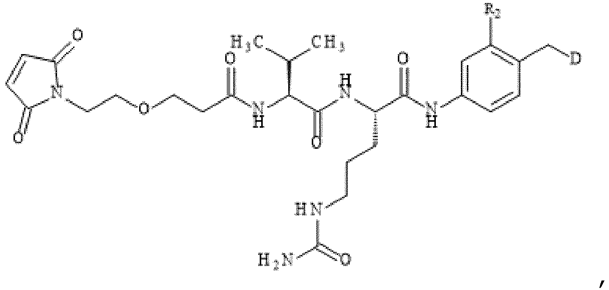 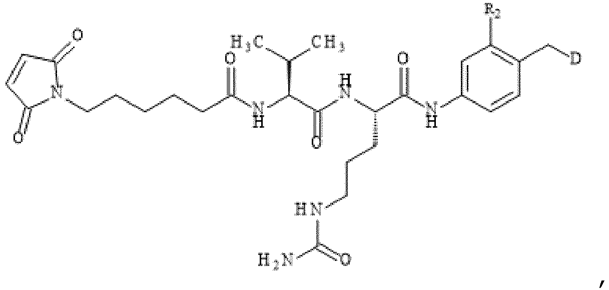 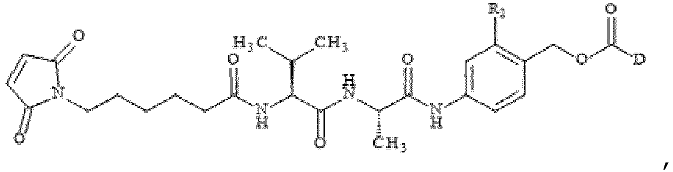 et 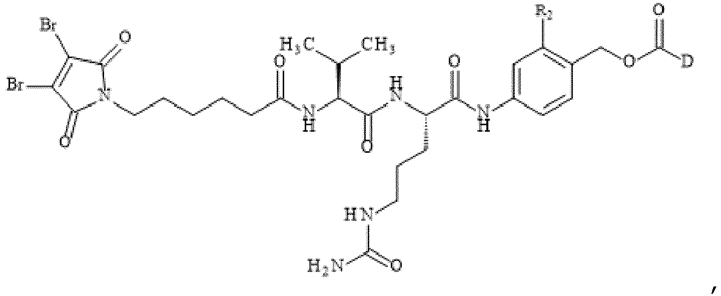 dans lequel R₂, et D sont tels que définis dans la revendication 9.

16. Composé de liaison-médicament selon la revendication 9, qui est :
- le 5-[[(2S)-2-[[(2S)-2-[3-[2-(2,5-dioxopyrrol-1-yl)éthoxy]propanoylamino]-3-méthyl-butanoyl]amino]-5-uréido-pentanoyl]amino]-2-[[[(1S)-1-[[(1S)-1-[[(1S,2R)-4-[(2S)-2-[(1R,2R)-3-[[[(1R,2S)-2-hydroxy-1-méthyl-2 phényl-éthyl]amino]-1-méthoxy-2-méthyl-3-oxo-propyl]pyrrolidin-1-yl]-2-méthoxy-1-[(1S)-1-méthylpropyl]-4-oxo-butyl]-méthyl-carbamoyl]-2-méthyl-propyl]carbamoyl]-2-méthyl-propyl]-méthyl-carbamoyl]oxyméthyl]benzènesulfonate de sodium ;
- le 5-[[(2S)-2-[[(2S)-2-[6-(2,5-dioxopyrrol-1-yl)hexanoylamino]-3-méthyl-butanoyl]amino]-5-uréido-pentanoyl]amino]-2-[[[(1S)-1-[[(1S)-1-[[(1S,2R)-4-[(2S)-2-[(1R,2R)-3-[[(1R,2S)-2-hydroxy-1-méthyl-2-phényl-éthyl]amino]-1-méthoxy-2-méthyl-3-oxo-propyl]pyrrolidin-1-yl]-2-méthoxy-1-[(1S)-1-méthylpropyl]-4-oxo-butyl]-méthyl-carbamoyl]-2-méthyl-propyl]carbamoyl]-2-méthyl-propyl]-méthyl-carbamoyl]oxyméthyl]benzènesulfonate de sodium ;
- le 5-[[(2S)-2-[[(2S)-2-[[2-[2-[2-(2azidoéthoxy)éthoxy]éthoxy]acetyl]amino]-3-méthyl-butanoyl]amino]-5-uréido-pentanoyl]amino]-2-[[[(1S)-1-[[(1S)-1-[[(1S,2R)-4-[(2S)-2-[(1R,2R)-3-[[(1R,2S)-2-hydroxy-1-méthyl-2-phényl-éthyl]amino]-1-méthoxy-2-méthyl-3-oxo-propyl]pyrrolidin-1-yl]-2-méthoxy-1-[(1S)-1-méthylpropyl]-4-oxo-butyl]-méthyl-carbamoyl]-2-méthyl-propyl]carbamoyl]-2-méthyl-propyl]-méthyl-carbamoyl]oxyméthyl]benzènesulfonate de sodium ;
- l'acide 5-[[(2S)-2-[[1-[2-[2-(2,5-dioxopyrrol-1-yl)éthoxy]éthylcarbamoyl]cyclobutanecarbonyl]amino]-5-uréido-pentanoyl]amino]-2-[[[(1S)-1-[[(1S)-1-[[(1S,2R)-4-[(2S)-2-[(1R,2R)-3-[[(1R,2S)-2-hydroxy-1-méthyl-2-phényl-éthyl]amino]-1-méthoxy-2-méthyl-3-oxo-propyl]pyrrolidin-1-yl]-2-méthoxy-1-[(1S)-1-méthylpropyl]-4-oxo-butyl]-méthyl-carbamoyl]-2-méthyl-propyl]carbamoyl]-2-méthyl-propyl]-méthyl-carbamoyl]oxyméthyl]benzènesulfonique ;
- le 5-[[(2S)-2-[[(2S)-2-[3-[2-(2,5-dioxopyrrol-1-yl)éthoxy]propanoylamino]-3-méthyl-butanoyl]amino]propanoyl]amino]-2-[[[(1S)-1-[[(1S)-1-[[(1S,2R)-4-[(2S)-2-[(1R,2R)-3-[[[(1R,2S)-2-hydroxy-1-méthyl-2 phényl-éthyl]amino]-1-méthoxy-2-méthyl-3-oxo-propyl]pyrrolidin-1-yl]-2-méthoxy-1-[(1S)-1-méthylpropyl]-4-oxo-butyl]-méthyl-carbamoyl]-2-méthyl-propyl] carbamoyl]-2-méthyl-propyl]-méthyl-carbamoyl]oxyméthyl]benzènesulfonate de sodium ;
- l'acide 5-[[(2S)-2-[[(2S)-2-[3-[2-(2,5-dioxopyrrol-1-yl)éthoxy]propanoylamino]-3-méthyl-butanoyl]amino]propanoyl]amino]-2-[[[(1S)-1-[[(1S)-1-[[(1S,2R)-4-[(2S)-2-[(1R,2R)-3-[[[(1R,2S)-2-hydroxy-1-méthyl-2 phényl-éthyl]amino]-1-méthoxy-2-méthyl-3-oxo-propyl]pyrrolidin-1-yl]-2-méthoxy-1-[(1S)-1-méthylpropyl]-4-oxo-butyl]-méthyl-carbamoyl]-2-méthyl-propyl] carbamoyl]-2-méthyl-propyl]-méthyl-carbamoyl]oxyméthyl]benzènesulfonique ;
- l'acide 5-[[(2S)-2-[[3-[2-[2-(2,5-dioxopyrrol-1-yl)éthoxy]éthylcarbamoyl]oxétane-3-carbonyl]amino]-5-uréido-pentanoyl]amino]-2-[[[(1S)-1-[[(1S)-1-[[(1S,2R)-4-[(2S)-2-[(1R,2R)-3-[[(1R,2S)-2-hydroxy-1-méthyl-2-phényl-éthyl]amino]-1-méthoxy-2-méthyl-3-oxo-propyl]pyrrolidin-1-yl]-2-méthoxy-1-[(1S)-1-méthylpropyl]-4-oxo-butyl]-méthyl-carbamoyl]-2-méthyl-propyl]carbamoyl]-2-méthyl-propyl]-méthyl-carbamoyl]oxyméthyl]benzènesulfonique ;
- l'acide [5-[[(2S)-2-[[(2S)-2-[3-[2-(2,5-dioxopyrrol-1-yl)éthoxy]propanoylamino]-3-méthyl-butanoyl]amino]-5-uréido-pentanoyl]amino]-2-[[[(1S)-1-[[(1S)-1-[[(1S,2R)-4-[(2S)-2-[(1R,2R)-3-[[(1R,2S)-2-hydroxy-1-méthyl-2-phényl-éthyl]amino]-1-méthoxy-2-méthyl-3-oxo-propyl]pyrrolidin-1-yl]-2-méthoxy-1-[(1S)-1-méthylpropyl]-4-oxo-butyl]-méthyl-carbamoyl]-2-méthyl-propyl]carbamoyl]-2-méthyl-propyl]-méthyl-carbamoyl]oxyméthyl]phényl]méthanesulfonique ;
- le 2,2,2-trifluoroacétate de [4-[[(2S)-2-[[(2S)-2-[3-[2-(2,5-dioxopyrrol-1-yl)éthoxy]propanoylamino]-3-méthyl-butanoyl]amino]-5-uréido-pentanoyl]amino]-2-sulfo-phényl]méthyl-[(1S)-1-[[(1S)-1-[[[(1S,2R)-4-[(2S)-2-[(1R,2R)-3-[[[(1R,2S)-2-hydroxy-1-méthyl-2-phényl-éthyl]amino]-1-méthoxy-2-méthyl-3-oxo-propyl]pyrrolidin-1-yl]-2-méthoxy-1-[(1S)-1-méthylpropyl]-4-oxo-butyl]-méthyl-carbamoyl]-2-méthyl-propyl]carbamoyl]-2-méthyl-propyl]-diméthyl-ammonium ;
- *la N*-({[4-({*N*-[6-(2,5-dioxo-2,5-dihydro-1H-pyrrol-1-yl)hexanoyl]-L-valyl-L-alanyl}amino)-2-sulfophényl]méthoxy}carbonyl)-*N*-méthyl-L-valyl-*N*-[(3*R*,4*S*,5*S*)-1-{(2S)-2-[(1*R*,2*R*)-3-{[(1*S*,2*R*)-1-hydroxy-1-phénylpropan-2-yl]amino}-1-méthoxy-2-méthyl-3-oxopropyl]pyrrolidin-1-yl}-3-méthoxy-5-méthyl-1-oxoheptan-4-yl]-*N*-méthyl-L-valinamide ;
- *le N*-[6-(2,5-dioxo-2,5-dihydro-1H-pyrrol-1-yl)hexanoyl]-L-valyl-*N*⁵-carbamoyl-*N*-(4-{[({[(4*S*)-4,11-diéthyl-9-hydroxy-3,14-dioxo-3,4,12,14-tetrahydro-1*H-*pyrano[3',4':6,7]indolizino[1,2-*b*]quinolin-4-yl]oxy}carbonyl)oxy]méthyl}-3-sulfophényl)-L-ornithinamide ;
- le (1*S*,3*S*)-3,5,12-trihydroxy-3-(hydroxyacétyl)-10-méthoxy-6,11-dioxo-1,2,3,4,6,11-hexahydrotétracén-1-yl 2,3,6-tridesoxy-3-[({[4-({*N*-[6-(2,5-dioxo-2,5-dihydro-1H-pyrrol-1-yl)hexanoyl]-L-valyl-*N*⁵-carbamoyl-L-ornithyl}amino)-2-sulfophényl]méthoxy}carbonyl)amino]-α-L-*lyxo*-hexopyranoside ;
- *le N*-{3-[2-(2,5-dioxo-2,5-dihydro-1*H*-pyrrol-1-yl)éthoxy]propanoyl}-L-valyl-N-{4-[(5*S*,8*S*,11*S*,12*R*)-11-[(2*S*)-butan-2-yl]-12-(2-{(2*S*)-2-[(1*R*,2*R*)-3,2*R*)-1-hydroxy-1-phénylpropan-2-yl]amino}-1-méthoxy-2-méthyl-3-oxopropyl]pyrrolidin-1-yl}-2-oxoéthyl)-4,10-diméthyl-3,6,9-trioxo-5,8-di(propan-2-yl)-2,13-dioxa-4,7,10-triazatetradécan-1-yl]-3-(2-sulfoéthyl)phényl}-*N*⁵-carbamoyl-L-ornithinamide ;
- l'acide 5-[[(2S)-2-[[(2S)-2-[6-(3,4-dibromo-2,5-dioxo-pyrrol-1-yl)hexanoylamino]-3-méthyl-butanoyl]amino]-5-uréido-pentanoyl]amino]-2-[[[(1S)-1-[[(1S)-1-[[(1S,2R)-4-[(2S)-2-[(1R,2R)-3-[[(1R,2S)-2-hydroxy-1-méthyl-2-phényl-éthyl]amino]-1-méthoxy-2-méthyl-3-oxo-propyl]pyrrolidin-1-yl]-2-méthoxy-1-[(1S)-1-méthylpropyl]-4-oxo-butyl]-méthyl-carbamoyl]-2-méthyl-propyl]carbamoyl]-2-méthyl-propyl]-méthyl-carbamoyl]oxyméthyl]benzènesulfonique.

17. Conjugué anticorps-médicament de formule (III) : 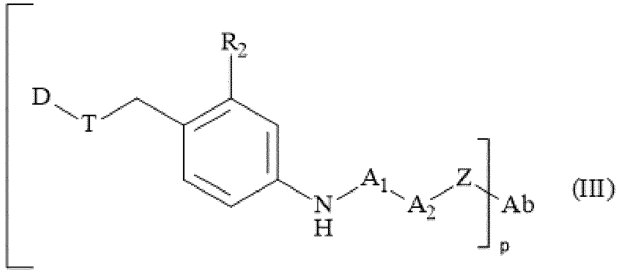 dans lequel
• Ab représente un anticorps ou un fragment de liaison à l'antigène de celui-ci ;
• D représente un groupement médicamenteux ;
• T est une liaison, -O-C(O)-N(CH₃)-CH₂-CH₂-N(CH₃)-C(O)-*, - O-*, -NR₅ -*, -NR₅-C(O)-*, ou -O-C(O)-*, dans lequel * indique le point de fixation à D ;
• Z représente un motif espaceur ;
• A₁ représente un groupe -C(O)-CH(R₃)-NH- ;
• A₂ représente un groupe -C(O)-CH(R₄)-NH-, un groupe 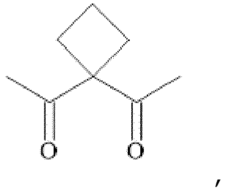 un groupe 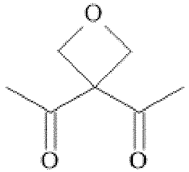 ou un groupe 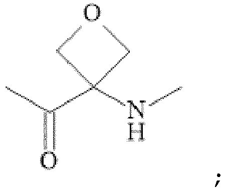
• R₂ représente un groupe -S(O)₂(OH), un groupe -S(O)₂(O-M⁺), un groupe -(alkyl en C₁-C₄)-S(O)₂(OH) linéaire ou ramifié, un groupe - (alkyl en C₁-C₄)-S(O)₂(O⁻M⁺)linéaire ou ramifié, un groupe -halogéno(alkyl en C₁-C₄)-S(O)₂(OH) linéaire ou ramifié, ou un groupe -halogéno (alkyl en C₁-C₄)-S(O)₂(0⁻M⁺) linéaire ou ramifié ;
• R₃ et R₄, indépendamment l'un de l'autre, représentent la chaîne latérale d'un acide aminé ;
• R₅ représente un groupe alkyle en C₁-C₄ ;
• M⁺ représente un cation monovalent pharmaceutiquement acceptable ; et
• p est un nombre entier de 1 à 8.

18. Conjugué anticorps-médicament selon la revendication 17, dans lequel T est une liaison ou -O-C(O)-*, dans lequel * indique le point de fixation à D.

19. Conjugué anticorps-médicament selon la revendication 17, dans lequel R₂ représente un groupe -S(O)₂(OH), un groupe - S(O)₂(O-M⁺), un groupe -CH₂-S(O)₂(OH), un groupe -CH₂-S(O)₂(O-M⁺), un groupe -CH₂-CH₂-S(O)₂(OH), un groupe -CH₂-CH₂-S(O)₂(O-M⁺), un groupe -CH₂-CH₂-CH₂-S(O)₂(OH) ou un groupe -CH₂-CH₂-CH₂-S(O)₂(O⁻M⁺).

20. Conjugué anticorps-médicament selon la revendication 17, dans lequel A₁ représente un groupe -C(O)-CH(R₃)-NH- dans lequel R₃ représente un groupe -(CH₂)₃-NH-CO-NH₂ ou un groupe méthyle.

21. Conjugué anticorps-médicament selon la revendication 17, dans lequel A₂ représente un groupe -C(O)-CH(R₄)-NH- dans lequel R₄ représente un groupe isopropyle ; un groupe 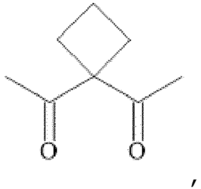 un groupe 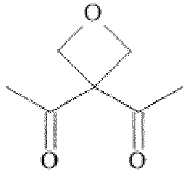 ou un groupe

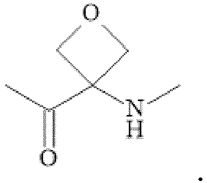

22. Conjugué anticorps-médicament selon la revendication 17, dans lequel ledit conjugué anticorps-médicament est formé à partir d'un composé de liaison-médicament de formule (II) choisi parmi : 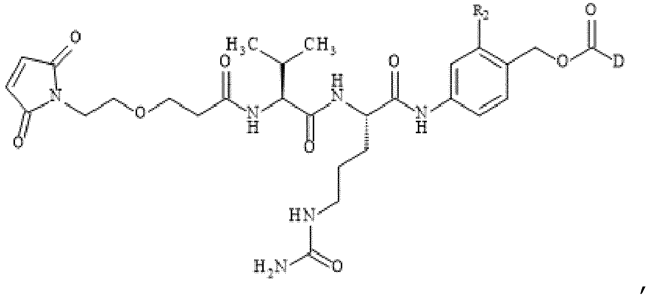 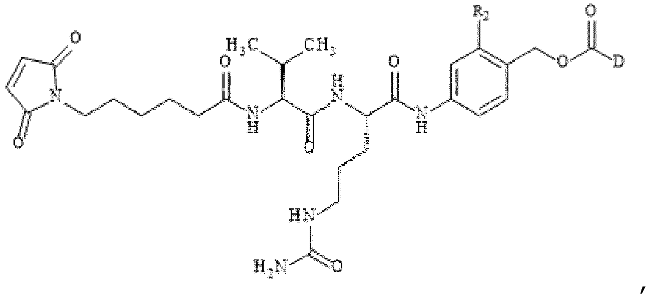 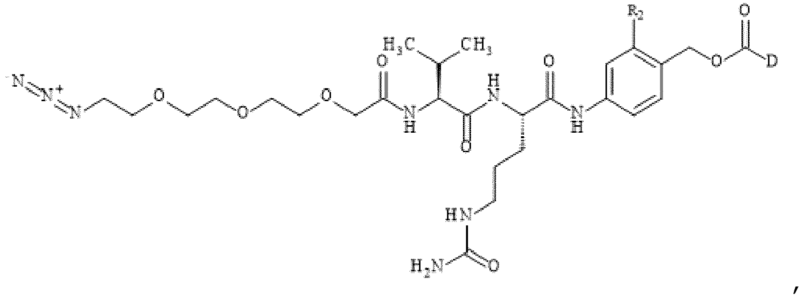 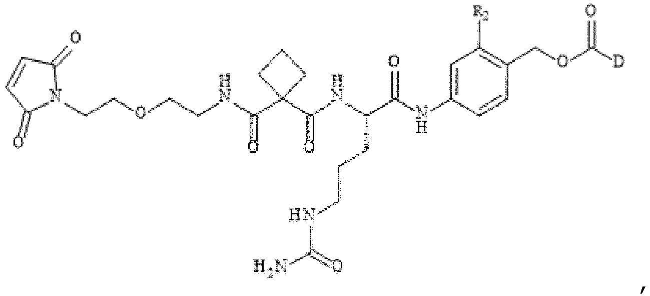 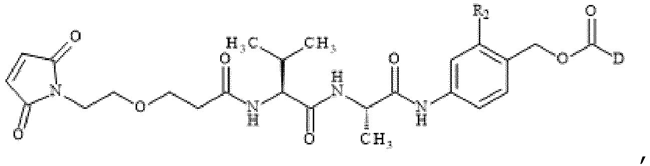 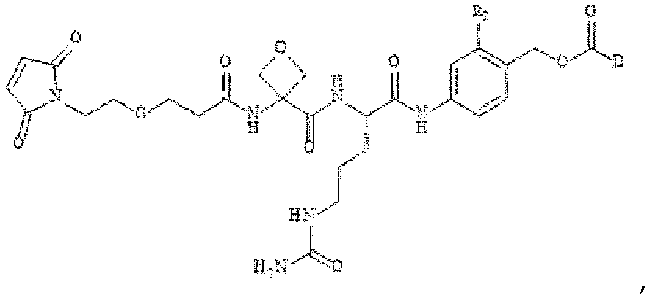 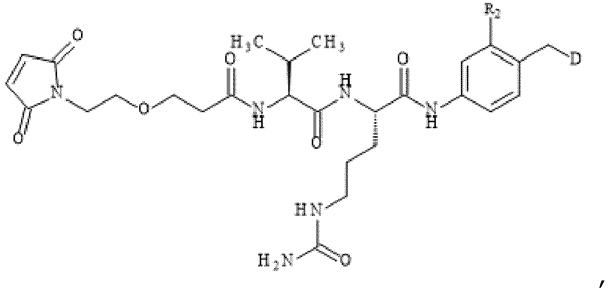 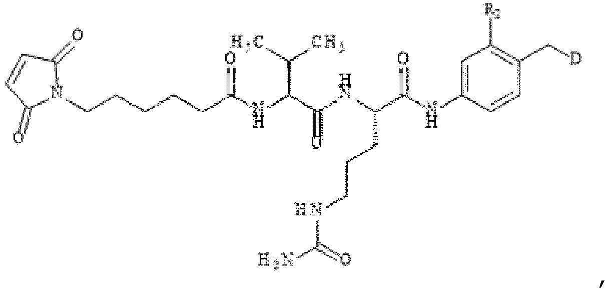 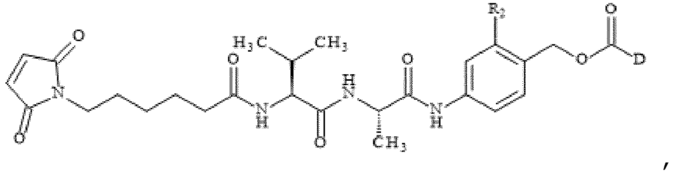 et 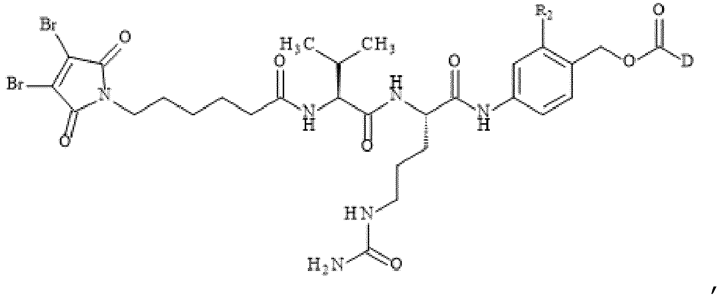 dans lequel R₂, et D sont tels que définis dans la revendication 17.

23. Conjugué anticorps-médicament selon la revendication 17, dans lequel ledit conjugué anticorps-médicament comprend une formule choisie parmi : 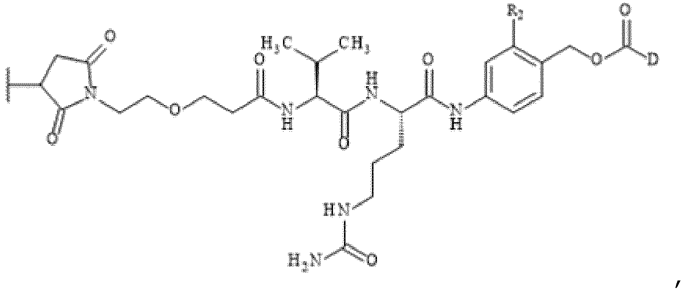 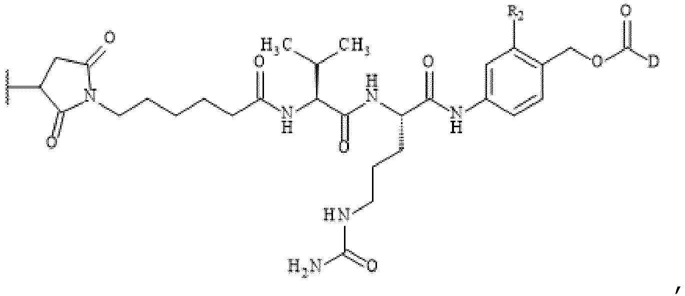 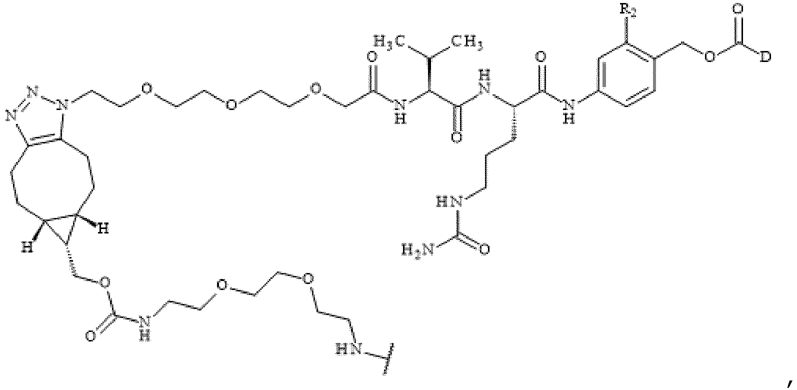 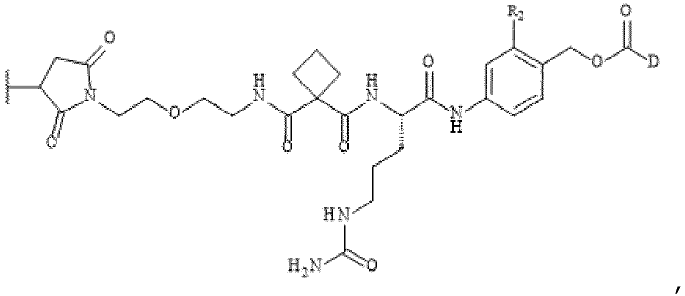 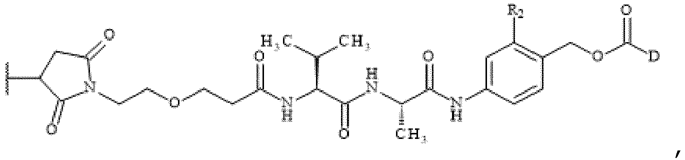 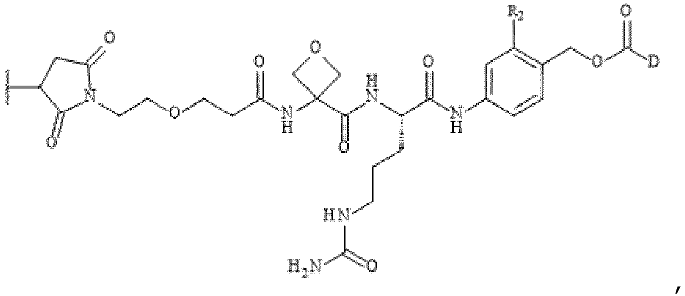 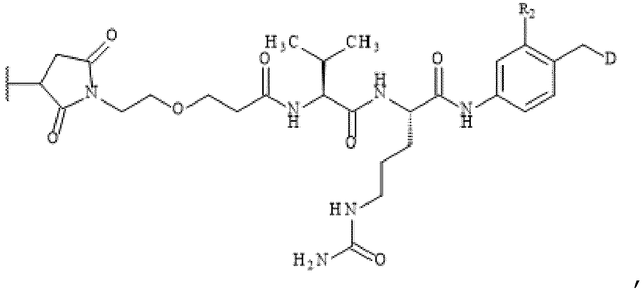 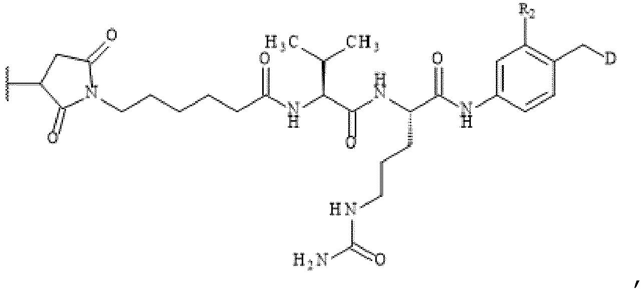 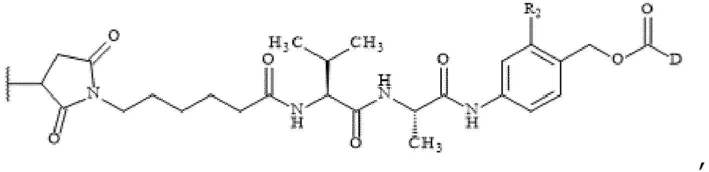 et 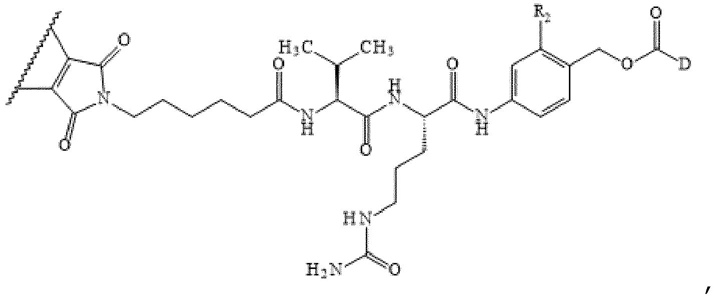 dans lequel une ligne ondulée indique le site de fixation covalent à l'anticorps ou à un fragment de liaison à l'antigène de celui-ci et D et R₂ sont tels que définis dans la revendication 17.

24. Composition pharmaceutique comprenant le conjugué anticorps-médicament selon l'une quelconque des revendications 17 à 23 et un véhicule pharmaceutiquement acceptable.

25. Conjugué anticorps-médicament selon l'une quelconque des revendications 17 à 23, destiné à être utilisé dans le traitement d'un mammifère en ayant besoin.
